(19) Europäisches Patentamt
European Patent Office
Office européen des brevets

(11) EP 1 918 284 A1

(12) EUROPEAN PATENT APPLICATION

(43) Date of publication:
**07.05.2008 Bulletin 2008/19**

(21) Application number: **06381045.1**

(22) Date of filing: **30.10.2006**

(51) Int Cl.:
*C07D 239/42* (2006.01) *C07D 401/12* (2006.01)
*C07D 403/12* (2006.01) *C07D 405/12* (2006.01)
*A61K 31/505* (2006.01) *A61K 31/506* (2006.01)
*A61P 33/06* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **GLAXO GROUP**
**Berkeley Avenue**
**Brentford, Middlesex UB6 0NN (GB)**

(72) Inventor: **The designation of the inventor has not yet been filed**

(74) Representative: **Elzaburu Marquez, Alberto et al**
**Elzaburu S.A.,**
**Miguel Angel, 21**
**28010 Madrid (ES)**

(54) **Hydrazinopyrimidines as cysteine protease inhibitors**

(57) Substituted heteroaryl nitrile derivatives of Formula I,

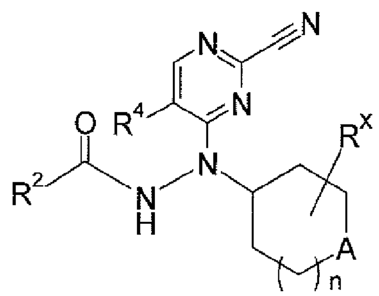

I

processes for their preparation, pharmaceutical compositions comprising such compounds and use of the compounds as cysteine protease inhibitors are provided.

EP 1 918 284 A1

## Description

## FIELD OF THE INVENTION

The invention is directed to certain substituted heteroaryl nitrile derivatives, which are protease inhibitors. More specifically, the compounds are inhibitors of cysteine proteases. In particular, the compounds inhibit cysteine proteases of the papain superfamily, more specifically those of the falcipain family, which are cysteine proteases found in the malaria parasite *Plasmodium falciparum,* and also cysteine proteases of the cathepsin family such as cathepsins K, L, S and B.

## BACKGROUND OF THE INVENTION

Malaria is one of the major disease problems of the developing world. The most virulent malaria-causing parasite in humans is *Plasmodium falciparum*, which is the cause of hundreds of millions of cases of malaria per annum, and is thought to cause over 1 million deaths each year, Breman, J. G., et al., (2001) Am. Trop. Med. Hyg. 64, 1-11. One problem encountered in the treatment of malaria is the build-up of resistance by the parasite to available drugs. Thus there is a need to develop new antimalarial drugs.

One way of identifying a potential new drug with antimalarial activity is to study biological targets found in the *Plasmodium falciparum* parasite, in turn by investigating biological pathways in which particular targets might be identified. In *Plasmodium falciparum,* haemoglobin is transported to an acidic food vacuole, where it is degraded. It appears that multiple enzymes, including food vacuole cysteine, aspartic, and metalloproteases, and a cytosolic aminopeptidase, contribute to haemoglobin hydrolysis, Francis S.E. et al., (1997) Annu. Rev. Microbiol. 51, 97-123; Rosenthal P.J. Protease inhibitors. In: Rosenthal P.J., ed. Antimalarial Chemotherapy: Mechanisms of Action, Resistance, and New Directions in Drug Discovery, Totowa, N.J.: Humana Press, (2001) 325-345. Plasmodial haemoglobinases are therefore potential therapeutic targets.

Cysteine protease inhibitors were shown some years ago to block haemoglobin degradation by erythrocytic parasites, causing a characteristic morphological abnormality in which the food vacuole fills with undegraded haemoglobin and parasite development is blocked, Rosenthal P. J., et al., (1998) J. Clin. Invest. 82, 1560-6; Gamboa de Dominguez N.D. and Rosenthal P.J., (1996) Blood 87, 4448-54. Efforts to identify enzymes responsible for haemoglobin degradation led to the characterization of "falcipain" as a trophozoite food vacuole cysteine protease, Rosenthal P.J. and Nelson R.G., (1992) Mol Biochem Parasitol 51, 143-52; Salas F. et al., (1995) Infect. Immun. 63 2120-5. It has more recently been found that "falcipain" actually constitutes three related papain-family cysteine proteases which share a number of unusual features, known as falcipain-1, falcipain-2 and falcipain-3, Rosenthal, P. J., et al., (2002) Curr. Pharm. Des. 8, 1669-1672. Falcipain-2 is the principal cysteine protease of *Plasmodium falciparum* trophozoites, Shenai B.R. et. al., (2000) J Biol Chem 275, 29000-10. Importantly, cysteine protease inhibitors that inhibit falcipain-2 consistently block haemoglobin hydrolysis and parasite development. These data suggest that falcipain-2 is a key target enzyme, but it is likely that the other two falcipains are also appropriate targets and that, in many cases, they are inhibited by the same compounds that are active against falcipain-2. Like falcipain-2, falcipain-3 readily hydrolyzes native haemoglobin under mildly reducing conditions that are similar to those found in physiological systems, Shenai B.R. et al., (2000) J. Biol. Chem. 275, 29000-10; Sijwali P.S. et al., (2001) Biochem. J. 360, 481-9; Shenai B.R. and Rosenthal P.J., (2002) Mol. Biochem. Parasitol. 122, 99-104. Falcipain-2 and falcipain-3 are similar in structure but falcipain-1 is a more distant relative; it is thought that this enzyme plays a key role in the invasion of erythrocytes by *Plasmodium falciparum* merozoites but that it is not essential for normal development during the erythrocytic stage, Sijwali, P. S., et al., Proceedings of the National Academy of Sciences of the United States of America 101, 8721-8726. Whether falcipain-1 also plays a role in haemoglobin processing is unknown. Very recently, a fourth papain-family cysteine protease has been found, now known as falcipain-2'. Falcipain-2' is nearly identical in sequence to falcipain-2, differing by only 3 amino acids, none of which are located at the active site. The structure of falcipain-2' is not known, but is likely to be very similar to that of falcipain-2. The biological role of falcipain-2' is also expected to be very similar, although probably not identical, to that of falcipain-2. In any event, cysteine protease inhibition, in particular the inhibition of falcipain-2, blocks parasite development. Falcipain-2 and related plasmodial cysteine proteases are thus logical targets for antimalarial chemotherapy and therefore there is a need for compounds which are inhibitors of these targets.

*P. vivax* is the second most important human malaria parasite, after *P. falciparum.* Although less virulent than *P. falciparum, P. vivax* is the most widely distributed human malaria parasite, and it causes extensive morbidity (Mendis, K., Sina, B. J., Marchesini, P. and Carter, R. (2001) "The neglected burden of Plasmodium vivax malaria" Am. J. Trop. Med. Hyg. 64, 97-106). These two parasites are responsible for more than 90% of episodes of human malaria, totalling several hundred million cases annually. However, comprehensive studies of *P. vivax* have been limited due to technical shortcomings. Notably, unlike the case with *P. falciparum,* routine *in vitro* culture of *P. vivax* is not available, and animal models are limited to primates. Very recently (Na, B.K., Shenai, B. R., Sijwali, P. S., Choe, Y., Pandey, K. C., Singh, A.,

Craik, C. S., Rosenthal, P. J. (2004) identification and biochemical characterization of vivapains, cysteine proteases of the malaria parasite Plasmodium vivax. Biochem. J. 378, 529-538), two cysteine protease genes (vivapain-2 and vivapain-3) from *P. vivax* have been identified and cloned and the heterologously expressed gene products have been characterized biochemically. It was found that these cysteine proteases are apparent orthologues of falcipain-2 and falcipain-3, but key differences in the biochemical properties of the plasmodial proteases warrant attention to the inhibition of each enzyme in the evaluation of antimalarial protease inhibitors.

Cathepsins are a family of enzymes which are part of the papain superfamily of cysteine proteases. Certain cathepsins, for example cathepsins K, B, L, and S have been described in the literature. Cathepsin K polypeptide and the cDNA encoding such polypeptide were disclosed in U.S. Patent No. 5,501,969. Cathepsin K has also been variously denoted as cathepsin O or cathepsin 02 in the literature. The designation cathepsin K is considered to be the most appropriate and is used herein. Cathepsin K has been expressed, purified, and characterised, Bossard, M. J., et al., (1996) J. Biol. Chem. 271, 12517-12524; Drake, F.H., et al., (1996) J. Biol. Chem. 271, 12511-12516; Bromme, D., et al., (1996) J. Biol. Chem. 271, 2126-2132.

Cathepsins function in the normal physiological process of protein degradation in animals, including humans, e.g. in the degradation of connective tissue. However, elevated levels of these enzymes in the body can result in pathological conditions leading to disease. Thus, cathepsins have been implicated as causative agents in various disease states, including but not limited to, infections by *Pneumocystis Carinii, Trypsanoma cruzi, Trypsanoma brucei,* and *Crithidia fusiculata;* as well as in schistosomiasis, malaria, cancer, for example pancreatic cancer (see Joyce J. A. et al., Cancer Cell (2004) 5, 443-453 and Gocheva V., Genes & Development (2006) 20, 543-556), tumour invasion and tumour metastasis, metachromatic leukodystrophy, muscular dystrophy, amytrophy, inflammation, rheumatoid arthritis, osteoarthritis, osteoporosis, coronary disease, atherosclerosis, autoimmune diseases, respiratory diseases such as obstructive pulmonary disorder (COPD), immunologically mediated diseases (for example, transplant rejection), and other related diseases, see: International Publication Number WO 94/04172, published on March 3, 1994, and references cited therein; see also: European Patent Application EP 0 603 873 A1, and references cited therein. Two bacterial cysteine proteases from *P. gingivallis,* called gingipains, have been implicated in the pathogenesis of gingivitis, Potempa, J., et al., (1994) Perspectives in Drug Discovery and Design 2, 445-458.

Cathepsin K is believed to play a causative role in diseases of excessive bone or cartilage loss. Bone is composed of a protein matrix in which spindle- or plate-shaped crystals of hydroxyapatite are incorporated. Type I collagen represents the major structural protein of bone comprising approximately 90% of the protein matrix. The remaining 10% of matrix is composed of a number of non-collagenous proteins, including osteocalcin, proteoglycans, osteopontin, osteonectin, thrombospondin, fibronectin, and bone sialoprotein. Skeletal bone undergoes remodeling at discrete foci throughout life. These foci, or remodeling units, undergo a cycle consisting of a bone resorption phase followed by a phase of bone replacement.

Bone resorption is carried out by osteoclasts, which are multinuclear cells of haematopoietic lineage. In several disease states, such as osteoporosis and Paget's disease, the normal balance between bone resorption and formation is disrupted, and there is a net loss of bone at each cycle of resorption and formation. Ultimately, this leads to weakening of the bone and may result in increased fracture risk with minimal trauma. Several published studies have demonstrated that inhibitors of cysteine proteases are effective at inhibiting osteoclast-mediated bone resorption, thus indicating an essential role for cysteine proteases in bone resorption. For example, Delaisse, et al., (1980) Biochem. J., 192, 365, suggests that inhibitors of cysteine proteases (e.g., leupeptin, Z-Phe-Ala-$CHN_2$) prevent bone resorption, while serine protease inhibitors were ineffective. Delaisse et. al., (1984) Biochem. Biophys. Res. Commun. 125, 441, discloses that E-64 (L-trans-epoxysuccinyl-leucinamido-(4-guanidino)butane) and leupeptin are also effective at preventing bone resorption *in vivo* in rats. Lerner, et al., (1992) J. Bone Min. Res. 7, 433, discloses that cystatin, an endogenous cysteine protease inhibitor, inhibits PTH stimulated bone resorption in mouse calvariae. Other studies report a correlation between inhibition of cysteine protease activity and bone resorption. Tezuka, et al., (1994) J. Biol. Chem. 269, 1106; Inaoka, et al., (1995) Biochem. Biophys. Res. Commun., 206, 89 and Shi, et al., (1995) FEBS Lett. 357, 129 disclose that under normal conditions cathepsin K is abundantly expressed in osteoclasts and may be the major cysteine protease present in these cells.

The abundant selective expression of cathepsin K in osteoclasts strongly suggests that this enzyme is essential for bone resorption. Thus, inhibition of cathepsin K may provide an effective treatment for diseases of excessive bone loss, including, but not limited to, osteoporosis, gingival diseases such as gingivitis and periodontitis, Paget's disease, hypercalcemia of malignancy, and metabolic bone disease. Cathepsin K levels have also been demonstrated to be elevated in chondroclasts of osteoarthritic synovium. Cathepsin K is also expressed in synovial giant cells taken from osteoarthritic patients (Dodds, et al., (1999) Arthritis & Rheumatism, 42, 1588, and Hou, et al., (2002), American Journal of Pathology 159, 2167). Cathepsin K staining is observed in osteoarthritic as well as rheumatoid arthritic samples (Hou, et al., (2002), American Journal of Pathology 159, 2167). The expression of cathepsin K has also been localized to cartilage tissue and a decrease in pH in cartilage correlated with severity of damage (Konttinen, et al., (2002), Arthritis & Rheumatism, 46, 953). This observation, combined with the fact that cathepsin K is an acidic lysosomal protease,

strongly suggests a physiological role of cathepsin K in cartilage turnover in addition to bone resorption. These researchers also demonstrated that cathepsin K can degrade aggrecan and type II collagen, the two major protein components of the cartilage matrix. Thus, inhibition of cathepsin K may also be useful for treating diseases of excessive cartilage or matrix degradation, including, but not limited to, osteoarthritis and rheumatoid arthritis. Cathepsin K has been shown to be abnormally or overexpressed in numerous tumors and in prostate cancer (Littlewood-Evans, et al., (1997), Cancer Res., 57, 5386 and Brubaker, et al., (2003), J. Bone Miner. Res., 18, 222). Furthermore, increased levels of bone resorption marker have been detected in bone metastases of prostate cancer suggesting that cathepsin K inhibitor may have utility in preventing metastasis of tumors to bone (Ishikawa, et al., (2001), Mol. Carcinog., 32, 84 and Brubaker, et al., (2003), J. Bone Miner. Res., 18, 222). Metastatic neoplastic cells also typically express high levels of other proteolytic enzymes such as cathepsin B, S and L that degrade the surrounding matrix. Thus, inhibition of cathepsin K may also be useful for treating certain tumors and neoplastic diseases.

Cathepsin L has been implicated in several diseases including osteoporosis, osteoarthritis, rheumatoid arthritis, lymphoproliferative diseases, cancer, for example pancreatic cancer, metastasis, atherosclerosis (Lecaille, et al., (2002) Chem. Rev. 102, 4459 and Liu, et al., (2004), Arterioscler Throm Vasc Biol. 24, 1359). Cathepsin L-deficient mice have also been shown to have increased resistance to osteoporosis following ovariectomy suggesting its potential for osteoporosis (Potts, et al., (2004) Int. J. Exp. Path. 85, 85). Cathepsin L is required for endothelial progenitor cell-induced neovascularization (Urbich, et al., (2005) Nat. Med. 11, 206). Similarly, targeting cathepsin L by specific ribozymes decreases cathepsin L protein synthesis and cartilage destruction in rheumatoid arthritis (Schedel, et al., (2004) Gene Ther. 11, 1040) suggesting its potential role in rheumatoid arthritis.

Cathepsin S has been implicated in several diseases including immune and auto-immune disorders, rheumatoid arthritis, inflammation, inflammatory bowel disease, myesthania gravis, atherosclerosis, lymphoproliferative diseases, cancer, for example pancreatic cancer, metastasis (Lecaille, et al., (2002) Chem. Rev. 102, 4459 and Liu, et al., (2004), Arterioscler Throm Vasc Biol. 24, 1359). Cathepsin S is thought to play a role in invariant chain degradation and antigen presentation and cathepsin S null mice have been shown to have a diminished collagen-induced arthritis (Nakagawa, et al., (1999) Immunity, 10, 207) suggesting its potential role in rheumatoid arthritis.

Cathepsin B has been implicated in immune and auto-immune disorders, rheumatoid arthritis, inflammation, inflammatory bowel disease, myesthania gravis, osteoarthritis, lymphoproliferative diseases, cancer, for example pancreatic cancer, metastasis (Lecaille, et al., (2002) Chem. Rev. 102, 4459 and Lang, et al., (2000), J. Rheumatol. 27, 1970). Cathepsin B has been implicated in the processing of invariant chain (Zhang, et al., (2000) Immunology, 100, 13) suggesting its role in immune disorders such as those listed above. Cathepsin B is one of the most highly expressed cysteine protease in cartilage and inhibitors of cathepsin B has been shown to inhibit cartilage degradation. Cathepsin B may contribute to matrix degradation through cleavage of aggrecan and collagen, two components of cartilage matrix (Mort et al., (1998), Biochem. J., 335, 491). Additionally, cathepsin B could contribute to the mechanical loading component of osteoarthritis by cleaving lubricin, an abundant lubricating protein in synovial fluid. Cleavage of lubricin by cathepsin B has been shown to increase the coefficient of friction in synovial fluid and intact joints (Elsaid, K.A. et al. (2005), Transactions of the Orthopedic Research Society, 51st Annual Meeting, Abstract 924). These data suggest potential for cathepsin B inhibitors in osteoarthritis.

In view of the number of pathological responses and conditions that are mediated by cathepsins K, L, S and B, there is a need for inhibitors of these cathepsins which can be used in the treatment of a variety of conditions.

WO 2005/085210 A1 discloses certain fused bicyclic pyrimidine compounds as inhibitors of cathepsin K, useful in the treatment of bone diseases such as osteoporosis and the like. WO 2005/103012 A1 discloses certain hydrazine-heterocyclic nitrile compounds as inhibitors of cathepsin K, useful in the treatment of bone diseases such as osteoporosis and the like.

## SUMMARY OF THE INVENTION

The invention is directed to novel heteroaryl nitrile derivatives and their use as protease inhibitors, more specifically inhibitors of cysteine protease, even more specifically inhibitors of cysteine proteases of the papain superfamily. In one aspect of the invention the cysteine proteases are those of the falcipain family, for example falcipain-2 and falcipain-3, which are examples of cysteine proteases indicated in malaria. In another aspect of the invention the cysteine proteases are those of the cathepsin family for example cathepsins K, L, S and B, which is a cysteine protease indicated for example in conditions characterised by excessive bone loss such as osteoporosis and bone metastasis, and other bone and joint diseases such as osteoarthritis. The compounds of the invention may also have utility as serine protease inhibitors.

The invention involves the compounds represented hereinbelow, pharmaceutical compositions comprising such compounds and use of the compounds as protease inhibitors.

## DETAILED DESCRIPTION OF THE INVENTION

The present invention provides at least one chemical entity selected from compounds of Formula I:

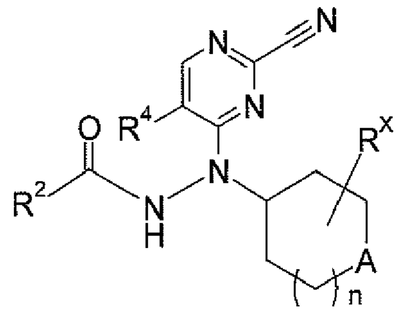

I

Wherein:

Either A represents $CH_2$ and n represents 0 or 1; or A represents -O- or $N(C(O)C_{1-3}$ alkyl) and n represents 1;

When A represents $CH_2$, $R^X$ represents an optional methyl substituent on any carbon atom of the ring to which it is attached, otherwise $R^X$ is absent;

$R^4$ represents halogen;

When A represents $CH_2$ or $N(C(O)C_{1-3}alkyl)$, $R^2$ represents -phenyl-$C_{1-3}$alkylene-X or -phenyl-$C_1$-$_3$alkylene-X-$R^J$ otherwise $R^2$ represents -phenyl-$C_{1-3}$alkylene-X-$R^J$;
wherein any phenyl group in $R^2$ is optionally substituted with at least one group independently selected from halogen or $CF_3$;

$R^J$ represents Z, $C_{1-4}$alkylene-Z or C(O)Z;

X and Z independently represent a monocyclic 4-, 5- or 6-membered, saturated hydrocarbon group containing one or two nitrogen atoms and optionally an oxygen atom, which is optionally substituted with a group selected from: $C_{1-4}$alkyl, OH and $C_{1-4}$ alkylOH;

and pharmaceutically acceptable derivatives thereof.

In one embodiment of the invention A represents $CH_2$. In another embodiment of the invention when A represents $CH_2$, n represents 0. In a further embodiment, when A represents $CH_2$, n represents 1. In a further embodiment, A represents -O- or $N(C(O)C_{1-3}alkyl)$.

In one embodiment of the invention when A represents $CH_2$, $R^X$ is absent. In another embodiment, when A represents $CH_2$, n represents 0 and $R^X$ represents methyl. In a further embodiment, when A represents $CH_2$, n represents 0 and $R^X$ represents methyl, the methyl group is in the 2- or 3-position relative to the point of attachment of the ring to the rest of the molecule. In another embodiment, when A represents $CH_2$, n represents 0 and $R^X$ represents methyl, the methyl group is in the 2-position relative to the point of attachment of the ring to the rest of the molecule. In a further embodiment, when A represents $CH_2$, n represents 0, $R^X$ represents methyl and the methyl group is in the 2-position relative to the point of attachment of the ring to the rest of the molecule, $R^X$ is in trans orientation relative to the point of attachment of the ring to the rest of the molecule. In another embodiment, when A represents $CH_2$, n represents 0 and $R^X$ represents methyl, the methyl group is in the 3-position relative to the point of attachment of the ring to the rest of the molecule.

In one embodiment of the invention, $R^4$ represents chlorine, bromine or iodine. In another embodiment, $R^4$ represents chlorine or bromine. In a further embodiment, $R^4$ represents bromine.

In one embodiment of the invention, when A represents $CH_2$ or $N(C(O)C_{1-3}alkyl)$, $R^2$ represents -phenyl-$C_{1-3}$alkylene-X-$R^J$, wherein phenyl is optionally substituted with one group selected from halogen or $CF_3$. In one embodiment, the alkylene group or groups in $R^2$ is methylene. In one embodiment, the phenyl group in $R^2$ is unsubstituted. In one embodiment, the groups directly bonded to the phenyl group in $R^2$ (excluding optional substituents) are in para

orientation relative to one another. In another embodiment, the groups directly bonded to the phenyl group in $R^2$ (excluding optional substituents) are in *meta* orientation relative to one another.

In one embodiment of the invention, $R^J$ represents Z. In another aspect, $R^J$ represents -$C_{1-3}$alkylene-Z. In a further aspect, $R^J$ represents -C(O)Z.

In one embodiment of the invention, X represents piperidine, piperazine or morpholine, each of which is optionally substituted. In another embodiment, X represents piperidine or piperazine, each of which is optionally substituted. In one embodiment, X is unsubstituted.

In one embodiment of the invention, Z represents piperidine, piperazine or morpholine, each of which is optionally substituted. In another embodiment, Z represents piperidine or piperazine, each of which is optionally substituted. In one embodiment, Z is unsubstituted.

In one embodiment of the invention, X is optionally substituted with $C_{1-4}$alkyl (for example methyl) or OH. In another embodiment, Z is optionally substituted with $C_{1-4}$alkyl. In a further embodiment, Z is optionally substituted with methyl.

The meaning of any functional group or substituent thereon at any one occurrence in Formula I or any subformula thereof, is independent of its meaning, or any other functional group's or substituent's meaning, at any other occurrence, unless stated otherwise.

It is to be understood that the present invention covers all combinations of the groups according to different aspects of the invention as described hereinabove.

**Terms and Definitions**

As used herein, the term "alkyl" as a group or a part of a group refers to a linear or branched alkyl group containing the indicated number of carbon atoms. Examples of such groups include methyl, ethyl, n-propyl, isopropyl, n-butyl, isobutyl, sec-butyl, *tert*-butyl, and the like.

As used herein, the term "alkylene" as a group or a part of a group refers to a linear or branched saturated hydrocarbon linker group containing the indicated number of carbon atoms. Examples of such groups include methylene, ethylene and the like.

As used herein, the term "halogen" or "halo" refers to a fluorine (fluoro), chlorine (chloro), bromine (bromo) or iodine (iodo) atom.

As used herein, the term "proteases" are enzymes that catalyze the cleavage of amide bonds of peptides and proteins by nucleophilic substitution at the amide bond, ultimately resulting in hydrolysis. Proteases include: cysteine proteases, serine proteases, aspartic proteases, and metalloproteases. Protease "inhibitors" bind more strongly to the enzyme than the substrate and in general are not subject to cleavage after enzyme catalyzed attack by the nucleophile. They therefore competitively prevent proteases from recognizing and hydrolysing natural substrates and thereby act as inhibitors.

In one aspect of the invention there is provided at least one chemical entity selected from the list:

N'-(5-bromo-2-cyano-4-pyrimidinyl)-N'-cyclopentyl-4-[(4-methyl-1-piperazinyl)methyl]benzohydrazide;
N'-(5-bromo-2-cyano-4-pyrimidinyl)-N'-[(1R,2S+1 S,2R)-2-methylcyclopentyl]-4-[(4-methyl-1-piperazinyl)methyl] benzohydrazide;
N'-(5-bromo-2-cyano-4-pyrim idinyl)-N'-[(1R,2R+1S,2S)-2-methylcyclopentyl]-4-[(4-methyl-1-piperazinyl)methyl] benzohydrazide;
N'-(5-bromo-2-cyano-4-pyrim idinyl)-N'-(3-methylcyclopentyl)-4-{[4-(4-methyl-1-piperazinyl)-1-piperidinyl]methyl} benzohydrazide;
N'-(5-bromo-2-cyano-4-pyrimidinyl)-N'-(3-methylcyclopentyl)-4-({4-[(1-methyl-4-piperidinyl)methyl]-1-piperazinyl} methyl)benzohydrazide;
N'-(5-bromo-2-cyano-4-pyrimidinyl)-N'-cyclopentyl-4-{[4-(4-morpholinyl)-1-piperidinyl]methyl}benzohydrazide;
N'-(5-bromo-2-cyano-4-pyrimidinyl)-N'-cyclopentyl-4-({4-[(1-methyl-4-piperidinyl)methyl]-1-piperazinyl}methyl) benzohydrazide;
N'-(5-bromo-2-cyano-4-pyrimidinyl)-N'-cyclopentyl-4-({4-[(1-methyl-3-piperidinyl)methyl]-1-piperazinyl}methyl) benzohydrazide;
N'-(5-bromo-2-cyano-4-pyrimidinyl)-N'-cyclopentyl-4-({4-[(4-methyl-1-piperazinyl)carbonyl]-1-piperidinyl}methyl) benzohydrazide;
N'-(5-bromo-2-cyano-4-pyrimidinyl)-N'-cyclopentyl-4-{[4-(4-methyl-1-piperazinyl)-1-piperidinyl]methyl}benzohydrazide;
N'-(5-bromo-2-cyano-4-pyrimidinyl)-N'-cyclopentyl-3-{[4-(4-methyl-1-piperazinyl)-1-piperidinyl]methyl}benzohydrazide;
N'-(5-bromo-2-cyano-4-pyrimidinyl)-N'-cyclopentyl-3-({4-[(4-methyl-1-piperazinyl)carbonyl]-1-piperidinyl}methyl)benzohydrazide;
N'-(5-bromo-2-cyano-4-pyrimidinyl)-N'-cyclopentyl-3-{[4-(4-morpholinyl)-1-piperidinyl]methyl}benzohydrazide;
N'-(5-bromo-2-cyano-4-pyrimidinyl)-N'-cyclopentyl-3-({4-[(1-methyl-3-piperidinyl)methyl]-1-piperazinyl}methyl) benzohydrazide;
N'-(5-bromo-2-cyano-4-pyrimidinyl)-N'-cyclopentyl-3-({4-[(1-methyl-4-piperidinyl)methyl]-1-piperazinyl}methyl) benzohydrazide;
N'-(5-bromo-2-cyano-4-pyrimidinyl)-N'-cyclohexyl-4-[(4-methyl-1-piperazinyl)methyl]benzohydrazide;
N'-(5-bromo-2-cyano-4-pyrimidinyl)-N'-cyclohexyl-4-{[4-(4-methyl-1-piperazinyl)-1-piperidinyl]methyl}benzohydrazide;
N'-(5-bromo-2-cyano-4-pyrimidinyl)-N'-cyclohexyl-4-{[4-(4-morpholinyl)-1-piperidinyl]methyl}benzohydrazide;
N'-(5-bromo-2-cyano-4-pyrimidinyl)-N'-cyclohexyl-4-({4-[(1-methyl-4-piperidinyl)methyl]-1-piperazinyl}methyl)benzohydrazide;
N'-(5-bromo-2-cyano-4-pyrimidinyl)-N'-cyclohexyl-3-{[4-(4-methyl-1-piperazinyl)-1-piperidinyl]methyl}benzohydrazide;
N'-(5-bromo-2-cyano-4-pyrimidinyl)-N'-cyclohexyl-4-[(4-hydroxy-1-piperidinyl)methyl]benzohydrazide;
N'-(5-bromo-2-cyano-4-pyrimidinyl)-N'-cyclohexyl-4-({4-[(4-methyl-1-piperazinyl)carbonyl]-1-piperidinyl}methyl) benzohydrazide;
N'-(5-bromo-2-cyano-4-pyrimidinyl)-N'-cyclohexyl-3-({4-[(4-methyl-1-piperazinyl)carbonyl]-1-piperidinyl}methyl) benzohydrazide;
N'-(5-bromo-2-cyano-4-pyrimidinyl)-N'-cyclohexyl-3-{[4-(4-morpholinyl)-1-piperidinyl]methyl}benzohydrazide;
N'-(5-bromo-2-cyano-4-pyrimidinyl)-N'-cyclohexyl-4-({4-[(1-methyl-3-piperidinyl)methyl]-1-piperazinyl}methyl)benzohydrazide;
N'-(5-bromo-2-cyano-4-pyrimidinyl)-N'-(3-methylcyclopentyl)-4-[(4-methyl-1-piperazinyl)methyl]benzohydrazide;
N'-(5-bromo-2-cyano-4-pyrimidinyl)-N'-cyclohexyl-3-({4-[(1-methyf-3-piperidinyl)methyl]-1-piperazinyl}methyl)benzohydrazide;
N'-(5-bromo-2-cyano-4-pyrimidinyl)-N'-cyclohexyl-3-({4-[(1-methyl-4-piperidinyl)methyl]-1-piperazinyl}methyl)benzohydrazide;
N'-(5-bromo-2-cyano-4-pyrimidinyl)-4-{[4-(4-methyl-1-piperazinyl)-1-piperidinyl]methyl}-N'-(tetrahydro-2H-pyran-4-yl)benzohydrazide;
N'-(5-bromo-2-cyano-4-pyrimidinyl)-4-({4-[(1-methyl-4-piperidinyl)methyl]-1-piperazinyl}methyl)-N'-(tetrahydro-2H-pyran-4-yl)benzohydrazide;
N'-(5-bromo-2-cyano-4-pyrimidinyl)-4-({4-[(1-methyl-3-piperidinyl)methyl]-1-piperazinyl}methyl)-N'-(tetrahydro-2H-pyran-4-yl)benzohydrazide;
N'-(5-bromo-2-cyano-4-pyrimidinyl)-4-({4-[(4-methyl-1-piperazinyl)carbonyl]-1-piperidinyl}methyl)-N'-(tetrahydro-2H-pyran-4-yl) benzohydrazide;
N'-(5-bromo-2-cyano-4-pyrimidinyl)-4-{[3-(1-pyrrolidinyl)-1-azetidinyl]methyl}-N'-(tetrahydro-2H-pyran-4-yl)benzohydrazide;
N'-(5-chloro-2-cyano-4-pyrimidinyl)-4-{[4-(4-methyl-1-piperazinyl)-1-piperidinyl]methyl}-N'-(tetrahydro-2H-pyran-4-yl)benzohydrazide;
N'-(5-chloro-2-cyano-4-pyrimidinyl)-4-({4-[(1-methyl-4-piperidinyl)methyl]-1-piperazinyl}methyl)-N'-(tetrahydro-2H-pyran-4-yl)benzohydrazide;
N'-(5-chloro-2-cyano-4-pyrimidinyl)-4-({4-[(4-methyl-1-piperazinyl)carbonyl]-1-piperidinyl}methyl)-N'-(tetrahydro-2H-pyran-4-yl)benzohydrazide;
N'-(1-acetyl-4-piperidinyl)-N'-(5-bromo-2-cyano-4-pyrimidinyl)-4-[(4-methyl-1-piperazinyl)methyl]benzohydrazide;
N'-(1-acetyl-4-piperidinyl)-N'-(5-bromo-2-cyano-4-pyrimidinyl)-4-{[4-(4-methyl-1-piperazinyl)-1-piperidinyl]methyl} benzohydrazide;

and pharmaceutically acceptable derivatives thereof.

As used herein, the term "pharmaceutically acceptable derivative", means any pharmaceutically acceptable salt, solvate, or prodrug e.g. ester or carbamate of a compound of Formula I, which upon administration to the recipient is capable of providing (directly or indirectly) a compound of Formula I, or an active metabolite or residue thereof. Such derivatives are recognizable to those skilled in the art, without undue experimentation. Nevertheless, reference is made to the teaching of Burger's Medicinal Chemistry and Drug Discovery, 5th Edition, Vol 1: Principles and Practice, which is incorporated herein by reference to the extent of teaching such derivatives. In one aspect of the invention pharmaceutically acceptable derivatives are salts, solvates, esters and carbamates. In another aspect of the invention pharmaceutically acceptable derivatives are salts, solvates and esters. In a further aspect, pharmaceutically acceptable derivatives are salts and solvates.

The compounds of the present invention may be in the form of and/or may be administered as a pharmaceutically

acceptable salt. Indeed, in certain embodiments of the invention, pharmaceutically acceptable salts of the compounds according to Formula I, may be preferred over the respective free base or free acid because such salts impart greater stability or solubility to the molecule thereby facilitating formulation into a dosage form. Accordingly, the invention is further directed to pharmaceutically acceptable salts of the compounds according to Formula I.

As used herein, the term "pharmaceutically acceptable salts" refers to salts that retain the desired biological activity of the subject compound and exhibit minimal undesired toxicological effects. For a review on suitable salts see Berge et al, J. Pharm. Sci., 1977, 66, 1-19. The term "pharmaceutically acceptable salts" includes both pharmaceutically acceptable acid addition salts and pharmaceutically acceptable base addition salts. These pharmaceutically acceptable salts may be prepared *in situ* during the final isolation and purification of the compound, or by separately reacting the purified compound in its free acid or free base form with a suitable base or acid, respectively. The salt may precipitate from solution and be collected by filtration or may be recovered by evaporation of the solvent.

A pharmaceutically acceptable acid addition salt can be formed by reaction of a compound of Formula I, with a suitable inorganic or organic acid (such as hydrobromic, hydrochloric, sulfuric, sulfamic, nitric, phosphoric, succinic, maleic, hydroxymaleic, acrylic, formic, acetic, hydroxyacetic, phenylacetic, butyric, isobutyric, propionic, fumaric, citric, tartaric, lactic, mandelic, benzoic, o-acetoxybenzoic, chlorobenzoic, methylbenzoic, dinitrobenzoic, hydroxybenzoic, methoxybenzoic salicylic, glutamaic, stearic, ascorbic, palmitic, oleic, pyruvic, pamoic, malonic, lauric, glutaric aspartic, p-toluenesulfonic, benzenesulfonic, methanesulfonic, ethanesulfonic, 2-hydroxyethanesulfonic, naphthalenesulfonic (e.g. 2-naphthalenesulfonic), p-aminobenzenesulfonic (i.e. sulfanilic), hexanoic, heptanoic, or phthalic acid), optionally in a suitable solvent such as an organic solvent, to give the salt which is usually isolated for example by crystallisation and filtration. A pharmaceutically acceptable acid addition salt of a compound of Formula I, can comprise or be for example a hydrobromide, hydrochloride, hydroiodide, sulfate, bisulfate, nitrate, phosphate, hydrogen phosphate, succinate, maleate, malate, formate, acetate, trifluoroacetate, saccharate, propionate, fumarate, citrate, tartrate, lactate, benzoate, salicylate, glutamate, aspartate, p-toluenesulfonate, benzenesulfonate, methanesulfonate, ethanesulfonate, naphthalenesulfonate (e.g. 2- naphthalenesulfonate), methanesulphonic, ethanesulphonic, p-toluenesulphonic, isethionate or hexanoate salt. In one embodiment there is provided the trifluoroacetic acid salts of the compounds of the invention. In another embodiment there is provided the hydrochloric acid salts of the compounds of the invention.

A pharmaceutically acceptable base addition salt can be formed by reaction of a compound of Formula with a suitable inorganic or organic base (e.g. ammonia, triethylamine, ethanolamine, triethanolamine, choline, arginine, lysine or histidine), optionally in a suitable solvent such as an organic solvent, to give the base addition salt which is usually isolated for example by crystallisation and filtration. Pharmaceutically acceptable base salts include ammonium salts and salts with organic bases, including salts of primary, secondary and tertiary amines, including aliphatic amines, aromatic amines, aliphatic diamines, and hydroxy alkylamines, such as methylamine, ethylamine, isopropylamine, diethylamine, ethylenediamine, ethanolamine, trimethylamine, dicyclohexyl amine, diethanolamine, cyclohexylamine and N-methyl-D-glucamine. Other suitable pharmaceutically acceptable base salts include pharmaceutically acceptable metal salts, for example pharmaceutically acceptable alkali-metal or alkaline-earth-metal salts such as hydroxides, carbonates and bicarbonates of sodium, potassium, lithium, calcium, magnesium, aluminium, and zinc; in particular pharmaceutically acceptable metal salts of one or more carboxylic acid moieties that may be present in the compound of Formula I.

Other non-pharmaceutically acceptable salts, for example oxalates may be used, for example in the isolation of compounds of the invention.

The invention includes within its scope all possible stoichiometric and non-stoichiometric forms of the salts of the compounds of Formula I.

As used herein, the term "compounds of the invention" means the compounds according to Formula I, and the pharmaceutically acceptable derivatives thereof. The term "a compound of the invention" means any one of the compounds of the invention as defined above.

As used herein, the term "at least one chemical entity" means at least one chemical substance chosen from the group of compounds consisting of compounds of Formula 1, and pharmaceutically acceptable derivatives thereof.

The compounds of the invention may exist as solids or liquids, both of which are included in the invention. In the solid state, the compounds of the invention may exist as either amorphous material or in crystalline form, or as a mixture thereof. It will be appreciated that solvates of the compounds of the invention may be formed wherein solvent molecules are incorporated into the crystalline lattice during crystallisation. Solvates may involve non-aqueous solvents such as ethanol, isopropanol, DMSO, acetic acid, ethanolamine, and ethyl acetate, or they may involve water as the solvent that is incorporated into the crystalline lattice. Solvates wherein water is the solvent that is incorporated into the crystalline lattice are typically referred to as "hydrates." The invention includes all such solvates.

It will be further appreciated that all crystalline forms, polymorphs, geometric isomers, stereoisomers (including enantiomers and diastereomers) and tautomers of the compounds of the invention, or mixtures thereof, are contemplated to be within the scope of the present invention. Unless otherwise specfied, for compounds which posesses stereocentres and which can therefore form enantiomers, the compound contains a 1:1 mixture of enantiomers, i.e. a racemic mixture of enantiomers.

According to another aspect of the invention there is provided at least one chemical entity selected from a compound of Formula I, or a pharmaceutically acceptable derivative thereof for use in human or veterinary medical therapy.

The compounds of the invention are cysteine protease inhibitors, such as inhibitors of cysteine proteases of the papain superfamily, for example of the falcipain family, including falcipain-2 or falcipain-3. The compounds of the invention are also inhibitors of cysteine proteases of the papain superfamily, for example those of the cathepsin family such as cathepsins K, L, S and B.

The compounds of the invention may be useful for treating conditions in which cysteine proteases are implicated, including infections by *Plasmodium falciparum* which is the most virulent malaria-causing parasite, and by *Plasmodium vivax, Pneumocystis carinii, Trypsanoma cruzi, Trypsanoma brucei,* and *Crithidia fusiculata;* as well as in treating conditions such as schistosomiasis, malaria, cancer, tumour invasion and tumor metastasis, metachromatic leukodystrophy, muscular dystrophy, amytrophy, chronic obstructive pulmonary disorder (COPD), atherosclerosis; and especially conditions in which cathepsin K is implicated, including diseases of excessive bone or cartilage loss and other bone and joint diseases such as osteoporosis, bone metastasis, gingival disease (including gingivitis and periodontitis), arthritis (including osteoarthritis and rheumatoid arthritis), Paget's disease; hypercalcemia of malignancy, and metabolic bone disease. In addition, metastatic neoplastic cells also typically express high levels of proteolytic enzymes that degrade the surrounding matrix, and certain tumors and metastatic neoplasias may be effectively treated with the compounds of the invention. Accordingly, the invention is directed to methods of treating such conditions.

In one aspect of the invention, there is provided at least one chemical entity selected from a compound of Formula I, or a pharmaceutically acceptable derivative thereof, for use in the treatment of a condition mediated by inhibition of a cysteine protease, particularly inhibition of a cysteine protease of the papain superfamily such as those of the falcipain family, including falcipain-2 or falcipain-3, for example malaria.

In another aspect of the invention, there is provided at least one chemical entity selected from a compound of Formula I, or a pharmaceutically acceptable derivative thereof for use in the treatment of a condition mediated by inhibition of a cysteine protease, particularly inhibition of a cysteine protease of the papain superfamily, such as those of the cathepsin family for example cathepsins K, L, S and B, i) in one embodiment cathepsin K, for example conditions characterised by excessive bone loss such as osteoporosis and bone metastasis, and other bone and joint diseases such as osteoarthritis, or ii) in another embodiment cathepsin L or S, for example pancreatic cancer.

In another aspect of the invention there is provided the use of at least one chemical entity selected from a compound of Formula I, or a pharmaceutically acceptable derivative thereof in the manufacture of a medicament for the treatment of a condition mediated by inhibition of a cysteine protease, particularly inhibition of a cysteine protease of the papain superfamily such as those of the falcipain family, including falcipain-2 or falcipain-3, for example malaria.

In a further aspect of the invention there is provided the use of at least one chemical entity selected from a compound of Formula I, or a pharmaceutically acceptable derivative thereof in the manufacture of a medicament for the treatment of a condition mediated by inhibition of a cysteine protease, particularly inhibition of a cysteine protease of the papain superfamily, such as those of the cathepsin family, for example cathepsins K, L, S and B, i) in one embodiment cathepsin K, for example conditions characterised by excessive bone loss such as osteoporosis and bone metastasis, and other bone and joint diseases such as osteoarthritis, or ii) in another embodiment cathepsin L or S, for example pancreatic cancer.

In another aspect of the invention there is provided a method for the treatment of a human or animal subject suffering from a condition mediated by inhibition of a cysteine protease, particularly inhibition of a cysteine protease of the papain superfamily such as those of the falcipain family, including falcipain-2 or falcipain-3, for example malaria, which method comprises administering an effective amount of at least one chemical entity selected from a compound of Formula I, or a pharmaceutically acceptable derivative thereof or a pharmaceutical composition comprising at least one chemical entity selected from a compound of Formula I, or a pharmaceutically acceptable derivative thereof.

In another aspect of the invention there is provided a method for the treatment of a human or animal subject suffering from a condition mediated by inhibition of a cysteine protease, particularly inhibition of a cysteine protease of the papain superfamily, such as those of the cathepsin family, for example cathepsins K, L, S and B, i) in one embodiment cathepsin K, for example conditions characterised by excessive bone loss such as osteoporosis and bone metastasis, and other bone and joint diseases such as osteoarthritis, or ii) in another embodiment cathepsin L or S, for example pancreatic cancer, which method comprises administering an effective amount of at least one chemical entity selected from a compound of Formula I, or a pharmaceutically acceptable derivative thereof or a pharmaceutical composition comprising at least one chemical entity selected from a compound of Formula I, or a pharmaceutically acceptable derivative thereof.

The compounds of the invention are cysteine protease inhibitors and can be useful in the treatment of a condition mediated by inhibition of a cysteine protease, particularly inhibition of a cysteine protease of the papain superfamily such as those of the falcipain family, including falcipain-2 or falcipain-3, for example in the treatment of malaria, or those of the cathepsin family for example cathepsins K, L, S and B, i) in one embodiment cathepsin K, for example conditions

characterised by excessive bone loss such as osteoporosis and bone metastasis, and other bone and joint diseases such as osteoarthritis, or ii) in another embodiment cathepsin L or S, for example pancreatic cancer. Accordingly, the invention is further directed to pharmaceutical compositions comprising at least one chemical entity selected from a compound of Formula I, or a pharmaceutically acceptable derivative thereof.

As used herein "excessive bone loss" is a disease state in which the normal balance between bone resorption and formation is disrupted, and there is a net loss of bone at each cycle. Diseases which are characterised by excessive bone loss include, but are not limited to, osteoporosis and gingival diseases, excessive cartilage or matrix degradation including osteoarthritis and rheumatoid arthritis.

The methods of treatment of the invention comprise administering a safe and effective amount of at least one chemical entity selected from a compound of Formula I, or a pharmaceutically acceptable derivative thereof, or a pharmaceutical composition containing at least one chemical entity selected from a compound of Formula I, or a pharmaceutically acceptable derivative thereof, to a patient in need thereof.

As used herein, "treatment" means: (1) the amelioration or prevention of the condition being treated or one or more of the biological manifestations of the condition being treated, (2) the interference with (a) one or more points in the biological cascade that leads to or is responsible for the condition being treated or (b) one or more of the biological manifestations of the condition being treated, or (3) the alleviation of one or more of the symptoms or effects associated with the condition being treated. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof.

As used herein, "safe and effective amount" means an amount of the compound sufficient to significantly induce a positive modification in the condition to be treated but low enough to avoid serious side effects (at a reasonable benefit/risk ratio) within the scope of sound medical judgment. A safe and effective amount of a compound of the invention will vary with the particular compound chosen (e.g. depending on the potency, efficacy, and half-life of the compound); the route of administration chosen; the condition being treated; the severity of the condition being treated; the age, size, weight, and physical condition of the patient being treated; the medical history of the patient to be treated; the duration of the treatment; the nature of concurrent therapy; the desired therapeutic effect; and like factors, but can nevertheless be routinely determined by the skilled artisan.

As used herein, "patient" refers to a human or other animal.

The compounds of the invention may be administered by any suitable route of administration, including both systemic administration and topical administration. Systemic administration includes oral administration, parenteral administration, transdermal administration, rectal administration, and administration by inhalation. Parenteral administration refers to routes of administration other than enteral, transdermal, or by inhalation, and is typically by injection or infusion. Parenteral administration includes intravenous, intramuscular, and subcutaneous injection or infusion. Inhalation refers to administration into the patient's lungs whether inhaled through the mouth or through the nasal passages. Topical administration includes application to the skin as well as intraocular, optic, intravaginal, and intranasal administration.

The compounds of the invention may be administered once or according to a dosing regimen wherein a number of doses are administered at varying intervals of time for a given period of time. For example, doses may be administered one, two, three, or four times per day. Doses may be administered until the desired therapeutic effect is achieved or indefinitely to maintain the desired therapeutic effect. Suitable dosing regimens for a compound of the invention depend on the pharmacokinetic properties of that compound, such as absorption, distribution, and half-life, which can be determined by the skilled artisan. In addition, suitable dosing regimens, including the duration such regimens are administered, for a compound of the invention depend on the condition being treated, the severity of the condition being treated, the age and physical condition of the patient being treated, the medical history of the patient to be treated, the nature of concurrent therapy, the desired therapeutic effect, and like factors within the knowledge and expertise of the skilled artisan. It will be further understood by such skilled artisans that suitable dosing regimens may require adjustment given an individual patient's response to the dosing regimen or over time as individual patient needs change.

Typical daily dosages may vary depending upon the particular route of administration chosen. Typical daily dosages for oral administration range from about 0.01 to about 25 mg/kg, in one embodiment from about 0.1 to about 14 mglkg. Typical daily dosages for parenteral administration range from about 0.001 to about 10 mg/kg; in one embodiment from about 0.01 to about 6 mg/kg.The compounds of Formula I, may also be used in combination with other therapeutic agents. The invention thus provides, in a further aspect, a combination comprising a compound of Formula I, or a pharmaceutically acceptable derivative thereof together with a further therapeutic agent. When a compound of Formula I, or a pharmaceutically acceptable derivative thereof is used in combination with a second therapeutic agent active against the same disease state the dose of each compound may differ from that when the compound is used alone. Appropriate doses will be readily appreciated by those skilled in the art. It will be appreciated that the amount of a compound of the invention required for use in treatment will vary with the nature of the condition being treated and the age and the condition of the patient and will be ultimately at the discretion of the attendant physician or veterinarian.

The compounds of the present invention may be used alone or in combination with one or more additional active agents, such as other inhibitors of cysteine and serine proteases, antimalarial drugs or drugs to treat excessive bone loss.

Such other active agents include inhibitors of bone resorption or other bone diseases, for example bisphosphonates (i.e., allendronate, risedronate, etidronate, and ibandronate), hormone replacement therapy, anti-estrogens, calcitonin, and anabolic agents such as bone morphogenic protein, iproflavone, and PTH. In the alternative, such other active agents include antimalarial drugs, such as folates (e.g. chloroquine, mefloquine, primaquine pyrimethamine, quinine artemisinin, halofantrine, doxycycline, amodiquine, atovaquine [atovaquone], tafenoquine) and antifolates (e.g. dapsone, proguanil, sulfadoxine, pyrimethamine, chlorcycloguanil, cycloguanil) or antibacterial drugs such as azithromycin, doxycycline, ciprofloxacin and clindamycin. In another alternative, such other active agents include anti-cancer agents.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations by any convenient route.

When administration is sequential, either the compound of the present invention or the second therapeutic agent may be administered first. When administration is simultaneous, the combination may be administered either in the same or different pharmaceutical composition. When combined in the same formulation it will be appreciated that the two compounds must be stable and compatible with each other and the other components of the formulation. When formulated separately they may be provided in any convenient formulation, conveniently in such manner as are known for such compounds in the art.

**Compositions**

The compounds of the invention will normally, but not necessarily, be formulated into pharmaceutical compositions prior to administration to a patient. In one aspect, the invention is directed to pharmaceutical compositions comprising a compound of the invention. In another aspect the invention is directed to pharmaceutical compositions comprising a compound of the invention and a pharmaceutically acceptable carrier and/or excipient. The carrier and/or excipient must be "acceptable" in the sense of being compatible with the other ingredients of the formulation and not deleterious to the receipient thereof.

The pharmaceutical compositions of the invention may be prepared and packaged in bulk form wherein a safe and effective amount of a compound of the invention can be extracted and then given to the patient such as with powders or syrups. Alternatively, the pharmaceutical compositions of the invention may be prepared and packaged in unit dosage form wherein each physically discrete unit contains a safe and effective amount of a compound of the invention. When prepared in unit dosage form, the pharmaceutical compositions of the invention typically contain from about 0.5 mg to about 1750 mg, e.g. from about 5 mg to about 1000 mg for oral dosage forms and from about 0.05 mg to about 700 mg, e.g. from about 0.5 mg to about 500 mg for parenteral dosage forms.

The pharmaceutical compositions of the invention typically contain one compound of the invention. However, in certain embodiments, the pharmaceutical compositions of the invention contain more than one compound of the invention. For example, in certain embodiments the pharmaceutical compositions of the invention contain two compounds of the invention. In addition, the pharmaceutical compositions of the invention may optionally further comprise one or more additional pharmaceutically active compounds. Conversely, the pharmaceutical compositions of the invention typically contain more than one pharmaceutically acceptable excipient. However, in certain embodiments, the pharmaceutical compositions of the invention contain one pharmaceutically acceptable excipient.

As used herein, the term "pharmaceutically acceptable" means suitable for pharmaceutical use.

The compound of the invention and the pharmaceutically acceptable excipient or excipients will typically be formulated into a dosage form adapted for administration to the patient by the desired route of administration. For example, dosage forms include those adapted for (1) oral administration such as tablets, capsules, caplets, pills, troches, powders, syrups, elixers, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as aerosols and solutions; and (6) topical administration such as creams, ointments, lotions, solutions, pastes, sprays, foams, and gels.

Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be

chosen for their ability to facilitate the carrying or transporting the compound or compounds of the invention once administered to the patient from one organ, or portion of the body, to another organ, or portion of the body. Certain pharmaceutically acceptable excipients may be chosen for their ability to enhance patient compliance.

Suitable pharmaceutically acceptable excipients include the following types of excipients: binders, disintegrants, lubricants, glidants, granulating agents, coating agents, wetting agents, solvents, co-solvents, suspending agents, emulsifiers, sweeteners, flavoring agents, flavor masking agents, coloring agents, anticaking agents, humectants, chelating agents, plasticizers, viscosity increasing agents, antioxidants, preservatives, stabilizers, surfactants, and buffering agents. The skilled artisan will appreciate that certain pharmaceutically acceptable excipients may serve more than one function and may serve alternative functions depending on how much of the excipient is present in the formulation and what other ingredients are present in the formulation.

Skilled artisans possess the knowledge and skill in the art to enable them to select suitable pharmaceutically acceptable excipients in appropriate amounts for use in the invention. In addition, there are a number of resources that are available to the skilled artisan which describe pharmaceutically acceptable excipients and may be useful in selecting suitable pharmaceutically acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The pharmaceutical compositions of the invention are prepared using techniques and methods known to those skilled in the art. Some of the methods commonly used in the art are described in Remington's Pharmaceutical Sciences (Mack Publishing Company).

In one aspect, the invention is directed to a solid or liquid oral dosage form such as a liquid, tablet, lozenge or a capsule, comprising a safe and effective amount of a compound of the invention and a carrier. The carrier may be in the form of a diluent or filler. Suitable diluents and fillers in general include lactose, sucrose, dextrose, mannitol, sorbitol, starch (e.g. corn starch, potato starch, and pre-gelatinized starch), cellulose and its derivatives (e.g. microcrystalline cellulose), calcium sulfate, and dibasic calcium phosphate. A liquid dosage form will generally consist of a suspension or solution of the compound or salt in a liquid carrier for example, ethanol, olive oil, glycerine, glucose (syrup) or water (e.g. with an added flavouring, suspending, or colouring agent). Where the composition is in the form of a tablet or lozenge, any pharmaceutical carrier routinely used for preparing solid formulations may be used.

Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers or a semi solid e.g. mono di-glycerides of capric acid, Gelucire™ and Labrasol™, or a hard capsule shell e.g gelatin. Where the composition is in the form of a soft shell capsule e.g. gelatin, any pharmaceutical carrier routinely used for preparing dispersions or suspensions may be considered, for example aqueous gums or oils, and may be incorporated in a soft capsule shell.

An oral solid dosage form may further comprise an excipient in the form of a binder. Suitable binders include starch (e.g. corn starch, potato starch, and pre-gelatinized starch), gelatin, acacia, sodium alginate, alginic acid, tragacanth, guar gum, povidone, and cellulose and its derivatives (e.g. microcrystalline cellulose). The oral solid dosage form may further comprise an excipient in the form of a disintegrant. Suitable disintegrants include crospovidone, sodium starch glycolate, croscarmelose, alginic acid, and sodium carboxymethyl cellulose. The oral solid dosage form may further comprise an excipient in the form of a lubricant. Suitable lubricants include stearic acid, magnesium stearate, calcium stearate, and talc.

There is further provided by the present invention a process of preparing a pharmaceutical composition, which process comprises mixing at least one compound of Formula I, or a pharmaceutically acceptable derivative thereof, together with a pharmaceutically acceptable carrier and/or excipient.

Preparations for oral administration may be suitably formulated to give controlled/extended release of the active compound.

All publications, including but not limited to patents and patent applications cited in this specification are herein incorporated by reference as if each individual publication were specifically and individually indicated to be incorporated by reference as though fully set forth.

## Abbreviations

In describing the invention, chemical elements are identified in accordance with the Periodic Table of the Elements. Abbreviations and symbols utilized herein are in accordance with the common usage of such abbreviations and symbols by those skilled in the chemical arts. The following abbreviations are used herein:

| | |
|---|---|
| ACN | acetonitrile |
| AcOEt | ethyl acetate |

(continued)

| | |
|---|---|
| AcOH | acetic acid |
| AFC | 7-amido-4-trifluoromethylcoumarin |
| AMC | 7-amido-4-methylcoumarin |
| anh. | anhydrous |
| aq. | aqueous |
| cat. | catalytic |
| $CDCl_3$ | deuterated chloroform |
| CHAPS | 3-[(3-cholamidopropyl)dimethylammonio]-1-propanesulfonate |
| CYS | cysteine |
| DABCO | 1,4-diazabicyclo[2.2.2]octane |
| DCM | dichloromethane |
| DIPEA | diisopropylamine |
| DMF | dimethylformamide |
| DMSO-d6 | deuterated dimethylsulfoxide |
| DMSO | dimethylsulfoxide |
| DTT | dithiothreitol |
| E64 | *trans*-epoxysuccinyl-L-leucylamido(4-guanidino)butane |
| EDCI | N-(3-dimethylaminopropyl)-N'-ethylcarbodiimide |
| EDTA | (ethyfenedinitrilo)tetraacetic acid |
| ES+ MS | Positive Electrospray mass spectrometry |
| ES- MS | Negative Electrospray mass spectrometry |
| EtOH | ethanol |
| h | hours |
| H-D-VLR-AFC | HD-Valyl-Leucyl-Arginyl-7-Amido-4-trifluoromethylcoumarin |
| Hex | hexane |
| HPLC | high performance liquid chromatography |
| i-PrOH | isopropanol |
| kg | kilogram(s) |
| KQKLR-AMC | N-Acetyl-Lysyl-Glutaminyl-Lysyl-Leucyl-Arginyl-7-Amido-4-methylcoumarin |
| MeOH | methanol |
| MES | 2-(N-morpholino)ethanesulfonic acid |
| min | minutes |
| mg | miligram(s) |
| nM | Nanomolar |
| NMR | Nuclear Magnetic Resonance spectroscopy |
| TEA | triethylamine |
| TFA | trifluoroacetic acid |
| THF | tetrahydrofuran |
| Z-LR-AMC | benzyloxycarbonyl-leucyl-arginyl-7-amido-4-methylcoumarin |

**Compound Preparation**

The general procedures used to synthesise the compounds of Formula I are described in reaction Schemes 1-10 and are illustrated in the Examples.

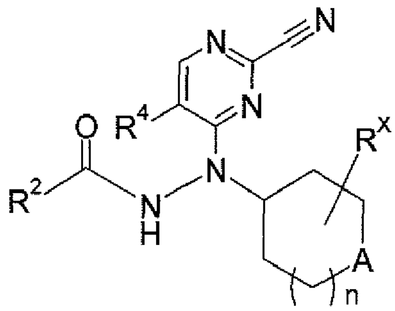

I

Compounds of Formula I may be prepared from a reaction between compounds of Formula II, wherein A, n, $R^4$ and $R^X$ are as defined for Formula I, compounds of Formula III, wherein Hal is chlorine or bromine, and compounds of Formula IV, wherein X and $R^J$ are as defined for Formula I, according to Scheme 1. Compounds II are reacted with compounds III in the presence of a suitable base, such as DIPEA, in a suitable solvent such as THF, followed by the addition of compounds IV, to give compounds I.

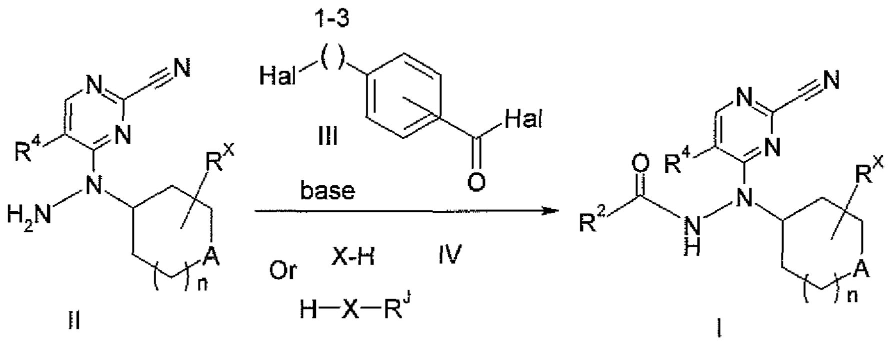


Scheme 1

Alternatively, compounds of Formula I may be prepared from a reaction between compounds of Formula V, wherein A, n, $R^4$ and $R^X$ are as defined for Formula I and Hal is chlorine or bromine, and compounds of Formula IV, according to Scheme 2. Compounds V are reacted with IV in the presence of a suitable base, such as DIPEA, in a suitable solvent, such as THF or ACN.

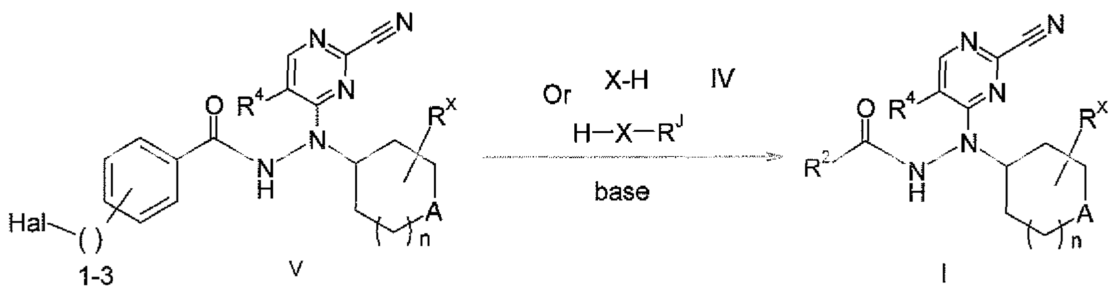


Scheme 2

Alternatively, compounds of Formula I may be prepared from a reaction between compounds of Formula II and compounds of Formula VI, wherein X and $R^J$ are as defined for Formula I and Hal is chlorine or bromine, according to Scheme 3. Compounds II are reacted with compounds VI in the presence of a base, for example an inorganic base such as potassium carbonate, or an organic base such as an amine, e.g. DIPEA.

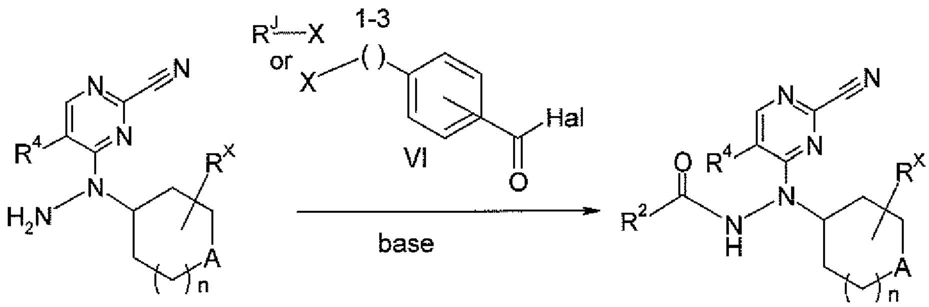


II I

Scheme 3

Compounds of Formula III are either commercially available, or they may be synthesised from the corresponding benzoic acid by reaction with a suitable reagent such as thionyl chloride (to make the acid chloride).

Compounds VI may be synthesised starting from a reaction between the corresponding methyl ester of the benzoic acid precursor of compounds of Formula III and compounds IV in the presence of a suitable base such as potassium carbonate in a suitable solvent such as DMF, followed by conversion of the methyl ester moiety to a benzoic acid moiety using a suitable reagent such as lithium hydride in a suitable solvent such as MeOH, followed by conversion to the acid halide using a suitable reagent such as thionyl chloride (to make the acid chloride), resulting in compounds VI.

Compounds of Formula V may be prepared from a reaction between compounds of Formula II and compounds of Formula III, according to Scheme 4. Compounds II are reacted with compounds III in the presence of a suitable base such as DIPEA, in a suitable solvent such as THF or DCM.

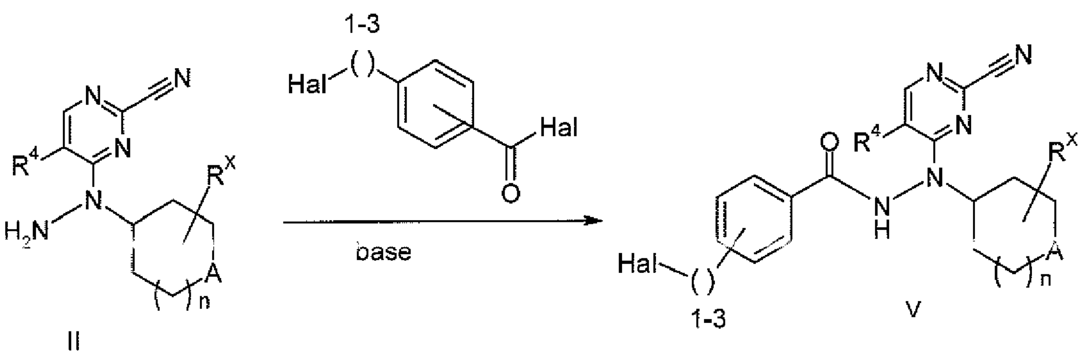


II V

Scheme 4

Compounds of Formula II may be prepared from compounds of Formula VII, wherein A, n, $R^4$ and $R^X$ are as defined for Formula I, according to Scheme 4 by deprotection in the presence of a suitable acid such as trifluoroacetic acid or TsOH, in a suitable solvent such as ACN.

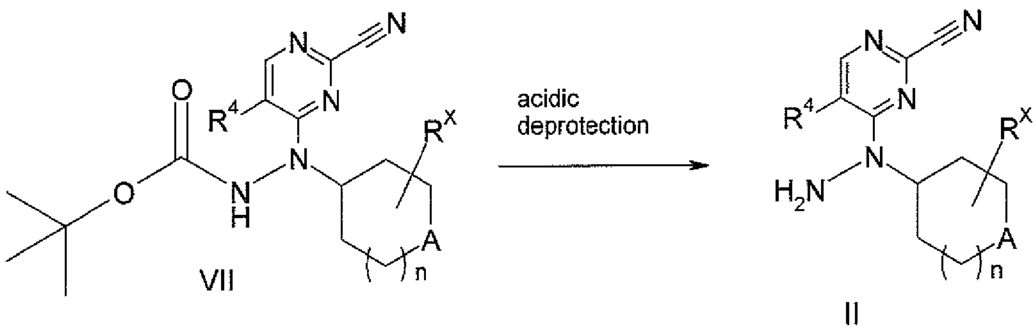


VII

II

Scheme 5

Compounds of Formula VII may be prepared from compounds of Formula VIII, wherein A, n, $R^4$ and $R^X$ are as defined above for Formula I, according to Scheme 6, by cyanation, by displacement of the chloro substituent of compounds of Formula VIII using a variety of conditions, for example by treatment with potassium or sodium cyanide in the presence of a suitable base such as DABCO in a suitable solvent such as DMSO.

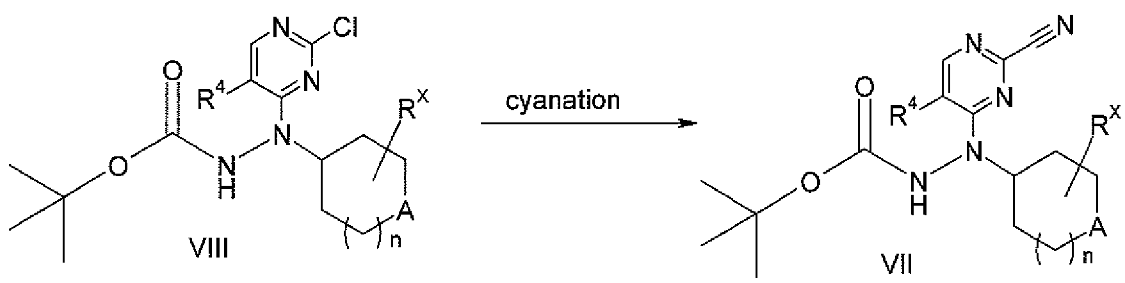


VIII

VII

Scheme 6

Compounds of Formula VIII may be prepared from a reaction between compounds of Formula IX, wherein A, n and $R^X$ are as defined above for Formula I, and compounds of Formula X, wherein $R^4$ is as defined above for Formula I, according to Scheme 7. Compounds IX are reacted with compounds X (commercially available from FLUKA or SIGMA), in a suitable solvent such as EtOH, for example at room temperature for 3-4 days, for example according to the literature procedure given in Luo G. et al., (2002) Tetrahedron Letters, 43 (33), 5739-5742. Alternatively, compounds IX are reacted with compounds X in the presence of a suitable base such as DIPEA, in the presence of a suitable solvent, such as i-PrOH.

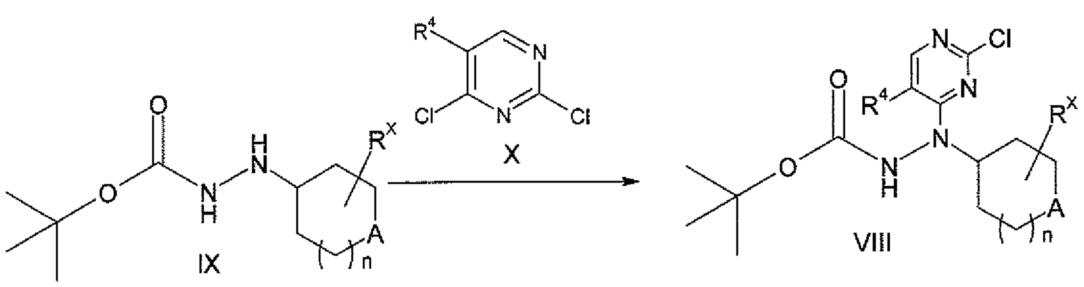

IX

X

VIII

Scheme 7

Compounds of Formula IX may be prepared from the compound of Formula XI by a reductive amination reaction with a ketone XII, according to Scheme 8. The compound of Formula XI, *tert*-butyl carbazate, is commercially available (ALDRICH). Compounds of Formula XII are also commercially available. Reductive amination of the compound XII with the compound XI is carried out in the presence of a suitable reducing agent such as hydrogen, and a suitable catalyst

such as platinum or palladium or platinum oxide, or alternatively using $NaBH_3CN$ in the presence of an acid such as AcOH, in a suitable solvent such as i-PrOH, EtOH or a mixture thereof, for example according to the literature procedures given in Hilpert, H. (2001) Tetrahedron, 57, 7675-7683 or Dyker. H. et al, (2001) J. Org. Chem. 66, 3760-3766).

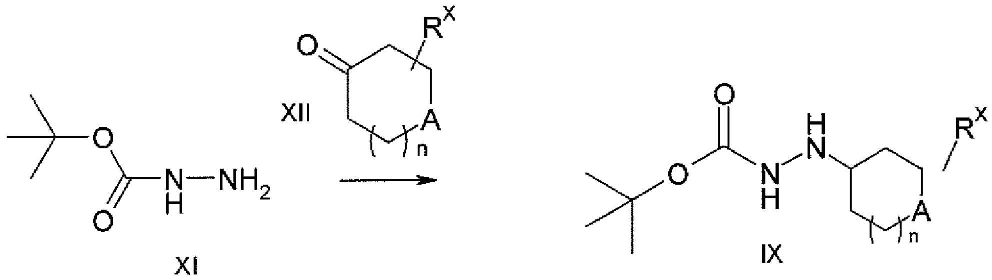

Scheme 8

Compounds of Formula I may be prepared from compounds of Formula XIII, wherein A, n, $R^2$, $R^4$ and $R^X$ are as defined for Formula I, according to Scheme 9 by cyanation, by displacement of the chloro substituent of compounds of Formula XIII using a variety of conditions, for example by treatment with potassium or sodium cyanide in the presence of a suitable base such as DABCO in a suitable solvent such as DMSO.

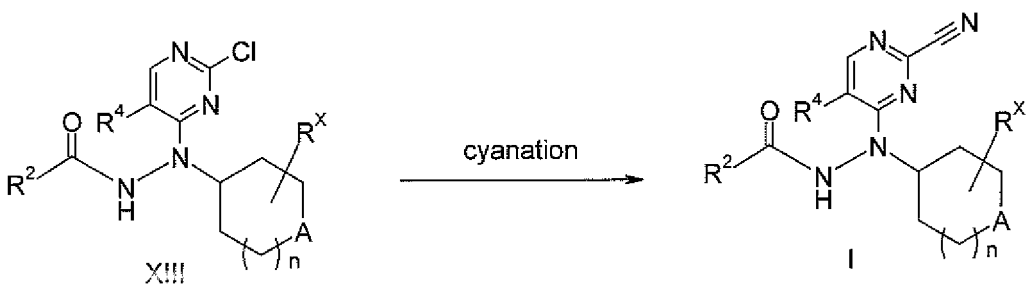


Scheme 9

Compounds of Formula XIII may be prepared from a reaction between compounds of Formula XIV, wherein A, n, $R^4$ and $R^X$ are as defined for Formula I and Hal is chlorine or bromine, and compounds of Formula IV, according to Scheme 10. Compounds XIV are reacted with IV in the presence of a suitable base, such as DIPEA, in a suitable solvent, such as THF orACN.

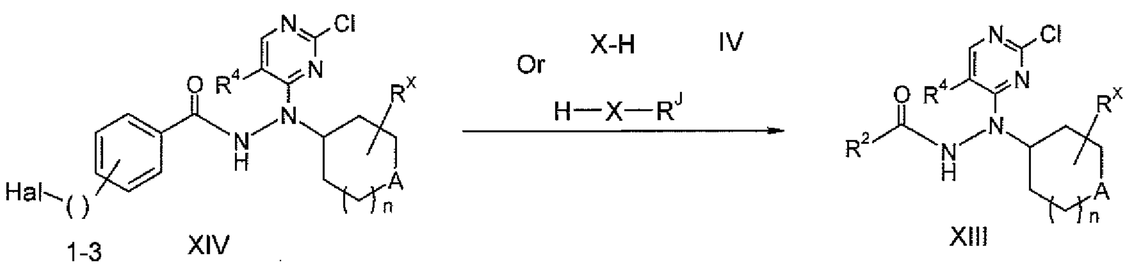

Scheme 10

Compounds of Formula XIV may be prepared according to procedures analogous to those described hereinabove for the preparation of compounds of Formula V.

It will be readily apparent to those skilled in the art that other compounds of Formula I may be prepared using methods analogous to those outlined above, or by reference to the experimental procedures detailed in the Examples provided herein.

Those skilled in the art will also appreciate that in the preparation of the compound of Formula I or a solvate thereof, it may be necessary and/or desirable to protect one or more sensitive groups in the molecule or the appropriate intermediate to prevent undesirable side reactions. Suitable protecting groups for use according to the present invention

are well known to those skilled in the art and may be used in a conventional manner. See, for example, "Protective groups in organic synthesis" by T.W. Greene and P.G.M. Wuts (John Wiley & sons 1991) or "Protecting Groups" by P.J. Kocienski (Georg Thieme Verlag 1994). Examples of suitable amino protecting groups include acyl type protecting groups (e.g. formyl, trifluoroacetyl, acetyl), aromatic urethane type protecting groups (e.g. benzyloxycarbonyl (Cbz) and substituted Cbz), aliphatic urethane protecting groups (e.g. 9-fluorenylmethoxycarbonyl (Fmoc), t-butyloxycarbonyl (Boc), isopropyloxycarbonyl, cyclohexyloxycarbonyl) and alkyl or aralkyl type protecting groups (e.g. benzyl, trityl, chlorotrityl). Examples of suitable oxygen protecting groups may include for example alky silyl groups, such as trimethylsilyl or *tert*-butyldimethylsilyl; alkyl ethers such as tetrahydropyranyl or *tert*-butyl; or esters such as acetate.

## Examples

The following examples illustrate the invention. These examples are not intended to limit the scope of the invention, but rather to provide guidance to the skilled artisan to prepare and use the compounds, compositions, and methods of the invention. While particular embodiments of the invention are described, the skilled artisan will appreciate that various changes and modifications can be made without departing from the spirit and scope of the invention.

Intermediates

Intermediate 1: **1,1-dimethylethyl 2-cyclopentylhydrazinecarboxylate.**

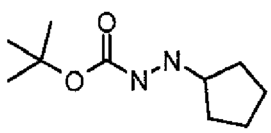

A solution of cyclopentanone (ALDRICH, 1.54 mL, 17.4 mmol) in MeOH (50 ml) was treated with tert-butyl carbazate (FLUKA, 2.3 g, 17.4 mmol), $NaBH_3CN$ (Aldrich, 1.64 g, 26.1 mmol) and glacial AcOH (5 mL). The mixture was stirred at room temperature overnight, cooled over an ice bath and neutralised with NaOH 2N solution. Solvent was evaporated and the residue was partitioned between DCM and $H_2O$. The organic phase was washed with brine, dried and the solvent evaporated under reduced pressure to give the title compound. $^1H$ NMR (300 MHz, DMSO-$d_6$) δ ppm: 8.20 (br.s, 1 H), 4.09 (br.s, 1 H), 1.30-1.59 (m, 8H), 1.37 (s, 9H).

Intermediate 2: **1,1-dimethylethyl 2-(5-bromo-2-chloro-4-pyrimidinyl)-2-cyclopentyl hydrazinecarboxylate.**

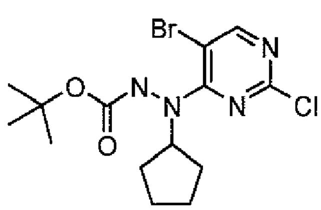

A mixture of Intermediate 1 (1.97 g, 9.8 mmol), 5-Bromo-2,4-dichloropyrimidine (ALDRICH, 2.4 g, 10.78 mmol), DIPEA (FLUKA, 5.1 ml, 29 mmol) and dry EtOH (35 ml) was refluxed for 4 hours. The mixture was concentrated under reduced pressure and the residue partitioned between DCM and 1 M ammonium chloride. The organic layer was treated with brine and dried over $MgSO_4$. The residue was purified by flash chromatography (eluent Hex/AcOEt mixtures 93:7 to 50:50) to give the title compound. $^1H$ NMR (300 MHz, DMSO-$d_6$) δ ppm: 9.77 (s, 1H), 8.37 (s, 1H), 4.82 (m, 1H), 1.46-1.88 (m, 8H), 1.41 (s, 9H). [ES+ MS] m/z 391 $(MH)^+$.

Intermediate 3: **1,1-dimethylethyl 2-(5-bromo-2-cyano-4-pyrimidinyl)-2-cyclopentyl hydrazinecarboxylate.**

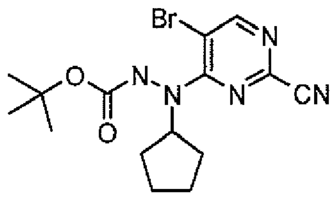

Potassium cyanide (ALDRICH, 213 mg, 3.26 mmol) was added to a suspension of Intermediate 2 (1.07 g, 2.72 mmol) and DABCO (ALDRICH, 305 mg, 2.72 mmol) in a mixture of DMSO/$H_2O$ 85/15 (10 ml) at room temperature. The reaction mixture was stirred at room temperature for 3 h and poured into ice water (15 ml). The white solid that appeared was filtered off and dried. The compound was purified by flash chromatography (eluent: Hex/AcOEt mixtures 95:5 to 50:50) to give the title compound. $^1$H NMR (300 MHz, DMSO-$d_6$) δ ppm: 9.87 (s, 1H), 8.61 (s, 1H), 4.86 (m, 1H), 1.46-1.88 (m, 8H), 1.41 (s, 9H). [ES+ MS] m/z 382 $(MH)^+$.

Intermediate 4: **5-bromo-4-(1-cyclopentylhydrazino)-2-pyrimidinecarbonitrile.**

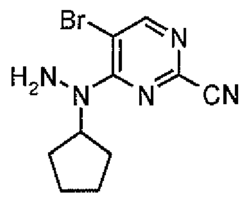

To a solution of Intermediate 3 (353 mg, 0.92 mmol) in acetonitrile (8 mL), p-toluensulfonic acid (ALDRICH, 391 mg, 2.3 mmol) was added and the resulting reaction mixture was stirred at room temperature overnight. The mixture was washed with water and a saturated solution of sodium bicarbonate. The organic layer was treated with brine and dried over anhydrous $Na_2SO_4$. The compound was purified by flash chromatography (eluent: Hex/AcOEt mixtures 100:0 to 40:60) to give the title compound. $^1$H NMR (300 MHz, $d_6$-DMSO) δ ppm: 8.47 (s, 1H), 4.86 (m, 1H), 4.81 (s, 2H), 1.48-1.79 (m, 8H). [ES+ MS] m/z 282 $(MH)^+$.

Intermediate 5: **1,1-dimethylethyl 2-[(*1R,2S+1S,2R*)-2-methylcyclopentyl] hydrazine carboxylate.**

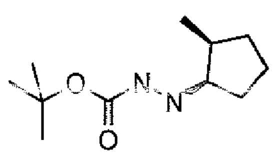

+

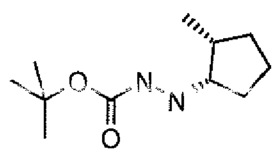

To a solution of 1,1-dimethylethyl hydrazinecarboxylate (ALDRICH, 3.0 g, 22.7 mmol) and 2-methylcyclopentanone (ALDRICH, 0.99 g, 10 mmol) in MeOH (20 mL), sodium cyanoborohydride (1.3 g, 23 mmol) and glacial acetic acid (3.6 mL, 63 mmol) were added. After stirring the resulting reaction mixture at room temperature for 16h, it was cooled down to 0 °C and neutralised with 2N aq. NaOH (3.6 mL). The organic solvent was evaporated under vacuum and product was extracted with DCM. The combined organic layers were washed with brine and dried over anhydrous $Na_2SO_4$, and the crude product was purified using flash chromatography (elute: Hex/EtOAc 100:0 to 7:3) to yield the title compound. $^1$H NMR (300 MHz, d6-DMSO) δ ppm: 8.25- 8.10 (m, 1H), 4.09- 3.98 (br., 1H), 3.21- 3.08 (br., 1H), 1.94-1.78 (m, 1H), 1.72- 1.56 (m, 2H), 1.56-1.42 (br. m, 3H), 1.42- 1.20 (br., 10H), 0.91 (d, 3H).

Intermediate 6: **1,1-dimethylethyl 2-[(1*R*,2*R*+1*S*,2*S*)-2-methylcyclopentyl] hydrazine carboxylate.**

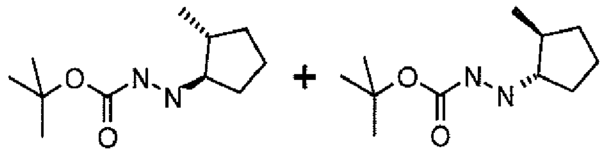

To a solution of 1,1-dimethylethyl hydrazinecarboxylate (ALDRICH, 3.0 g, 22.7 mmol) and 2-methylcyclopentanone (ALDRICH, 0.99 g, 10 mmol) in MeOH (20 mL), sodium cyanoborohydride (1.3 g, 23 mmol) and glacial acetic acid (3.6 mL, 63 mmol) were added. After stirring the resulting reaction mixture at room temperature for 16h, it was cooled down to 0 °C and neutralised with 2N aq. NaOH (3.6 mL). The organic solvent was evaporated under vacuum and product was extracted with DCM. The combined organic layers were washed with brine and dried over anhydrous $Na_2SO_4$, and the crude product was purified using flash chromatography (elute: Hex/EtOAc 100:0 to 7:3) to yield the title compound. $^1H$ NMR (300 MHz, d6-DMSO) $\delta$ ppm: 8.21 (br.s, 1H), 4.20 (br. s, 1H). 2.94- 2.77 (br.m, 1H), 1.88- 1.26 (br., 15H), 1.15-0.98 (m, 1H), 0.9 (d, 3H).

Intermediate 7: **1,1-dimethylethyl 2-(5-bromo-2-chloro-4-pyrimidinyl)-2-[(1*R*, 2*S*+ 1*S*,2*R*)-2-methylcyclopentyl] hydrazinecarboxylate.**

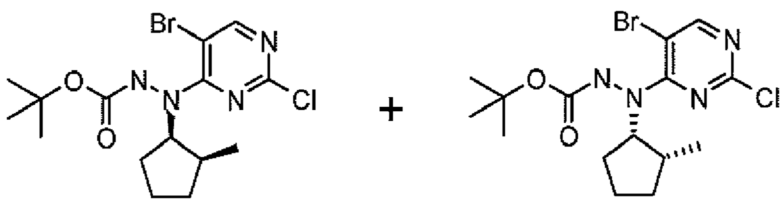

To a solution of 5-bromo-2,4-dichloropyrimidine (ALDRICH, 0.42 g, 1.8 mmol) and Intermediate 5 (0.34 g, 1.6 mmol) in *i*-PrOH (10 mL), DIPEA (0.6 mL, 3.5 mmol) was added and the resulting reaction mixture was stirred at room temperature for 4 days, then refluxed for 3h before it reached completion. The mixture was concentrated under reduced pressure and the residue partitioned between DCM and 1M ammonium chloride. The organic layer was washed with water and brine and dried over anhydrous $Na_2SO_4$. The residue was purified by flash chromatography (elute: Hex/EtOAc 100:0 to 3:2) to give the title compound. $^1H$ NMR (300 MHz, $CDCl_3$) $\delta$ ppm: 8.29 (br.s, 1H), 6.65-6.26 (br., 1H), 5.09-4.82 (br., 1H), 2.57- 2.38 (m, 1H), 2.32- 1.09 (br., 15H), 0.8 (d, 3H); [ES+MS] m/z 405 $(MH)^+$.

Intermediate 8: **1,1-dimethylethyl 2-(5-bromo-2-cyano-4-pyrimidinyl)-2-[(1*R*,2*S*+1*S*,2*R*)-2-methylcyclopentyl]hydrazinecarboxylate**

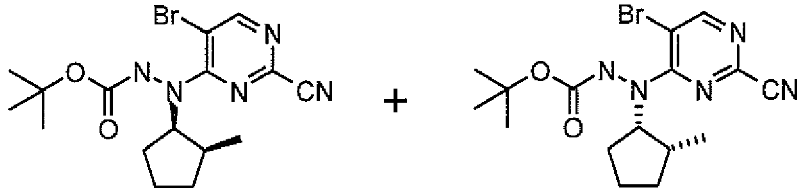

Potassium cyanide (0.16 g, 1.6 mmol) and DABCO (0.21 g, 1.9 mmol) were added to a solution of Intermediate 7 (0.6 g, 1.6 mmol) in a mixture of DMSO/$H_2O$ 9:1 (5 mL) at room temperature. The reaction mixture was stirred at room

temperature for 3 h and then ice was added. The solid that precipitated was filtered off and redissolved in DCM. The compound obtained upon solvent evaporation was purified by f!ash chromatography (eluent: Hex/EtOAC 100:0 to 3:2) to give the title compound. $^1$H NMR (300 MHz, $CDCl_3$) δ ppm: 8.48 (br.s, 1H), 6.6- 6.26 (br., 1H), 5.14- 4.81 (br., 1H), 2.60-2.37 (m, 1H), 2.12-1.10 (br., 15H), 0.8 (d, 3H); $^1$H NMR (300 MHz, $d_6$-DMSO, 80°C) δ ppm: 9.77- 9.44 (br., 1H), 8.61 (s, 1H), 4.87- 4.48 (br., 1H), 2.47- 2.34 (m, 1H), 2.09-1.08 (br., 15H), 1.02- 0.64 (br., 3H); [ES+MS] m/z 396 $(MH)^+$.

Intermediate 9: **5-bromo-4-{1-[(1*R*,2*S*+*1S*,*2R*)-2-methylcyclopentyl]hydrazino}-2-pyrimidinecarbonitrile.**

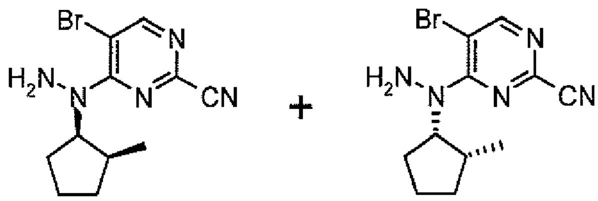

To a solution of Intermediate 8 (0.35g, 0.9 mmol) in dry acetonitrile (5 mL), p-toluensulfonic acid (0.46g, 2.7 mmol) was added and the resulting reaction mixture was stirred at room temperature overnight. The mixture was then concentrated *in vacuo* and the residue partitioned between DCM and a saturated solution of sodium bicarbonate. The organic layer was washed with water, brine and dried over anhydrous $Na_2SO_4$. The residue was purified by flash chromatography (elute: Hex/EtOAc 100:0 to 7:3) to give the title compound. $^1$H NMR (300 MHz, $CDCl_3$) δ ppm: 8.38 (s, 1H), 5.08- 5.00 (m, 1H), 2.40- 2.24 (m, 1H), 2.16- 2.00 (m, 1H), 2.00- .81 (m, 3H), 1.61- 1.45 (m, 2H), 0.96 (d, 3H); [ES+MS] m/z 296 $(MH)^+$.

Intermediate 10: **1,1-dimethylethyl 2-(5-bromo-2-chloro-4-pyrimidinyl)-2-[(1*R*,2*R*+ 1*S*,2*S*)-2-methylcyclopentyl] hydrazinecarboxylate.**

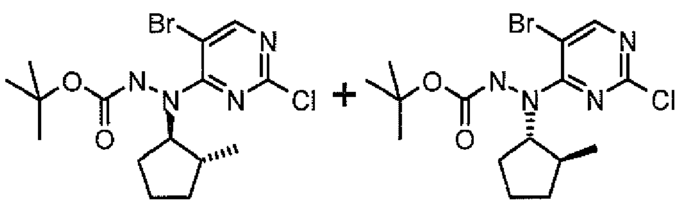

To a solution of 5-bromo-2,4-dichloropyrimidine (ALDRICH, 0.42 g, 1.8 mmol) and Intermediate 6 (0.34 g, 1.6 mmol) in *i*-PrOH (10 mL), DIPEA (0.6 mL, 3.5 mmol) was added and the resulting reaction mixture was stirred at room temperature for 4 days, then refluxed for 3h before it reached completion. The mixture was concentrated under reduced pressure and the residue partitioned between DCM and 1M ammonium chloride. The organic layer was washed with water and brine and dried over anhydrous $Na_2SO_4$. The residue was purified by flash chromatography (elute: Hex/EtOAc 100:0 to 3:2) to give the title compound. $^1$H NMR (300 MHz, $CDCl_3$) δ ppm: 8.27 (br.s, 1H), 6.75-6.22 (br., 1H), 4.88-4.35 (br., 1H), 2.26-1.81 (br., 4H), 1.80- 1.22 (br., 12H), 1.22-0.93 (br., 3H); [ES+MS] m/z 405 $(MH)^+$.

Intermediate 11: **1,1-dimethylethyl 2-(5-bromo-2-cyano-4-pyrimidinyl)-2-[(1*R*,2*R*+ *1S*,*2S*)-2-methylcyclopentyl] hydrazinecarboxylate.**

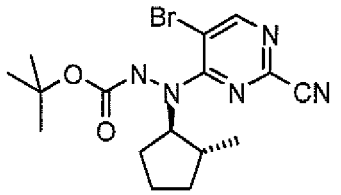
+
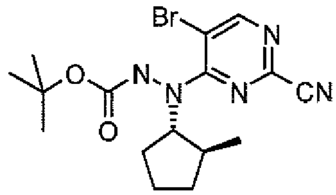

Potassium cyanide (0.52 g, 8 mmol) and DABCO (0.66 g, 5.9 mmol) were added to a solution of Intermediate 10 (2.1 g, 5.3 mmol)) in a mixture of DMSO/$H_2O$ 9:1 (10 mL) at room temperature. The reaction mixture was stirred at room temperature for 3 h and then ice was added. The solid that precipitated was filtered off and redissolved in DCM. The compound obtained upon solvent evaporation was purified by flash chromatography (eluent: Hex/EtOAC 100:0 to 3:2) to give the title compound. $^1$H NMR (300 MHz, $CDCl_3$) δ ppm: 8.46 (br.s, 1H), 6.74- 6.28 (br., 1H), 4.91- 4.39 (br., 1H), 2.22- 1.82 (br., 3H), 1.80-1.21 (br., 13H), 0.8 (d, 3H); $^1$H NMR (300 MHz, $d_6$-DMSO, 80 °C) δ ppm: 9.84- 9.46 (br., 1H), 8.59 (s, 1H), 4.78- 4.35 (br., 1H), 2.21- 1.99 (br., 1H), 1.99- 1.75 (br., 2H), 1.74- 1.55 (br., 3H), 1.55- 1.14 (br., 10H), 1.14- 0.89 (br., 3H); [ES+ MS] m/z 396 $(MH)^+$.

Intermediate 12: **5-bromo-4-{1-[(1*R*,2*R*+1*S*,2*S*)-2-methylcyclopentyl]hydrazino}-2-pyrimidine carbonitrile.**

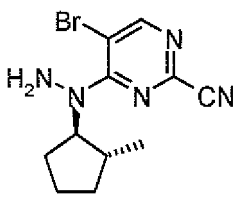
+
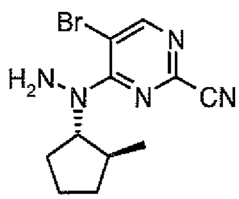

To a solution of Intermediate 11 (1.8g, 4.5 mmol) in dry acetonitrile (20 mL), p-toluensulfonic acid (2.33g, 13.6 mmol) was added and the resulting reaction mixture was stirred at room temperature overnight. The mixture was then concentrated in *vacuo* and the residue partitioned between DCM and a saturated solution of sodium bicarbonate. The combined organic layers were washed with water, brine and dried over anhydrous $Na_2SO_4$. The residue was purified by flash chromatography (elute: Hex/EtOAc 100:0 to 7:3) to give the title compound. $^1$H NMR (300 MHz, $CDCl_3$) δ ppm: 8.40 (s, 1H), 4.59- 4.51 (m, 1H), 2.34- 2.17 (m, 1H), 2.04-1.61 (m, 5H), 1.37- 1.19 (m, 1H), 0.95 (d, 3H); [ES+ MS] m/z 296 $(MH)^+$.

Intermediate 13: **1,1-dimethylethyl 2-(3-methylcyclopentyl)hydrazinecarboxylate.**

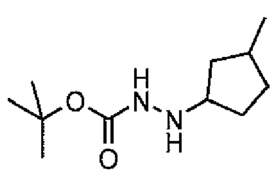

To a solution of 1,1-dimethylethyl hydrazinecarboxylate (ALDRICH, 3.0 g, 22.7 mmol) and 3-methylcyclopentanone (ALDRICH, 2.24 g, 22.8 mmol) in dry MeOH (30 mL), sodium cyanoborohydride (2.8 g, 45 mmol) and glacial acetic acid (8.2 mL, 143.2 mmol) were added. After stirring the resulting reaction mixture at room temperature overnight, it was cooled down in an ice bath and neutralised with 2N aq. NaOH (8.2 mL). The organic solvent was removed under

reduced pressure and product was extracted with DCM. The combined organic layers were washed with brine and dried over anhydrous $Na_2SO_4$, to yield the crude product which was used without any further purification. $^1$H NMR (300 MHz, $CDCl_3$) δ ppm: 6.70- 6.09 (br., 1H), 4.78- 3.98 (br., 1H), 3.62- 3.45 (m, 1H), 2.20- 1.97 (m, 2H), 1.98- 1.80 (m, 1H), 1.80-1.62 (m, 2H),1.60-1.34 (m, 10H), 1.34- 1.20 (m, 1H), 1.03- 0.96 (m, 3H).

Intermediate 14: **1,1-dimethylethyl 2-(5-bromo-2-chloro-4-pyrimidinyl)-2-(3-methyl cyclopentyl)hydrazinecarboxylate.**

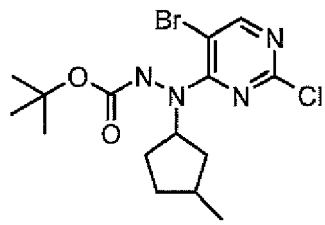

To a solution of 5-bromo-2,4-dichloropyrimidine (ALDRICH, 5.3 g, 23 mmol) and Intermediate 13 (22.7 mmol) in *i*-PrOH (40 mL), DIPEA (7.9 mL, 46 mmol) was added and the resulting reaction mixture was stirred at room temperature overnight, then refluxed for 5h before it reached completion. The mixture was concentrated under reduced pressure and the residue partitioned between DCM and 1M ammonium chloride. The combined organic layers were washed with water and brine and dried over anhydrous $Na_2SO_4$. The residue was purified by flash chromatography (elute: Hex/EtOAc 100:0 to 3:2) to give the title compound. $^1$H NMR (300 MHz, $CDCl_3$) δ ppm: 8.27 (s, 1H), 6.75- 6.21 (br., 1H), 5.12- 4.83 (br., 1H), 2.27- 0.77 (m, 19H); [ES+ MS] m/z 405 $(MH)^+$.

Intermediate 15: **1,1-dimethylethyl 2-(5-bromo-2-cyano-4-pyrimidinyl)-2-(3-methylcyclopentyl)hydrazinecarboxylate.**

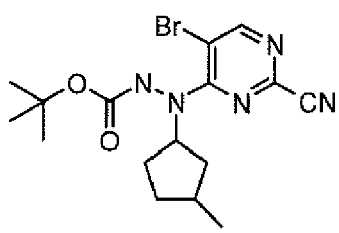

Potassium cyanide (1.5 g, 23.7 mmol) and DABCO (1.75 g, 15.6 mmol) were added to a solution of Intermediate 14 (6.3 g, 15.6 mmol) in a mixture of DMSO/$H_2O$ 9:1 (40 mL) at room temperature. The reaction mixture was stirred at room temperature for 4 h and then ice was added. The solid that precipitated was filtered off, washed abundantly with water, dried under air and redissolved in DCM. The compound obtained upon solvent evaporation was purified by flash chromatography (eluent: Hex/EtOAC 100:0 to 3:2) to give the title compound. $^1$H NMR (300 MHz, $CDCl_3$) δ ppm : 8,47 (s, 1H), 6.89- 6.28 (br., 1H), 5.15- 4.82 (br., 1H), 2.29-1.76 (br., 4H), 1.76-1.14 (br., 12H), 1.11-1.00 (m, 3H); $^1$H NMR (300 MHz, $d_6$-DMSO, 80 °C) δ ppm: 9.81- 9.30 (br., 1H), 8.59 (s, 1H), 5.11-4.75 (m, 1H), 2.15- 1.63 (br., 5H), 1.63- 1.06 (br., 11H), 1.03- 0.98 (m, 3H); [ES+ MS] m/z 396 $(MH)^+$.

Intermediate 16: **5-bromo-4-[1-(3-methylcyclopentyl)hydrazino]-2-pyrimidine carbonitrile.**

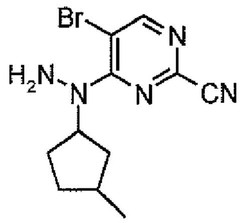

To a solution of Intermediate 15 (0.6 g, 1.5 mmol) in dry acetonitrile (20 mL), p-toluensulfonic acid (0.65 g, 3.8 mmol) was added and the resulting reaction mixture was stirred at room temperature overnight. The mixture was then concentrated in *vacuo* and the residue partitioned between DCM and a saturated solution of sodium bicarbonate. The combined organic layers were washed with water, brine and dried over anhydrous $Na_2SO_4$. The residue was purified by flash chromatography (elute: Hex/EtOAc 100:0 to 7:3) to give the title compound. $^1H$ NMR (300 MHz, $CDCl_3$) $\delta$ ppm: 8.40 (s, 1H), 5.19- 4.94 (m, 1H), 2.31- 2.14 (m, 1H), 2.08- 1.73 (br.m, 4H), 1.58- 1.13 (m, 2H), 1.10- 1.02 (m, 3H).

Intermediate 17: ***N'*-(5-bromo-2-cyano-4-pyrimidinyl)-4-(chloromethyl)-*N'*-(3-methylcyclopentyl)benzohydrazide.**

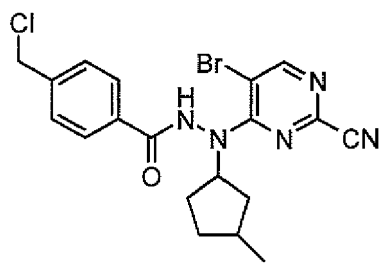

To a solution of Intermediate 16 (0.23 g, 0.8 mmol) in dry THF (5 mL), 4-(chloromethyl)benzoyl chloride (ALDRICH, 0.18 g, 0.94 mmol) and DIPEA (0.29 mL, 1.66 mmol) were added and the resulting reaction mixture was stirred at room temperature for 4h. Then, solvent was removed *in vacuo* and the residue partitioned between DCM and 1N aq, ammonium chloride. The combined organic layer were washed with water and brine and dried over anhydrous $Na_2SO_4$. The crude reaction mixture was redissolved in DCM and product was precipitated using hexane. The solid was filtered off, dried under air and used in the next steps without any further purification.

Intermediate 18: **N'-(5-bromo-2-cyano-4-pyrimidinyl)-4-(chloromethyl)-N'-cyclo pentylbenzohydrazide.**

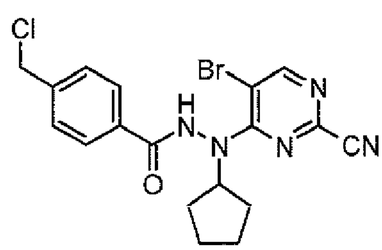

To a mixture of Intermediate 4 (1 g, 3.55 mmol) and 4-(chloromethyl)benzoyl chloride (ALDRICH, 0.805 g, 4.26 mmol) in dry THF (50 mL), DIPEA (1.235 mL, 7.1 mmol) was added and the reaction mixture was stirred at room temperature overnight. Then, solvent was removed *in vacuo* and the residue partitioned between DCM and 1N HCl. The organic layer was washed with brine, dried over anhydrous $MgSO_4$ and concentrated to dryness. The residue was

used in the next step without any further purification.

Intermediate 19: ***N*'-(5-bromo-2-cyano-4-pyrimidinyl)-3-(chloromethyl)-*N*'-cyclo pentylbenzohydrazide.**

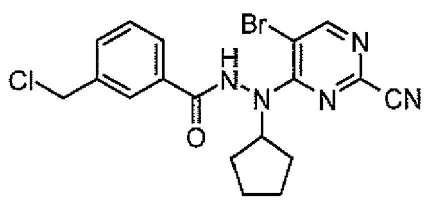

To a mixture of Intermediate 4 (1 g, 3.55 mmol) and 3-(chloromethyl)benzoyl chloride (ALDRICH, 0.757 mL, 5.32 mmol) in dry THF (50 mL), DIPEA (1.235 mL, 7.1 mmol) was added and the reaction mixture was stirred at room temperature overnight. Then, solvent was removed *in vacuo* and the residue partitioned between DCM and 1 N HCl. The organic layer was washed with brine, dried over anhydrous $MgSO_4$ and concentrated to dryness. The residue was treated with hexane, the precipitate that was formed was filtered and used in the next step without further purification.

Intermediate 20: **1,1-dimethylethyl 2-cyclohexylhydrazinecarboxylate.**

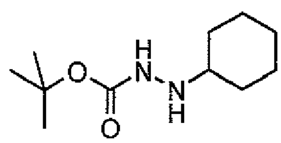

To a solution of 1,1-dimethylethyl hydrazinecarboxylate (ALDRICH, 3.0 g, 22.7 mmol) in *i*-PrOH (30 mL), cyclohexanone (ALDRICH, 2.4 mL, 23.2 mmol) was added and the resulting reaction mixture was heated to reflux. After 4h, the reaction had reached completion and hence solvent was removed *in vacuo.* The residue was partitioned between DCM and water. The combined organic phases were washed with brine and dried over anhydrous $Na_2SO_4$, yielding the corresponding imine as a white solid. $^1H$ NMR (300 MHz, $CDCl_3$) δ ppm: 7.79- 7.36 (br., 1H), 2.44- 2.35 (m, 2H), 2.29-2.1 (m, 2H), 1.93-1.81 (m, 1H), 1.79-1.57 (m, 5H), 1.54-1.44 (br., 9H).

The intermediate imine was dissolved in MeOH (30 mL) and sodium cyanoborohydride (ALDRICH, 45.9 mmol) and glacial acetic acid (8.2 mL, 143.2 mmol) were added. After stirring the resulting reaction mixture at room temperature for 16h, it was cooled down to 0 ˚C and neutralised with 2N aq. NaOH (8.2 mL). The organic solvent was evaporated under vacuum and product was extracted with DCM. The combined organic layers were washed with brine and dried over anhydrous $Na_2SO_4$, yielding the above title compound. $^1H$ NMR (300 MHz, $CDCl_3$) δ ppm: 2.90- 2.76 (m, 1H), 1.94- 1.8 (m, 2H), 1.79- 1.7 (m, 2H), 1.67- 1.57 (m, 1H), 1.54- 1.42 (br., 9H), 1.40- 1.02 (br. m, 6H).

Intermediate 21: **1,1-dimethylethyl 2-(5-bromo-2-chloro-4-pyrimidinyl)-2-cyclohexylhydrazinecarboxylate.**

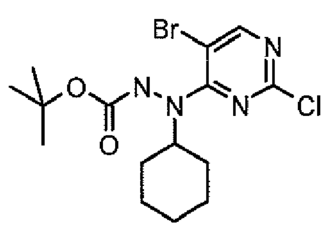

To a solution of 5-bromo-2,4-dichloropyrimidine (ALDRICH, 5.68 g, 25 mmol) and Intermediate 20 (4.8 g, 22.2 mmol) in *i*-PrOH (40 mL), DIPEA (7.8 mL, 45 mmol) was added and the resulting reaction mixture was stirred at room temperature for 16 h. The mixture was concentrated under reduced pressure and the residue partitioned between DCM and 1M ammonium chloride. The combined organic layers were washed with water and brine and dried over anhydrous $Na_2SO_4$. The residue was purified by flash chromatography (elute: Hex/EtOAc 100:0 to 7:3) to give the title compound. $^1H$ NMR (300 MHz, $CDCl_3$) δ ppm: 8.26 (s, 1H), 6.69- 6.16 (br., 1H), 4.64- 4.46 (m, 1H), 2.17-1.99 (m, 1H), 1.90-1.77 (m, 2H), 1.77- 1.60 (m, 2H), 1.60-1.00 (br., 14H); $^1H$ NMR (300 MHz, $d_6$-DMSO) δ ppm: 9.70 (br.s, 1H), 8.44- 8.32 (br., 1H), 4.50- 4.32 (m, 1H), 1.94-1.69 (br.m, 3H), 1.59-1.52 (br.m, 2H), 1.52- 0.94 (br., 14H); [ES+ MS] m/z 405 $(MH)^+$.

Intermediate 22: **1,1-dimethylethyl 2-(5-bromo-2-cyano-4-pyrimidinyl)-2-cyclohexylhydrazinecarboxylate.**

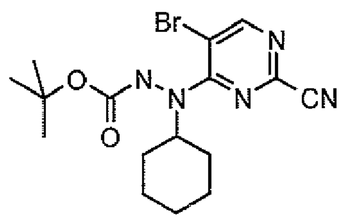

Potassium cyanide (0.78 g, 12 mmol) and DABCO (1.16 g, 10 mmol) were added to a suspension of Intermediate 21 (3.7 g, 9 mmol) in a mixture of DMSO/$H_2O$ 9:1 (160 mL) at room temperature. The reaction mixture was stirred at room temperature for 3.5 days, before heating it up to 70 ˚C for further 3h before it reached completion. After having left it to cool down to room temperature, ice was added. The light yellow solid that precipitated was filtered off and redissolved in DCM. The compound obtained upon solvent evaporation was purified by flash chromatography (eluent: Hex/EtOAC 100:0 to 3:2) to give the title compound. $^1H$ NMR (300 MHz, $CDCl_3$) δ ppm: 8.45 (s, 1H), 6.66-6.22 (br., 1H), 4.65- 4.50 (m, 1H), 2.16- 1.97 (m, 1H), 1.93- 1.79 (m, 2H), 1.79- 1.65 (m, 2H), 1.65- 1.04 (br., 14H); $^1H$ NMR (300 MHz, $d_6$-DMSO) δ ppm: 9.81 (br.s, 1H), 8.60 (s, 1H), 4.53- 4.39 (m, 1H), 1.94- 1.71 (br.m, 3H), 1.71- 1.55 (br.m, 2H), 1.55- 0.97 (br., 14H); [ES+ MS] m/z 396 $(MH)^+$.

Intermediate 23: **5-bromo-4-(1-cyclohexylhydrazino)-2-pyrimidinecarbonitrile.**

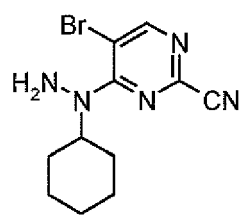

To a solution of Intermediate 22 (1g, 2.5 mmol) in dry acetonitrile (20 mL), p-toluensulfonic acid (1.0 g, 6 mmol) was added and the resulting reaction mixture was stirred at room temperature overnight. The mixture was then concentrated *in vacuo* and the residue partitioned between DCM and a saturated solution of sodium bicarbonate. The combined organic layers were washed with water, brine and dried over anhydrous $Na_2SO_4$. The residue was purified by flash chromatography (elute: Hex/EtOAc 100:0 to 3:2) to give the title compound. $^1H$ NMR (300 MHz, $CDCl_3$) δ ppm: 8.40 (s, 1H), 4.52-4.37 (m, 1H), 1.95-1.82 (m, 2H), 1.82-1.61 (m, 5H), 1.50- 1.31 (m, 2H), 1.29-1.08 (m, 1H); [ES+ MS] m/z 296 $(MH)^+$.

Intermediate 24: ***N*'-(5-bromo-2-cyano-4-pyrimidinyl)-4-(chloromethyl)-N'-cyclo hexylbenzohydrazide.**

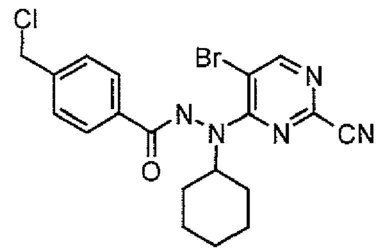

To a solution of Intermediate 23 (0.57 g, 1,9 mmol) in dry THF (4 mL), 4-(chloromethyl)benzoyl chloride (ALDRICH, 0.43 g, 2,3 mmol) and DIPEA (0.66 mL, 3.8 mmol) were added and the resulting reaction mixture was stirred at room temperature overnight. The mixture was concentrated *in vacuo* and the residue was partitioned between DCM and 1M aq. ammonium chloride. The combined organic layers were washed with water and brine and dried over anhydrous $Na_2SO_4$. The compound obtained upon solvent evaporation was purified by flash chromatography (eluent: Hex/EtOAC 100:0 to 3:2) to give the title compound. $^1H$ NMR (300 MHz, $CDCl_3$) δ ppm: 8.45 (s, 1H), 7.94 (br.s, 1H), 7.87- 7.82 (m, 2H), 7.55 (d, 2H), 4.77- 4.67 (br., 1H), 4.65 (s, 2H), 2.32- 2.05 (br., 1H), 2.01- 1.78 (br., 3H), 1.78- 1.66 (br., 1H), 1.66- 1.38 (br., 3H), 1.37- 1.01 (br., 2H).

Intermediate 25: ***N*'-(5-bromo-2-cyano-4-pyrimidinyl)-3-(chloromethyl)-N'-cyclohexylbenzohydrazide.**

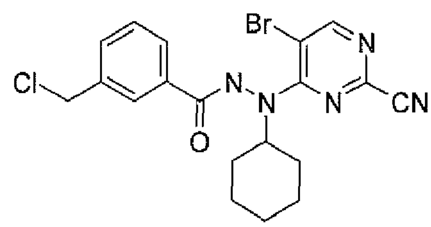

Although the aim of this experiment was to synthesise Example 20 using a one-pot reaction, only Intermediate 25 was isolated.

To a solution of Intermediate 23 (0.12 g, 0.4 mmol) in dry THF (4 mL), 3-(chloromethyl)benzoyl chloride (ALDRICH, 0.07 mL, 0.5 mmol) and DIPEA (0.08 mL, 0.46 mmol) were added and the resulting reaction mixture was stirred at room temperature overnight. Then, 1-methyl-4-(piperidin-4-yl)-piperazine (FLUOROCHEM, 0.09 g, 0.5 mmol) and catalytic sodium iodide were added, and the reaction mixture was stirred at room temperature for further 2 days. Then, more 1-methyl-4-(piperidin-4-yl)-piperazine (0.09 g, 0.5 mmol) and sodium iodide were added, along with ACN (2mL) to overcome solubility issues. After further 16h, more DIPEA (0.14 mL, 0.8 mmol) were added in order to drive the reaction to completion and the reaction mixture was stirred at room temperature for further 24h. Then, the mixture was concentrated *in vacuo* and the crude reaction mixture was purified by preparative HPLC (SUNFIRE 19x150 mm, ACN:$H_2O$ 0.1% TFA, gradient 10-100%) to give the title compound which was used in subsequent steps.

Intermediate 26: **1,1-Dimethylethyl 2-(tetrahydro-2*H*-pyran-4-yl)hydrazinecarboxylate.**

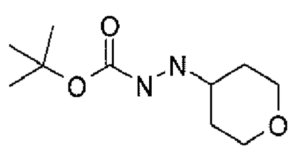

To a solution of *tert*-butyl carbazate (FLUKA, 4.6 g, 34.6 mmol) in MeOH (124 mL), tetrahydro-4H-pyran-4-one (ALDRICH, 3.47 g, 34.6 mmol), sodium cyanoborohydride (ALDRICH, 3.3 g, 52 mmol) and glacial acetic acid (10 mL) were added. The resulting reaction mixture was stirred at room temperature overnight. The reaction mixture was neutralised with 2N NaOH solution (20 mL) and concentrated *in vacuo.* Product was extracted with DCM and the organic layer was washed with brine, dried over anhydrous $MgSO_4$ and the solvent evaporated under reduced pressure to give the title compound. $^1H$ NMR (300 MHz, DMSO-$d_6$) δ ppm: 8.19 (br.s, 1H), 4.47- 4.21 (br.s, 1H), 3.82- 3.75 (dt, 2H), 3.29- 3.21 (td, 2H), 2.94- 2.81 (m, 1H), 1.68-1.55 (m, 2H), 1.37 (s, 9H), 1.31-1.11 (m, 2H).

Intermediate 27: **1,1-Dimethylethyl 2-(5-bromo-2-chloro-4-pyrimidinyl)-2-(tetrahydro-2*H*-pyran-4-yl)hydrazine-carboxylate.**

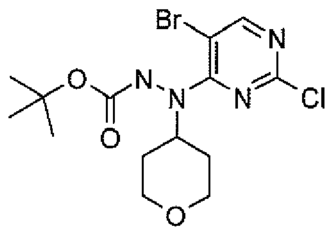

To a solution of Intermediate 26 (3.3 g, 15.1 mmol) in dry EtOH (40 mL), 5-bromo-2,4-dichloropyrimidine (ALDRICH, 3.8 g, 16.6 mmol) dissolved in EtOH (15 mL) and DIPEA (FLUKA, 7.9 mL, 45.4 mmol) were added and the resulting reaction mixture was refluxed for 5 hours. The mixture was concentrated under reduced pressure and the residue partitioned between DCM and 1M ammonium chloride. The organic layer was treated with brine and dried over anhydrous $Na_2SO_4$. The residue was purified by flash chromatography (eluent: Hex/AcOEt mixtures 100:0 to 2:3) to give the title compound. $^1H$ NMR (300 MHz, DMSO-$d_6$) δ ppm: 9.78 (s, 1H), 8.39 (s, 1H), 4.75- 4.56 (m, 1H), 3.96- 3.84 (m, 2H), 3.45- 3.32 (m, 2H), 1.83-1.47 (m, 4H), 1.42 (s, 9H).

Intermediate 28: **1,1-Dimethylethyl 2-(5-bromo-2-cyano-4-pyrimidinyl)-2-(tetrahydro-2*H*-pyran-4-yl)hydrazine-carboxylate.**

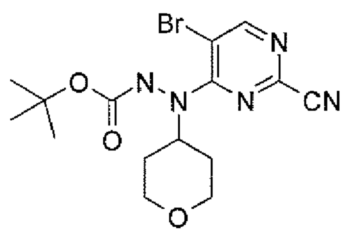

Potassium cyanide (ALDRICH, 0.29 g, 4.4 mmol) and DABCO (ALDRICH, 0.41 g, 3.7 mmol) were added to a solution of Intermediate 27 (1.5 g, 3.7 mmol) in a mixture of DMSO/$H_2O$ 9/1 (15 mL). The reaction mixture was stirred at room temperature for 3h and poured into iced water. The brown solid that precipitated was filtered off and the crude product was purified by flash chromatography (eluent: Hex/AcOEt mixtures 100:0 to 1:1) to give the title compound. $^1H$ NMR (300 MHz, DMSO-$d_6$) δ ppm: 9.87 (s, 1H), 8.63 (s, 1H), 4.78- 4.63 (m, 1H), 3.96- 3.83 (m, 2H), 3.45- 3.36 (m, 2H), 1.83-1.84 (m, 2H), 1.63-1.46 (m, 2H), 1.42 (s, 9H).

Intermediate 29: **5-Bromo-4-[1-(tetrahydro-2*H*-pyran-4-yl)hydrazino]-2-pyrimidinecarbonitrile.**

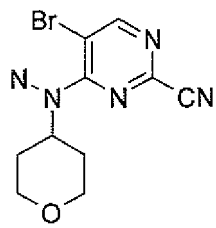

To a solution of Intermediate 28 (0.79 g, 2 mmol) in acetonitrile (16 mL), p-toluensulfonic acid (ALDRICH, 0.84 g, 4.9 mmol) was added and the resulting reaction mixture was stirred at room temperature overnight. Solvent was removed under reduced pressure and the residue was re-dissolved in DCM. Solid sodium bicarbonate was added and the resulting mixture was stirred at room temperature for 5 min. The organic layer was then washed with 10% aqueous sodium bicarbonate, water and brine and dried over anhydrous $MgSO_4$. Solvent evaporation *in vacuo* yielded the title compound. $^1H$ NMR (300 MHz, $d_6$-DMSO) δ ppm: 8.49 (s, 1 H), 4.79 (s, 2H), 4.64-4.49 (m, 1H), 3.97- 3.87 (m, 2H), 3.46- 3.34 (m, 2H), 1.99-1.85 (dq, 2H), 1.59-1.49 (m, 2H).

Intermediate 30: ***N'*-(5-Bromo-2-cyano-4-pyrimidinyl)-4-(bromomethyl)-*N'*-(tetrahydro-2*H*-pyran-4-yl)benzohydrazide.**

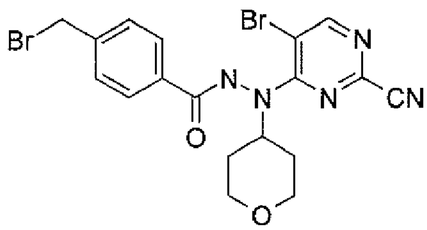

To a solution of Intermediate 29 (0.58 g, 1.95 mmol) in dry THF (16 mL), 4-bromomethyl benzoyl bromide (ALDRICH, 0.54 g, 1.95 mmol) and DIPEA (0.66 mL, 3.9 mmol) were added and the reaction mixture was stirred at room temperature overnight. Then, solvent was removed *in vacuo* and the residue partitioned between DCM and water. The organic layer was washed with 1N aqueous NaOH and brine, dried over anhydrous $MgSO_4$ and concentrated to dryness. The residue was dissolved in the minimum amount of DCM and the solution stirred with hexane, the title compound precipitating off as a white solid. $^1H$ NMR (300 MHz, $d_6$-DMSO) δ ppm: 11.17 (s, 1H), 8.65 (s, 1H), 7.9 (d, 2H), 7.6 (d, 2H), 4.89- 4.8 (m, 1H), 4.76 (s, 2H), 3.96- 3.84 (m, 2H), 3.54- 3.35 (m, 2H), 1.98- 1.70 (m, 3H), 1.55-1.36 (m, 1H).

Intermediate 31: **1,1-Dimethylethyl 2-(2,5-dichloro-4-pyrimidinyl)-2-(tetrahydro-2*H*-pyran-4-yl)hydrazinecarboxylate.**

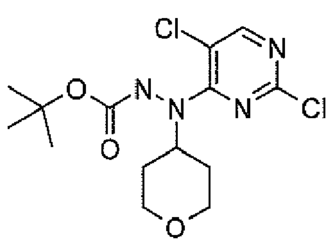

To a solution of Intermediate 26 (3.6 g, 16.8 mmol) in dry EtOH (60 mL), 2,4,5-trichloropyrimidine (ALDRICH,

3.4 g, 18.5 mmol) and DIPEA (FLUKA, 8.5 mL, 50 mmol) were added and the resulting reaction mixture was refluxed for 3h, then stirred at room temperature for 3 days. In order to drive the reaction to completion the reaction mixture was refluxed for further 7h, then stirred at room temperature for 12 h. The mixture was concentrated under reduced pressure and the residue partitioned between DCM and 1M ammonium chloride. The organic layer was treated with brine and dried over anhydrous $MgSO_4$. The residue was purified by flash chromatography (eluent: Hex/AcOEt mixtures 49:1 to 1:1) to give the title compound. $^1H$ NMR (300 MHz, DMSO-$d_6$) δ ppm: 9.80 (s, 1H), 8.28 (s, 1H), 4.75- 4.56 (m, 1H), 3.96- 3.82 (m, 2H), 3.46- 3.31 (m, 2H), 1.82-1.25 (m, 13H).

Intermediate 32: **1,1-Dimethylethyl 2-(5-chloro-2-cyano-4-pyrimidinyl)-2-(tetrahydro-2*H*-pyran-4-yl)hydrazine-carboxylate.**

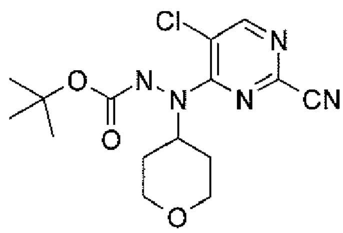

Potassium cyanide (ALDRICH, 0.58 g, 8.9 mmol) and DABCO (ALDRICH, 0.83 g, 7.4 mmol) were added to a solution of Intermediate 31 (2.7 g, 7.4 mmol) in a mixture of DMSO/$H_2O$ 9/1 (30 mL). The reaction mixture was stirred at room temperature for 4h and poured into iced water, but no precipitate appeared. Product was then extracted with ethyl acetate and the combined organic layers were washed with brine, dried over $MgSO_4$ and solvent was removed under reduced pressure. The crude product was purified by flash chromatography (eluent: Hex/AcOEt mixtures 49:1 to 1:1) to give the title compound. $^1H$ NMR (300 MHz, DMSO-$d_6$) δ ppm: 9.90 (s, 1H), 8.52 (s, 1H), 4.84-4.61 (m, 1H), 3.98- 3.82 (m, 2H), 3.51- 3.35 (m, 2H), 1.83-1.20 (m, 13H); [ES+ MS] m/z 354 $(MH)^+$.

Intermediate 33: **5-Chloro-4-[1-(tetrahydro-2*H*-pyran-4-yl)hydrazino]-2-pyrimidinecarbonitrile**

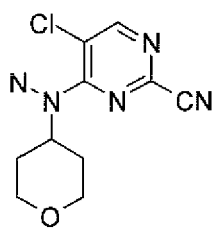

To a solution of Intermediate 32 (1.05 g, 3 mmol) in dry acetonitrile (24 mL), *p*-toluensulfonic acid (ALDRICH, 1.26 g, 7.4 mmol) was added and the resulting reaction mixture was stirred at room temperature overnight. Solvent was removed under reduced pressure and the residue was re-dissolved in DCM. Solid sodium bicarbonate was added and the resulting mixture was stirred at room temperature for 5 min. The organic layer was then washed with 10% aqueous sodium bicarbonate, water and brine and dried over anhydrous $MgSO_4$. After solvent was evaporated *in vacuo*, the crude product was purified by flash chromatography (eluent: Hex/AcOEt mixtures 49:1 to 1:1) to give the title compound. $^1H$ NMR (300 MHz, $d_6$-DMSO) δ ppm: 8.37 (s, 1H), 4.89 (s, 2H), 4.62- 4.48 (m, 1H), 3.98- 3.86 (m, 2H), 3.45- 3.37 (m, 2H), 2.00- 1.86 (m, 2H), 1.57-1.52 (m, 2H); [ES+ MS] m/z 254 $(MH)^+$.

Intermediate 34: **4-(Bromomethyl)-*N'*-(5-chloro-2-cyano-4-pyrimidinyl)-*N'*-(tetrahydro-2H-pyran-4-yl)benzohy-drazide.**

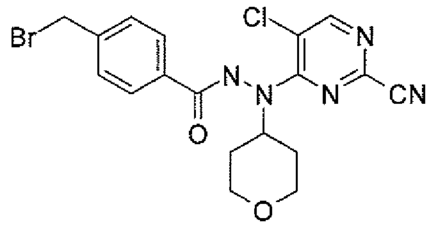

To a solution of Intermediate 33 (0.66 g, 2.6 mmol) in dry THF (22 mL), 4-bromomethyl benzoyl bromide (ALDRICH, 0.72 g, 2.6 mmol) and DIPEA (0.88 mL, 5.2 mmol) were added and the reaction mixture was stirred at room temperature overnight. Then, solvent was removed *in vacuo* and the residue partitioned between DCM and water. The organic layer was washed with 1N aqueous NaOH and brine, dried over anhydrous $MgSO_4$ and concentrated to dryness. The residue was dissolved in the minimum amount of DCM and the solution stirred with hexane, the title compound precipitating off. $^1H$ NMR (300 MHz, $d_6$-DMSO) δ ppm: 11.17 (s, 1H), 8.53 (s, 1H), 7.87 (d, 2H), 7.59 (d, 2H), 4.9- 4.75 (m, 3H), 4.00- 3.8 (m, 2H), 3.55- 3.31 (m, 2H), 2.00-1.70 (m, 3H), 1.58-1.35 (m, 1H).

Intermediate 35: **1,1-Dimethylethyl 2-(1-acetyl-4-piperidinyl)hydrazinecarboxylate.**

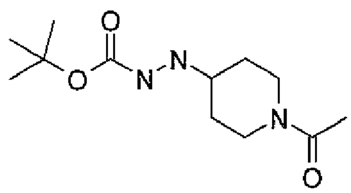

To a solution of 1-acetyl-4-piperidone (FLUKA, 2 mL, 16 mmol) in 1,2-dichloroethane (100 mL), *tert*-butyl carbazate (FLUKA, 2 g, 16 mmol), sodium triacetoxyborohydride (ALDRICH, 4.88 g, 23 mmol) and glacial acetic acid (0.91 mL) were added. The resulting reaction mixture was stirred at room temperature for 20 h. The reaction mixture was neutralised with 2N NaOH solution (100 mL) and the organic layer was then washed with brine, dried over anhydrous $MgSO_4$ and the solvent evaporated under reduced pressure to give the title compound. $^1H$ NMR (300 MHz, DMSO-$d_6$) δ ppm: 8.29- 8.13 (br.s, 1H), 4.40- 4.37 (m, 1H), 4.01- 3.90 (br., 1H), 3.72- 3.6 (m, 1H), 3.15- 2.80 (m, 2H), 1.96 (s, 3H), 1.73- 1.55 (m, 2H), 1.45- 1.35 (br., 9H), 1.27- 1.05 (br., 2H).

Intermediate 36: **1,1-Dimethylethyl 2-(1-acetyl-4-piperidinyl)-2-(5-bromo-2-chloro-4-pyrimidinyl)hydrazinecarboxylate.**

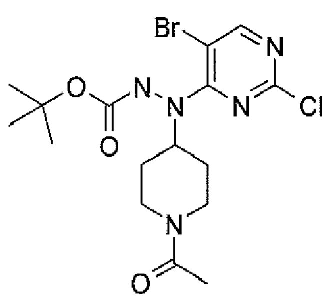

To a solution of Intermediate 35 (4.1 g, 16 mmol) and 5-bromo-2,4-dichloropyrimidine (ALDRICH, 4.1 g, 18

mmol) in EtOH (300 mL), DIPEA (FLUKA, 5.6 mL, 32 mmol) was added and the resulting reaction mixture was refluxed overnight. The mixture was concentrated under reduced pressure and the residue partitioned between DCM and 1M ammonium chloride. The organic layer was treated with brine, dried over anhydrous $MgSO_4$ and solvent was removed *in vacuo.* The crude product was purified by flash chromatography (eluent: DCM/MeOH mixtures 100:0 to 4:1) to give the title compound. $^1H$ NMR (300 MHz, DMSO-$d_6$) δ ppm: 9.75 (br.s, 1H), 8.36 (s, 1H), 4.72- 4.65 (m, 1H), 4.49- 4.35 (m, 1H), 3.94- 3.81 (m, 1H), 3.55- 3.44 (m, 1H), 3.19- 3.00 (m, 1H), 2.02-1.95 (br., 3H), 1.91-1.56 (m, 2H), 1.48-1.31 (br., 9H), 1.30-1.18 (br., 2H).

Intermediate 37: **1,1-Dimethylethyl 2-(1-acetyl-4-piperidinyl)-2-(5-bromo-2-cyano-4-pyrimidinyl)hydrazinecarboxylate.**

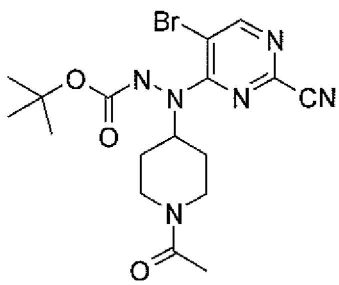

Potassium cyanide (ALDRICH, 0.72 g, 11 mmol) and DABCO (ALDRICH, 0.9 g, 8 mmol) were added to a solution of Intermediate 36 (3.2 g, 8 mmol) in a mixture of DMSO/$H_2O$ 9/1 (100 mL) and the reaction mixture was stirred at room temperature overnight. Water and ethyl acetate were added, the organic layer was washed with brine, dried over $MgSO_4$ and solvent was removed under reduced pressure. The crude product was purified by flash chromatography (eluent: Hex/AcOEt mixtures 100:0 to 0:100) and then again by preparative HPLC (XTERRA 19x150 mm, ACN:$H_2O$, 0.1%TFA, gradient 30-100%) to give the title compound. $^1H$ NMR (300 MHz, DMSO-$d_6$) δ ppm: 9.88- 9.81 (br., 1H), 8.64 (s, 1H), 4.79- 4.69 (m, 1H), 4.53- 4.38 (m, 1H), 4.07- 3.61 (br., 1H), 3.22- 3.06 (m, 1H), 2.69-2.54 (m, 1H), 2.03-1.97 (br., 3H), 1.93-1.58 (br., 2H), 1.41 (s, 9H), 1.26- 1.18 (m, 2H); [ES+ MS] m/z 439 $(MH)^+$.

Intermediate 38: **4-[1-(1-Acetyl-4-piperidinyl)hydrazino]-5-bromo-2-pyrimidinecarbonitrile.**

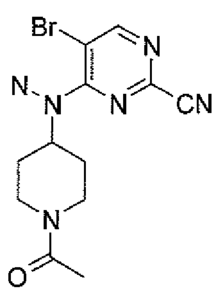

To a solution of Intermediate 37 (3.3 g, 8 mmol) in acetonitrile (300 mL), *p*-toluensulfonic acid (ALDRICH, 2.7 g, 24 mmol) was added and the resulting reaction mixture was stirred at room temperature overnight. Then, more *p*-toluensulfonic acid (2.7 g, 24 mmol) was added and the reaction mixture was stirred for further 3 h at room temperature before heating it to 40- 50 °C for 2 h more. Solvent was removed under reduced pressure and the residue was partitioned between DCM and saturated aqueous sodium bicarbonate. The organic layer was washed with brine and dried over anhydrous $MgSO_4$. Solvent was evaporated *in vacuo* and the crude product was purified by flash chromatography (eluent: Hex/AcOEt mixtures 100:0 to 0:100) to yield the title compound. $^1H$ NMR (300 MHz, $d_6$-DMSO) δ ppm: 8.51 (br., 1H), 4.77 (s, 2H), 4.65- 4.40 (br., 2H), 3.95- 3.81 (m, 1H), 3.21- 3.05 (m, 1H), 2.68- 2.51 (m, 1H), 2.01 (s, 3H), 1.89-1.52 (br., 4H).

Intermediate 39: ***N'*-(1-Acetyl-4-piperidinyl)-*N*'-(5-bromo-2-cyano-4-pyrimidinyl)-4-(bromomethyl)benzohydrazide.**

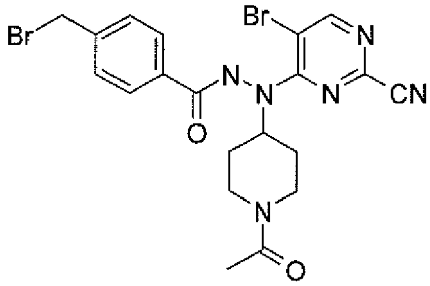

To a solution of Intermediate 38 (0.60 g, 2 mmol) in THF (50 mL), 4-bromomethyl benzoyl bromide (ALDRICH, 0.56 g, 2 mmol) and DIPEA (0.7 mL, 4 mmol) were added and the reaction mixture was stirred at room temperature for 30 minutes to yield the title compound which was used in the following reaction without any further purification.

Intermediate 40: **1-(Diphenylmethyl)-3-azetidinyl methanesulfonate.**

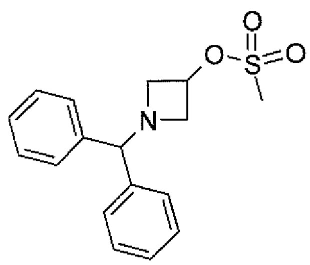

Under a nitrogen atmosphere, triethylamine (0.9 mL, 6.5 mmol) was added to a solution of 1-benzhydrylazetan-3-ol (MAYBRIDGE, 1.033 g, 4.3 mmol) in dry DCM (15 mL), previously cooled to 0 ˚C. After 5 minutes, methanesulfonyl chloride (ALDRICH, 0.4 mL, 5.2 mmol) was added and the resulting reaction mixture was stirred at 0 ˚C for 2 h. Water was then added and product was extracted with DCM. The combined organic layers were dried over anhydrous $MgSO_4$ and solvent was removed *in vacuo* to yield the title compound. $^1$H NMR (300 MHz, DMSO-$d_6$) δ ppm: 7.58- 7.10 (br., 10H), 5.19- 5.03 (br., 1 H), 4.60- 4.42 (br., 1 H), 3.53-3.42 (br., 2H), 3.17 (br.s, 3H), 3.15- 2.97 (br., 2H).

Intermediate 41: **1-[1-(Diphenylmethyl)-3-azetidinyl]pyrrolidine.**

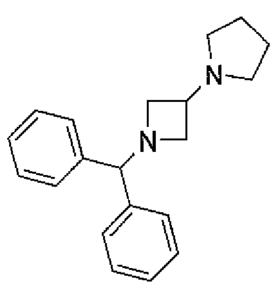

Intermediate 40 (0.5 g, 1.6 mmol) was dissolved in pyrrolidine (ALDRICH, 3 mL, 36 mmol) and the resulting reaction mixture was heated to 60 ˚C for 2 h, then to 70 ˚C for further 2h and finally refluxed for 8 h, before being cooled down to room temperature overnight. A saturated aqueous solution of sodium bicarbonate was added and the product was extracted with DCM. The combined organic layers were dried over anhydrous $Na_2SO_4$ and solvent was evaporated under reduced pressure to yield the title compound. $^1H$ NMR (300 MHz, DMSO-$d_6$) δ ppm: 7.45- 7.36 (m, 4H), 7.31- 7.11 (m, 6H), 4.39 (s, 1H),3.55- 3.46 (m, 1H), 3.13- 3.04 (m, 1H), 3.04- 2.92 (m, 1H), 2.84-2.76 (m, 2H), 2.37- 2.23 (br., 4H), 1.70- 1.56 (br., 4H).

Intermediate 42: **1-(3-Azetidinyl)pyrrolidine dihydrochloride.**

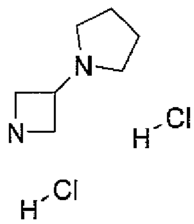

To a solution of Intermediate 41 (1.6 mmol) in EtOH (5.2 mL), 1N HCl (1.4 mL) and palladium hydroxide on carbon (ALDRICH, 0.05 mg, 0.38 mmol) were added and the resulting reaction mixture was hydrogenated at room temperature and 3 bar over the weekend. More 1N HCl (3 mL) was added and the mixture was hydrogenated under the same conditiones for further 4 days. The reaction mixture was then freeze-dried and then MeOH was added. The resulting solution was washed with ETOAc, hexane, DCM and diethyl ether to yield the title compound which was used in subsequent steps without any further purification.

Intermediate 43: **1,1-dimethylethyl 2-(2-methylpropyl)hydrazinecarboxylate.**

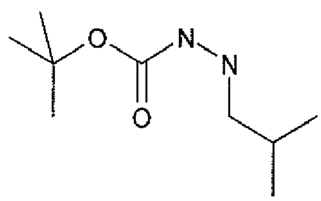

A solution of 1,1-dimethylethyl hydrazinecarboxylate (ALDRICH, 9.2 g, 70 mmol) in i-PrOH (50 ml) was treated at 0˚C with *i*-butylaldehyde (ALDRICH; 6.4 ml, 70 mmol) over 15 min and stirring at 0˚C for 2 h, then the mixture was stirred 5 h at room temperature. To this solution containing the intermediate hydrazone was added $PtO_2$ and the suspension was hydrogenated at room temperature and 2.6 bar for 48 h. The suspension was filtered and the solvent was removed under reduced pressure to give the title compound. $^1H$ NMR (300 MHz, $CDCl_3$) δ ppm: 6.02 (br.s, 1H), 3.92 (br.s, 1H), 2.66 (d, 2H), 1.73 (m, 1H), 1.46 (s, 9H), 0.93 (d, 6H). [ES+ MS] m/z 189 $(MH)^+$.

Intermediate 44: **1,1-dimethylethyl 2-(2,2-dimethylpropyl)hydrazinecarboxylate.**

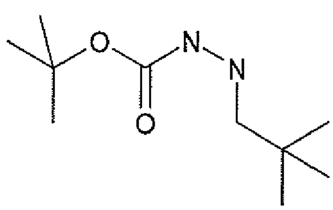

The title compound was prepared by a method analogous to that described for Intermediate 43 replacing *i*-butylaldehyde with trimethylacetaldehyde (ALDRICH). $^1$H NMR (300 MHz, $CDCl_3$) δ ppm: 8.19 (s, 1H), 3.34 (br.s, 1H), 2.46 (d, 2H), 1.37 (s, 9H), 0.85 (s, 9H) [ES+ MS] m/z 203 $(MH)^+$.

Intermediate 45: **1,1-dimethylethyl 2-(5-bromo-2-chloro-4-pyrimidinyl)-2-(2,2-dimethylpropyl)hydrazinecarboxylate.**

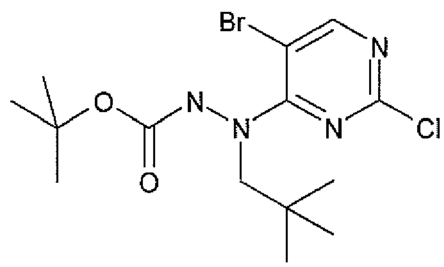

To a solution of 5-bromo-2,4-dichloropyrimidine (15.4 g, 68 mmol) and Intermediate 44 (12.5 g, 62 mmol) in *i*-PrOH (150 mL), N,N-diisopropylethylamine (14 mL, 80 mmol) was added and the resulting reaction mixture was refluxed for 2.5 h, then stirred at room temperature overnight and again refluxed for further 3h. The mixture was concentrated under reduced pressure and the residue partitioned between DCM and 1 M ammonium chloride. The organic layer was treated with brine and dried over anhydrous $MgSO_4$. The residue was purified by flash chromatography (elute: Hex/EtOAc mixtures 95:1 to 1:1) to give the title compound. $^1$H NMR (300 MHz, $CDCl_3$) δ ppm: 8.27 (br.s, 1H), 6.85 (br.s, 1H), 4.85-4.63 (br.m, 1H), 2.90-2.65 (br.m, 1H), 1.47 (s, 9H), 0.98 (s, 9H). [ES+ MS] m/z 393 $(M)^+$.

Intermediate 46: **1,1-dimethylethyl 2-(5-bromo-2-cyano-4-pyririmidinyl)-2-(2,2-dimethylpropyl)hydrazinecarboxylate.**

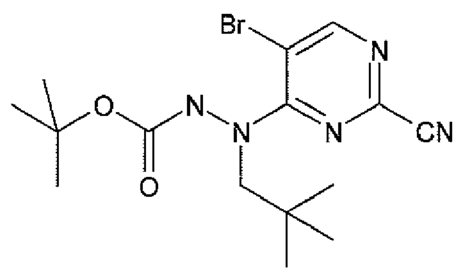

Potassium cyanide (1.6 g, 25 mmol) was added to a suspension of Intermediate 45 (9 g, 23 mmol) and DABCO (2.6 g, 23 mmol) in a mixture of DMSO/$H_2O$ 9:1 (100 mL) at room temperature. The reaction mixture was heated at 80 ˚C for 1.5 h, and then poured into iced water. After being stirred for 1.5h, the yellow product that precipitated was filtered off and washed abundantly with water The compound was redissolved in DCM and the resulting solution was washed with water (twice) and brine and the organic layer was dried over $MgSO_4$. The compound was purified by flash chromatography (eluent: Hex/EtOAC 7:3) to give the title compound. $^1$H NMR (300 MHz, $CDCl_3$) δ ppm: 8.47 (br.s, 1H), 6.86 (br.s, 1H), 4.85-4.65 (br.m, 1H), 2.90-2.70 (br.m, 1H), 1.47 (s, 9H), 0.99 (s, 9H). [ES+ MS] m/z 384 $(M)^+$.

Intermediate 47: **5-bromo-4-[1-(2,2-dimethylpropyl)hydrazino]-2-pyrimidine carbonitrile.**

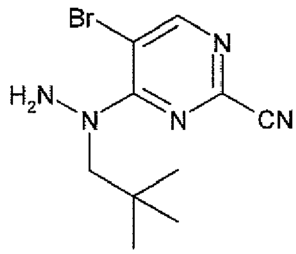

To a solution of Intermediate 46 (2 g, 5.2 mmol) in dry acetonitrile (100 mL), *p*-toluenesulfonic acid (13 mmol) was added and the resulting reaction mixture was stirred at room temperature overnight. The mixture was then concentrated *in vacuo* and the residue partitioned between DCM and a saturated solution of sodium bicarbonate. The organic layer was washed with brine and dried over anhydrous $NaHCO_3$. The residue was purified by preparative HPLC (X-TERRA 19x150 mm, ACN:$H_2O$, 0.1%TFA, gradient 10-100%) to give the title compound. $^1H$ NMR (300 MHz, $CDCl_3$) δ ppm: 8.39 (s, 1H), 3.85 (s, 2H), 1.00 (s, 9H) [ES+ MS] m/z 284 ($M^+$).

Intermediate 48: **methyl 4-[(4-methyl-1-piperazinyl)methyl]benzoate.**

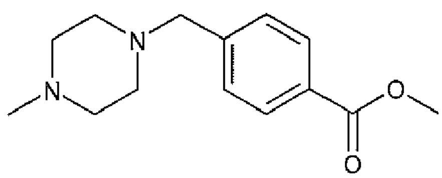

A solution of N-methylpiperazine (ALDRICH, 1.46 ml, 13.1 mmol) in dimethylformamide (5 ml) was cooled to 0° C and, then, potassium carbonate (1.81 g, 13.1 mmol) was added. This mixture was stirred at 0° C for 30 min. Then, methyl 4-(bromomethyl) benzoate (ALDRICH, 3 g, 13.1 mmol) was added. The reaction mixture was allowed to warm up to room temperature and stirred for 17 h. The mixture was concentrated under reduce pressure. The residue was dissolved in DCM and washed with water, the aqueous layer was extracted with DCM. The organic layers were combined, washed with water, dried over $MgSO_4$ filtered and the solvent removed under reduce pressure to give the title compound. $^1H$ RMN (300 MHz, CDCl3-d6): 7.97 (d, 2H), 7 40 (d, 2H), 3.90 (s, 3H), 3.55 (s, 2H), 2.47 (br. m, 8H), 2.28 (s, 3H).

Intermediate 49: **4-[(4-methyl-1-piperazinyl)methyl]benzoic acid.**

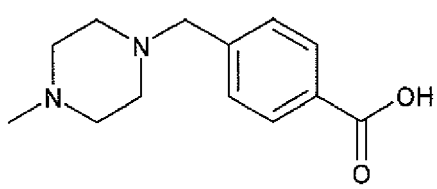

A solution of lithium hydroxide (ALDRICH, 337 mg, 14.1 mmol) in $H_2O$ (10 ml) was added to a solution of Intermediate 48 (1.4 g, 5.63 mmol) in MeOH (20 ml) and the mixture was heated at reflux for 2h. The mixture was concentrated under reduced pressure. The residue was dissolved in DCM and 2N hydrochloric acid was added to give pH 5. The aqueous layer was partitioned with n-Butanol (5 times) and the fractions were combined, dried over $MgSO_4$,

filtered and evaporated under reduce pressure to give the title compound as a white solid. $^1$H RMN (300 MHz, DMSO-d6): 12.83 (br. m, 1H), 11.05 (br. m, 1H), 7.90 (d, 2H), 7.43 (d, 2H), 4.36 (m, 1H), 3.60 (s, 2H), 3.38-2.80 (br. m, 8H), 2.68 (s, 3H). [ES+ MS] m/z 235 $(MH)^+$.

Examples

Example 1: ***N'*-(5-bromo-2-cyano-4-pyrimidinyl)-N'-cyclopentyl-4-[(4-methyl-1-piperazinyl)methyl]benzohydrazide.**

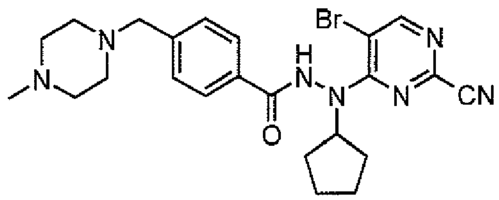

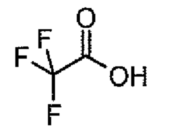

To a stirred mixture of the Intermediate 4 (84 mg, 0.30 mmol) and DIPEA (FLUKA, 0.1 ml, 0.6 mmol) in THF (5 mL), 4-(chloromethyl)benzoyl chloride (ALDRICH, 68 mg, 0.36 mmol) was added and stirring was continued for 2 hours at room temperature. After this time, N-methylpiperazine (ALDRICH, 0.07 mL, 0.6 mmol) was added and the mixture was stirred at room temperature overnight. The solvent was evaporated under reduced pressure and the resulting crude product was purified by preparative HPLC (XTERRA 19x150 mm, ACN:$H_2O$, 0.1%TFA, gradient 30-100%) to give the title compound. $^1$H NMR (300 MHz, $d_6$-DMSO) δ ppm: 11.16 (s, 1H), 8.63 (s, 1H), 7.89 (dd, 2H), 7.47 (dd, 2H), 4.93 (m, 1H), 3.32-3.43 (m, 2H), 2.88-3.08 (m, 4H), 2.78 (s, 3H), 2.25-2.44 (m, 2H), 1.84-1.97 (m, 3H), 1.45-1.68 (m, 5H). [ES+ MS] m/z 498 $(MH)^+$.

Example 2: **N'-(5-bromo-2-cyano-4-pyrimidinyl)-N'-[(1R,2S+1S,2R)-2-methylcyclo pentyl]-4-[(4-methyl-1-piperazinyl)methyl]benzohydrazide trifluoroacetate.**

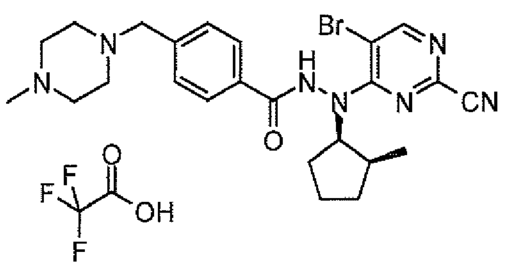

+

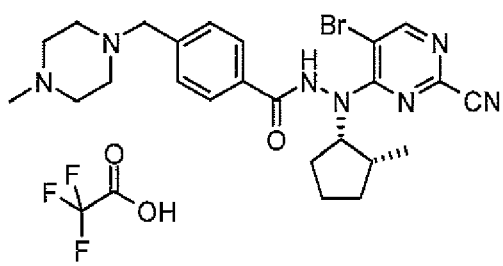

To a solution of Intermediate 9 (0.14 g, 0.5 mmol) in dry THF (4 mL), 4-(chloromethyl) benzoyl chloride (ALDRICH, 0.14 g, 0.73 mmol) and DIPEA (0.18 mL, 1.05 mmol) were added and the resulting reaction mixture was stirred at room temperature for 22 h. N-methylpiperazine (ALDRICH, 0.07 mL, 0.6 mmol) and catalytic sodium iodide were added, and the reaction mixture was stirred at room temperature for further 7 h. Then, more N, N-diisopropylethylamine (0.18 mL, 1.05 mmol) was added in order to drive the reaction to completion. After 40 h, the mixture was concentrated *in vacuo* and the crude reaction mixture was purified first by flash chromatography (elute DCM/MeOH 100:0 to 19:1) and then by preparative HPLC (SUNFIRE 19x150 mm, ACN:$H_2O$ 0.1% TFA, gradient 10-100%) to give the title compound. $^1$H NMR (300 MHz, d6-DMSO, 80˚C) δ ppm: 10.94 (br.s, 1H), 8.62 (s, 1H), 7.9 (d, 2H), 7.47 (d, 2H), 4.82-4.72 (m, 1H), 4.20- 3.56 (br., 4H), 3.38- 3.02 (br., 4H), 2.79 (s, 3H), 2.79- 2.42 (br., 3H), 2.04-1.85 (m, 2H), 1.84- 1.63 (m, 2H), 1.62- 1.33 (m, 2H), 0.85 (d, 3H); [ES+ MS] m/z 512 $(MH)^+$.

**Example 3: *N*'-(5-bromo-2-cyano-4-pyrimidinyl)-*N*'-[(1*R*,2*R*+1*S*,2*S*)-2-methylcyclo pentyl]-4-[(4-methyl-1-piperazinyl)methyl]benzohydrazide trifluoroacetate.**

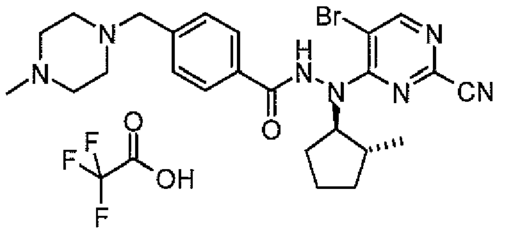

+

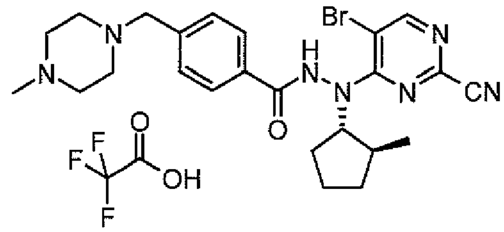

To a solution of Intermediate 12 (0.20 g, 0.7 mmol) in dry THF (4 mL), 4-(chloromethyl)benzoyl chloride (ALDRICH, 0.16g, 0.883 mmol) and DIPEA (0,23 mL, 1.37 mmol) were added and the resulting reaction mixture was stirred at r.t. for 4 h. Then, N-methylpiperazine (ALDRICH, 0.09 mL, 0.8 mmol) and catalytic sodium iodide were added, and the reaction mixture was stirred at room temperature for further 7 h. Then, more DIPEA (0.23 mL, 1.4 mmol) was added in order to drive the reaction to completion. After 40 h, the mixture was concentrated *in vacuo* and the crude reaction mixture was purified first by flash chromatography (elute DCM/MeOH 100:0 to 19:1) and then by preparative HPLC (SUNFIRE 19x150 mm, ACN:$H_2O$ 0.1% TFA, gradient 10-100%) to give the title compound. $^1H$ NMR (300 MHz, d6-DMSO, 80°C) δ ppm: 10.90 (br.s, 1H), 8.57 (s, 1H), 7.9 (d, 2H), 7.46 (d, 2H), 4.80- 4.58 (br., 1H), 3.69 (s, 2H), 3.43- 2.97 (br., 4H), 2.78 (s, 3H), 2.73- 2.54 (br., 2H), 2.43- 2.04 (br., 2H), 2.04-1.75 (br., 3H), 1.71- 1.49 (m, 2H), 1.35- 1.16 (m, 2H), 1.07 (d, 3H); [ES+ MS] m/z 512 $(MH)^+$.

Example 4: ***N*'-(5-bromo-2-cyano-4-pyrimidinyl)-N'-(3-methylcyclopentyl)-4-{[4-(4-methyl-1-piperazinyl)-1-piperidinyl]methyl}benzohydrazide trifluoroacetate.**

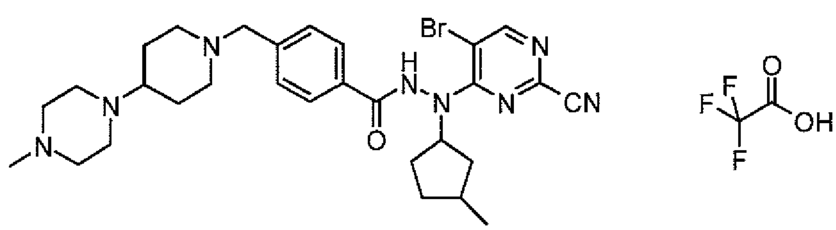

To a solution of Intermediate 17 (0.1 g, 0.2 mmol) in dry ACN (5 mL), 1-methyl-4-(piperidin-4-yl)-piperazine (FLUOROCHEM, 0.18 g, 0.94 mmol), DIPEA (0.08 mL, 0.44 mmol) and catalytic sodium iodide were added and the resulting reaction mixture was stirred at room temperature overnight. Then, the mixture was concentrated *in vacuo* and the crude reaction mixture was purified by preparative HPLC (X-TERRA 19x150 mm, ACN:$H_2O$ 0.1% TFA, gradient 20-100%) to give the title compound as a mixture of diastereomers, present in a non 1:1 ratio. $^1H$ NMR (300 MHz, d6-DMSO) δ ppm: 11.34-11.23 (br., 1H), 10.02- 9.75 (br., 1H), 8.65 (s, 1H), 7.99 (d, 2H), 7.65 (d, 2H), 5.17- 4.86 (br., 1H), 4.50- 3.64 (br., 4H), 3.55- 3.24 (br., 4H), 3.23- 2.85 (br., 6H), 2.84- 2.56 (br., 4H), 2.46- 1.45 (br., 10H), 1.26- 1.05 (br., 1H), 1.04- 0.88 (m, 3H); [ES+ MS] m/z 595 $(MH)^+$.

Example 5: **N'-(5-bromo-2-cyano-4-pyrimidinyl)-N'-(3-methylcyclopentyl)-4-({4-[(1-methyl-4-piperidinyl)methyl]-1-piperazinyl}methyl)benzohydrazide trifluoroacetate.**

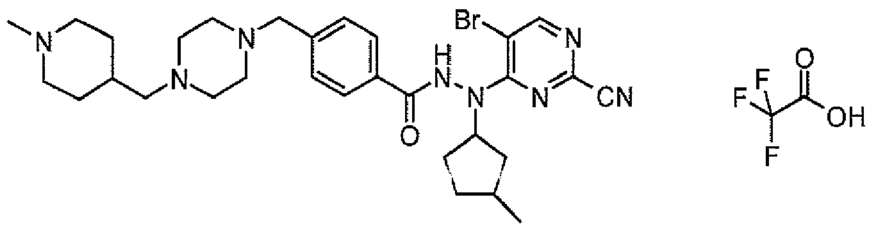

To a solution of Intermediate 17 (0.1 g, 0.2 mmol) in dry ACN (5 mL), 1-(N-methyl-4-piperidinmethyl)-piperazine (FLUOROCHEM, 0.05 g, 0.3 mmol), DIPEA (0.08 mL, 0.44 mmol) and catalytic sodium iodide were added and the resulting reaction mixture was stirred at room temperature overnight. Then, the mixture was concentrated *in vacuo* and the crude reaction mixture was purified by preparative HPLC (X-TERRA 19x150 mm, ACN:$H_2O$ 0.1% TFA, gradient 10-80%) to give the title compound as a mixture of diastereomers, present in a non 1:1 ratio.. $^1H$ NMR (300 MHz, d6-DMSO) δ ppm: 11.29- 11.17 (br., 1H), 9.54- 9.37 (br., 1H), 8.64 (s, 1H), 7.94 (d, 2H), 7.54 (d, 2H), 5.14- 4.87 (br., 1H), 4.08- 2.56 (br., 20H), 2.24- 1.44 (br., 8H), 1.44- 1.05 (br., 3H), 1.04- 0.89 (m, 3H); [ES+ MS] m/z 609 $(MH)^+$.

Example 6: ***N'*-(5-bromo-2-cyano-4-pyrimidinyl)-AF-cyclopentyl-4-{[4-(4-morpholinyl)-1-piperidinyl]methyl} benzohydrazide trifluoroacetate.**

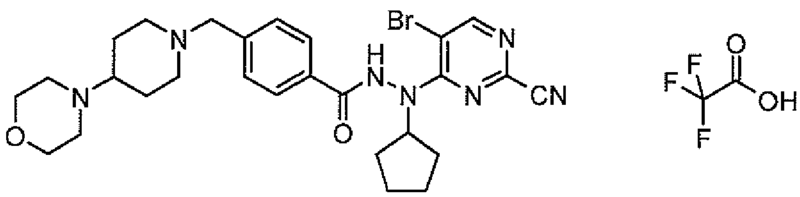

Intermediate 18 (75 mg, 0.173 mmol) was dissolved in ACN (5 mL) and, then, DIPEA (FLUKA, 0.05 ml, 0.288 mmol), cat. sodium iodide and 4-morpholinopiperidine (ALDRICH, 0.035 g, 0.21 mmol) were successively added. The reaction mixture was stirred at room temperature overnight. After this time, DIPEA (half equivalent) was added and the reaction mixture was stirred at 50 ˚C until completion. The solvent was evaporated under reduced pressure and the resulting crude product was purified by preparative HPLC (XTERRA, ACN:$H_2O$, 0.1%TFA, gradient 10-100%) to give the title compound. $^1H$ NMR (300 MHz, $d_6$-DMSO) δ ppm: 11.26 (s, 1H), 8.64 (s, 1H), 7.99 (dd, 2H), 7.62 (dd, 2H), 4.95 (m, 1H), 4.45-4.25 (m, 2H), 3.90-2.10 (m, 17H), 1.97-1.84 (m, 3H), 1.68-1.45 (m, 5H). [ES+ MS] m/z 568 $(MH)^+$.

Example 7: ***N'*-(5-bromo-2-cyano-4-pyrimidinyl)-*N'*-cyclopentyl-4-({4-[(1-methyl-4-piperidinyl)methyl]-1-piperazinyl}methyl)benzohydrazide trifluoroacetate.**

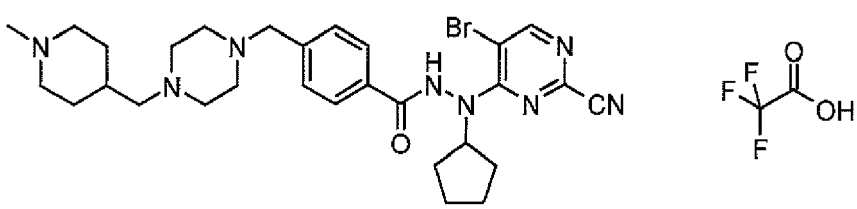

Intermediate 18 (75 mg, 0.173 mmol) was dissolved in ACN (5 mL) and, then, DIPEA (FLUKA, 0.05 ml, 0.288 mmol), cat. sodium iodide and 1-(N-methyl-4-piperidin methyl) piperazine (ALDRICH, 0.041 g, 0.21 mmol) were successively added. The reaction mixture was stirred at room temperature overnight. After this time, DIPEA (half equivalent) was added and the reaction mixture was stirred at 50 ˚C until completion.

The solvent was evaporated under reduced pressure and the resulting crude product was purified by preparative

HPLC (XTERRA, ACN:$H_2O$, 0.1%TFA, gradient 10-100%) to give the title compound. $^1$H NMR (300 MHz, $d_6$-DMSO) δ ppm: 11.20 (s, 1H), 9.40-9.30 (m, 1H), 8.63 (s, 1H), 7.93 (dd, 2H), 7.53 (dd, 2H), 4.94 (m, 1H), 4.45-2.10 (m, 24H), 1.97-1.84 (m, 3H), 1.68-1.45 (m, 5H). [ES+ MS] m/z 595 $(MH)^+$.

Example 8: ***N'*-(5-bromo-2-cyano-4-pyrimidinyl)-*N'*-cyclopentyl-4-({4-[(1-methyl-3-piperidinyl)methyl]-1-piperazinyl}methyl)benzohydrazide trifluoroacetate.**

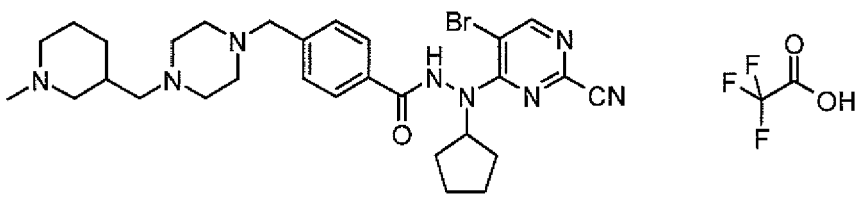

Intermediate 18 (75 mg, D.173 mmol) was dissolved in ACN (5 mL) and, then, DIPEA (FLUKA, 0.05 ml, 0.288 mmol), cat. sodium iodide and 1-(N-methyl-3-piperidylmethyl) piperazine (FLUOROCHEM, 41 mg, 0.21 mmol) were successively added. The reaction mixture was stirred at room temperature overnight. After this time, DIPEA (half equivalent) was added and the reaction mixture was stirred at 50 ˚C until completion. The solvent was evaporated under reduced pressure and the resulting crude product was purified by preparative HPLC (XTERRA, ACN:$H_2O$, 0.1%TFA, gradient 10-100%) to give the title compound. $^1$H NMR (300 MHz, $d_6$-DMSO) δ ppm: 11.23 (s, 1H), 9.40-9.30 (m, 1H), 8.64 (s, 1H), 7.96 (dd, 2H), 7.7-7.5 (m, 2H), 4.94 (m, 1H), 4.45-2.10 (m, 24H), 1.97-1.84 (m, 3H), 1.88-1.45 (m, 5H). [ES+ MS] m/z 595 $(MH)^+$.

Example 9: ***N'*-(5-bromo-2-cyano-4-pyrimidinyl)-*N'*-cyclopentyl-4-({4-[(4-methyl-1-piperazinyl)carbonyl]-1-piperidinyl}methyl)benzohydrazide trifluoroacetate.**

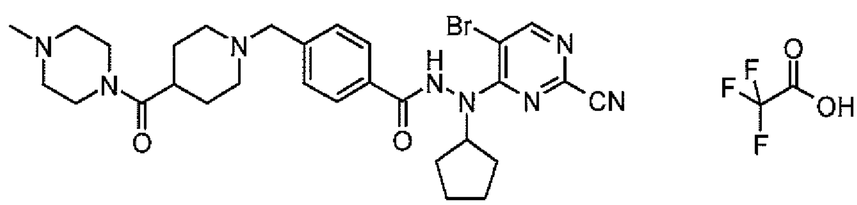

Intermediate 18 (75 mg, 0.173 mmol) was dissolved in ACN (5 mL) and, then, DIPEA (FLUKA, 0.05 ml, 0.288 mmol), cat. sodium iodide and (4-methyl-piperazin-1-yl)-piperidin-4-yl-methanone (FLUOROCHEM, 44 mg, 0.21 mmol) were successively added. The reaction mixture was stirred at room temperature overnight. After this time, DIPEA (half equivalent) was added and the reaction mixture was stirred at 50 ˚C until completion. The solvent was evaporated under reduced pressure and the resulting crude product was purified by preparative HPLC (XTERRA, ACN:$H_2O$, 0.1%TFA, gradient 10-100%) to give the title compound. $^1$H NMR (300 MHz, $d_6$-DMSO) δ ppm: 11.28 (s, 1H), 10.10-9.70 (m, 2H), 8.65 (s, 1H), 8.00 (dd, 2H), 7.65 (dd, 2H), 4.94 (m, 1H), 4.45-2.10 (m, 22H), 1.97-1.84 (m, 3H), 1.68-1.45 (m, 5H). [ES+ MS] m/z 609 $(MH)^+$.

Example 10: ***N'*-(5-bromo-2-cyano-4-pyrimidinyl)-*N'*-cyclopentyl-4-{[4-(4-methyl-1-piperazinyl)-1-piperidinyl] methyl}benzohydrazide trifluoroacetate**.

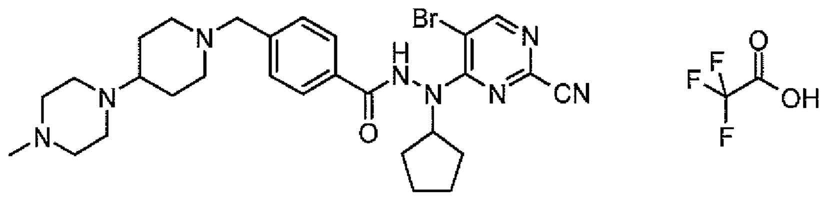

Intermediate 18 (75 mg, 0.173 mmol) was dissolved in ACN (5 mL) and, then, DIPEA (FLUKA, 0.05 ml, 0.288 mmol), cat. sodium iodide and 1-methyl-4-(piperidin-4yl)-piperazine (FLUOROCHEM, 38 mg, 0.21 mmol) were successively added. The reaction mixture was stirred at room temperature overnight. After this time, DIPEA (half equivalent) was added and the reaction mixture was stirred at 50°C until completion. The solvent was evaporated under reduced pressure and the resulting crude product was purified by preparative HPLC (XTERRA, ACN:$H_2O$, 0.1%TFA, gradient 10-100%) to give the title compound. $^1H$ NMR (300 MHz, $d_6$-DMSO) δ ppm: 11.27 (s, 1H), 10.0-9.4 (m, 2H), 8.64 (s, 1H), 8.00 (dd, 2H), 7.63 (dd, 2H), 4.94 (m, 1H), 3.90-2.10 (m, 22H), 1.97-1.84 (m, 3H), 1.68-1.45 (m, 5H). [ES+ MS] m/z 581 $(MH)^+$.

Example 11: ***N'*-(5-bromo-2-cyano-4-pyrimidinyl)-*N'*-cyclopentyl-3-{[4-(4-methyl-1-piperazinyl)-1-piperidinyl] methyl}benzohydrazide trifluoroacetate.**

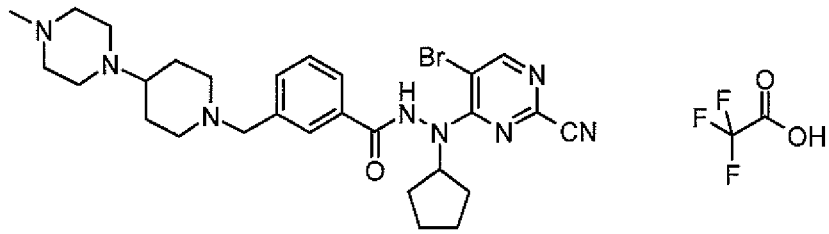

Intermediate 19 (75 mg, 0.173 mmol) was dissolved in ACN (5 mL) and, then, DIPEA (FLUKA, 0.06 ml, 0.344 mmol), cat sodium iodide and 1-methyl-4-(piperidin-4yl)-piperazine (FLUOROCHEM, 38 mg, 0.21 mmol) were successively added. The reaction mixture was stirred at room temperature overnight. The reaction crude was filtered and the solvent was evaporated under reduced pressure and the resulting crude product was purified by preparative HPLC (XTERRA, ACN:$H_2O$, 0.1%TFA, gradient 10-100%) to give the title compound. $^1H$ NMR (300 MHz, $d_6$-DMSO) δ ppm: 11.29 (s, 1H), 9.8-9.5 (m, 2H), 8.64 (s, 1H), 8.15-7.55 (m, 4H), 4.95 (m, 1H), 3.90-2.10 (m, 22H), 1.97-1.84 (m, 3H), 1.68-1.45 (m, 5H). [ES+ MS] m/z 581 $(MH)^+$.

Example 12: ***N'*-(5-bromo-2-cyano-4-pyrimidinyl)-*N'*-cyclopentyl-3-({4-[(4-methyl-1-piperazinyl)carbonyl]-1-piperidinyl}methyl)benzohydrazide trifluoroacetate.**

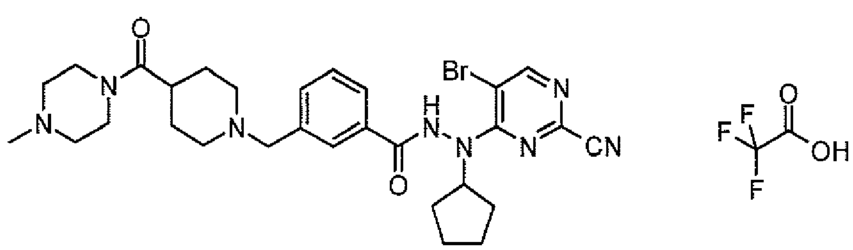

Intermediate 19 (75 mg, 0.173 mmol) was dissolved in ACN (5 mL) and, then, DIPEA (FLUKA, 0.06 ml, 0.344 mmol), cat. sodium iodide and (4-methyl-piperazin-1-yl)-piperidin-4-yl-methanone (FLUOROCHEM, 44 mg, 0.21 mmol)

were successively added. The reaction mixture was stirred at room temperature overnight. The reaction crude was filtered and the solvent was evaporated under reduced pressure and the resulting crude product was purified by preparative HPLC (XTERRA, ACN:$H_2O$, 0.1%TFA, gradient 10-100%) to give the title compound. $^1H$ NMR (300 MHz, $d_6$-DMSO) δ ppm: 11.30 (s, 1H), 10.05-9.55 (m, 2H), 8.65 (s, 1H), 8.15-7.60 (m, 4H), 4.95 (m, 1H), 3.90-2.10 (m, 22H), 1.97-1.84 (m, 3H), 1.68-1.45 (m, 5H). [ES+ MS] m/z 609 $(MH)^+$.

Example 13: ***N'*-(5-bromo-2-cyano-4-pyrimidinyl)-*N'*-cyclopentyl-3-{[4-(4-morpholinyl)-1-piperidinyl]methyl} benzohydrazide trifluoroacetate.**

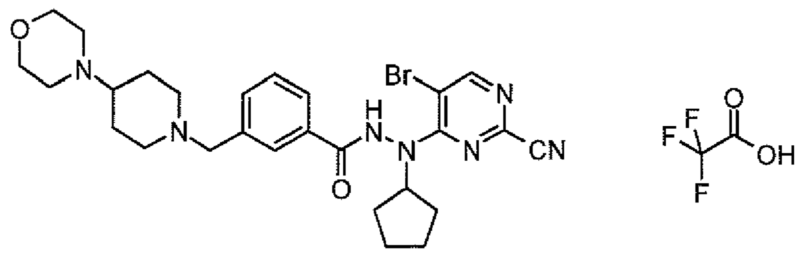

Intermediate 19 (75 mg, 0.173 mmol) was dissolved in ACN (5 mL) and, then, DIPEA (FLUKA, 0.06 ml, 0.344 mmol), cat. sodium iodide and 4-morpholinopiperidine (ALDRICH, 35 mg, 0.21 mmol) were successively added. The reaction mixture was stirred at room temperature overnight. The reaction crude was filtered and the solvent was evaporated under reduced pressure and the resulting crude product was purified by preparative HPLC (XTERRA, ACN:$H_2O$, 0.1%TFA, gradient 10-100%) to give the title compound. $^1H$ NMR (300 MHz, $d_6$-DMSO) δ ppm: 11.29 (s, 1H), 10.5-9.7 (m, 2H), 8.64 (s, 1H), 8.15-7.60 (m, 4H), 4.95 (m, 1H), 3.90-2.10 (m, 19H), 1.97-1.84 (m, 3H), 1.68-1.45 (m, 5H). [ES+ MS] m/z 568 $(MH)^+$.

Example 14: ***N'*-(5-bromo-2-cyano-4-pyrimidinyl)-*N'*-cyclopentyl-3-({4-[(1-methyl-3-piperidinyl)methyl]-1-piperazinyl}methyl)benzohydrazide trifluoroacetate.**

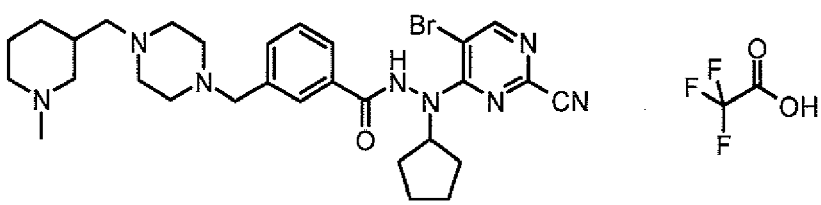

Intermediate 19 (75 mg, 0.173 mmol) was dissolved in ACN (5 mL) and, then, DIPEA (FLUKA, 0.06 ml, 0.344 mmol), cat. sodium iodide and 1-(N-methyl-3-piperidylmethyl) piperazine (FLUOROCHEM, 41 mg, 0.21 mmol) were successively added. The reaction mixture was stirred at room temperature overnight. The reaction crude was filtered and the solvent was evaporated under reduced pressure and the resulting crude product was purified by preparative HPLC (XTERRA, ACN:$H_2O$, 0.1%TFA, gradient 10-100%) to give the title compound. $^1H$ NMR (300 MHz, $d_6$-DMSO) δ ppm: 11.26 (s, 1H), 9.2-9.5 (m, 1H), 8.64 (s, 1H), 8.2-7.5 (m, 4H), 4.95 (m, 1H), 3.90-2.10 (m, 24H), 1.97-1.84 (m, 3H), 1.68-1.45 (m, 5H). [ES+ MS] m/z 595 $(MH)^+$.

Example 15: ***N'*-(5-bromo-2-cyano-pyrimidinyl)-*N'*-cyclopentyl-3-({4-[(1-methyl-4-piperidinyl)methyl]-1-piperazinyl}methyl)benzohydrazide trifluoroacetate.**

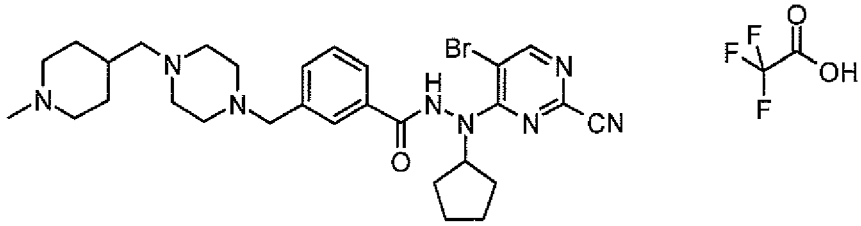

Intermediate 19 (75 mg, 0.173 mmol) was dissolved in ACN (5 mL) and, then, DIPEA (FLUKA, 0.06 ml, 0.344 mmol), cat. sodium iodide and 1-(N-methyl-4-piperidin methyl)piperazine (ALDRICH, 41 mg, 0.21 mmol) were successively added. The reaction mixture was stirred at room temperature overnight. The reaction crude was filtered and the solvent was evaporated under reduced pressure and the resulting crude product was purified by preparative HPLC (XTERRA, ACN:$H_2O$, 0.1%TFA, gradient 10-100%) to give the title compound. $^1H$ NMR (300 MHz, $d_6$-DMSO) δ ppm: 11.23 (s, 1H), 9.6-9.2 (m, 1H), 8.64 (s, 1H), 8.2-7.5 (m, 4H), 4.94 (m, 1H), 3.90-2.10 (m, 24H), 1.97-1.84 (m, 3H), 1.68-1.45 (m, 5H). [ES+ MS] m/z 595 $(MH)^+$.

Example 16: ***N'*-(5-bromo-2-cyano-4-pyrimidinyl)-*N'*-cyclohexyl-4-[(4-methyl-1-piperazinyl)methyl]benzohydrazide trifluoroacetate.**

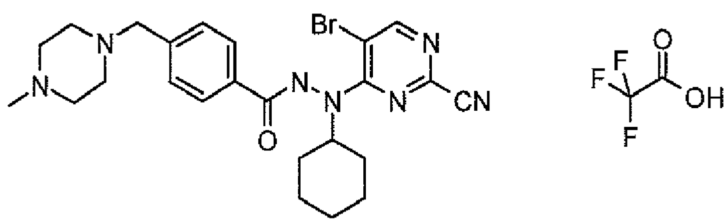

To a solution of Intermediate 23 (0.643 g, 2.17 mmol) in dry THF (10 mL), 4-(chloromethyl)benzoyl chloride (ALDRICH, 0.45 g, 2.4 mmol) and DIPEA (0.7 mL, 4.35 mmol) were added and the resulting reaction mixture was stirred at room temperature overnight. Then, N- methylpiperazine (ALDRICH, 0.29 mL, 2.6 mmol) and catalytic sodium iodide were added, and the reaction mixture was stirred at room temperature for further 24h. Then, the mixture was concentrated *in vacuo* and the crude reaction mixture was purified by preparative HPLC (X-TERRA 50x250 mm, ACN:$H_2O$ 0.1% TFA, gradient 10-100%) to give the title compound. $^1H$ NMR (300 MHz, d6-DMSO) δ ppm: 11.08 (br.s, 1H), 8.61 (s, 1H), 7.92 (d, 2H), 7.48 (d, 2H), 4.64- 4.50 (m, 1H), 3.83-3.63 (br., 2H), 3.51- 3.24 (br., 2H), 3.19- 2.88 (br., 4H), 2.79 (s, 3H), 2.46-2.19 (br., 2H), 1.98-1.50 (br. m, 6H), 1.50- 0.92 (br. m, 4H); [ES+ MS] m/z 512 $(MH)^+$.

Example 17: ***N'*-(5-bromo-2-cyano-4-pyrimidinyl)-N'-cyclohexyl-4-{[4-(4-methyl-1-piperazinyl)-1-piperidinyl]methyl}benzohydrazide trifluoroacetate.**

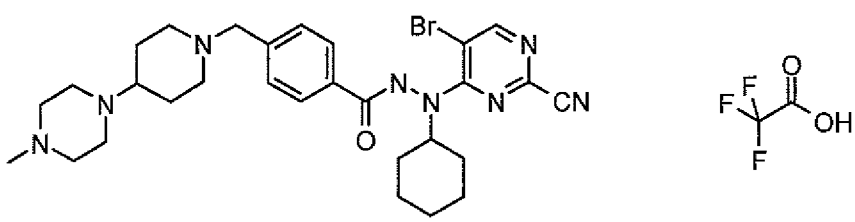

To a solution of Intermediate 24 (0.21 g, 0.5 mmol) in dry ACN (5 mL), 1-methyl-4-(piperidin-4-yl)-piperazine

(FLUOROCHEM, 0.11 g, 0.6 mmol), DIPEA (0.17 mL, 0.96 mmol) and catalytic sodium iodide were added and the resulting reaction mixture was stirred at room temperature overnight. Then, the mixture was concentrated *in vacuo* and the crude reaction mixture was purified by preparative HPLC (SUNFIRE 19x150 mm, ACN:$H_2O$ 0.1% TFA, gradient 10-100%) to give the title compound. $^1H$ NMR (300 MHz, d6-DMSO) δ ppm: 11.19 (s, 1H), 10.06- 9.81 (br., 1H), 8.63 (s, 1H), 8.01 (d, 2H), 7.64 (d, 2H), 4.64- 4.51 (m, 1H), 4.50- 3.70 (br., 4H), 3.54- 3.30 (br., 4H), 3.23- 2.86 (br., 6H), 2.77 (s, 3H), 2.70- 2.55 (br., 1H), 2.10- 1.50 (br., 10H), 1.51- 1.25 (br., 2H), 1.25- 0.96 (br., 2H); [ES+ MS] m/z 595 $(MH)^+$.

Example 18: ***N'*-(5-bromo-2-cyano-4-pyrimidinyl)-N'-cyclohexyl-4-{[4-(4-morpholinyl)-1-piperldinyl]methyl}benzohydrazide trifluoroacetate.**

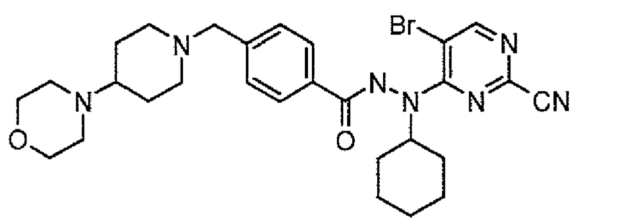

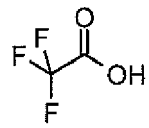

To a solution of Intermediate 24 (0.1 g, 0.22 mmol) in dry ACN (5 mL), 4-morpholinopiperidine (ALDRICH, 0.05 g, 0.3 mmol), DIPEA (0.08 mL, 0.44 mmol) and catalytic sodium iodide were added and the resulting reaction mixture was stirred at room temperature overnight. Then, the mixture was concentrated *in vacuo* and the crude reaction mixture was purified by preparative HPLC (SUNFIRE 19x150 mm, ACN:$H_2O$ 0.1 % TFA, gradient 10-100%) to give the title compound. $^1H$ NMR (300 MHz, d6-DMSO) δ ppm: 11.18 (s, 1H), 10.50- 9.98 (br., 1H), 8.63 (s, 1H), 8.01 (d, 2H), 7.63 (d, 2H), 4.66- 4.51 (m, 1H), 4.47- 3.57 (br., 6H), 3.57- 2.77 (br., 8H), 2.33- 2.15 (br., 2H), 2.13-1.68 (br., 7H), 1.68- 1.52 (br., 2H), 1.52- 1.28 (br., 2H), 1.28- 0.96 (br., 2H); [ES+ MS] m/z 582 $(MH)^+$.

Example 19: ***N'*-(5-bromo-2-cyano-4-pyrimidinyl)-N'-cyclohexyl-4-({4-[(1-methyl-4-piperidinyl)methyl]-1-piperazinyl}methyl)benzohydrazide trifluoroacetate.**

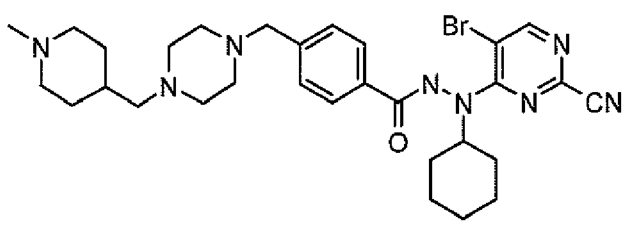

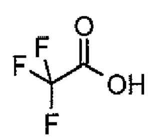

To a solution of Intermediate 24 (0.1 g, 0.22 mmol) in dry ACN (5 mL), 1-(N-methyl-4-piperidinmethyl)piperazine (FLUOROCHEM, 0.05 g, 0.3 mmol), DIPEA (0.8 mL, 0.45 mmol) and catalytic sodium iodide were added and the resulting reaction mixture was stirred at room temperature overnight. Then, the mixture was concentrated *in vacuo* and the crude reaction mixture was purified by preparative HPLC (SUNFIRE 19x150 mm, ACN:$H_2O$ 0.1% TFA, gradient 10-100%) to give the title compound. $^1H$ NMR (300 MHz, d6-DMSO) δ ppm: 11.11 (s, 1H), 9.56- 9.32 (br., 1H), 8.62 (s, 1 H), 7.96 (d, 2H), 7.58- 7.49 (m, 2H), 4.71- 4.50 (m, 1H), 4.17- 2.60 (br., 19H), 1.97- 1.53 (br., 9H), 1.53-0.93 (br., 6H); [ES+ MS] m/z 609 $(MH)^+$.

Example 20: ***N*'-(5-bromo-2-cyano-4-pyrimidinyl)-N'-cyclohexyl-3-{[4-(4-methyl-1-piperazinyl)-1-piperidinyl]methyl}benzohydrazide trifluoroacetate.**

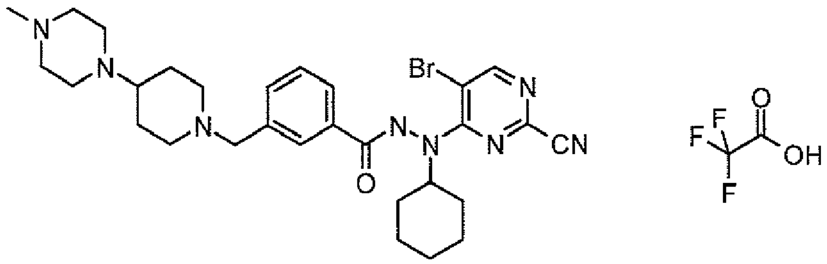

To a solution of Intermediate 25 (12 mg, 0.03 mmol) in dry ACN (4 mL), 1-methyl-4-(piperidin-4-yl)-piperazine (FLUOROCHEM, 7 mg, 0.03 mmol), DIPEA (0.01 mL, 0.05 mmol) and catalytic sodium iodide were added and the resulting reaction mixture was stirred at room temperature overnight. Then, the mixture was concentrated *in vacuo* and the crude reaction mixture was purified by preparative HPLC (SUNFIRE 19x150 mm, ACN:$H_2O$ 0.1% TFA, gradient 10-100%) to give the title compound. $^1H$ NMR (300 MHz, d6-DMSO) δ ppm: 11.20 (s, 1H), 9.94- 9.71 (br., 1H), 8.62 (s, 1H), 8.15- 7.95 (br., 2H), 7.80- 7.60 (m, 2H), 4.64- 4.51 (m, 1H), 4.50- 3.56 (br., 4H), 3.52- 3.26 (br., 4H), 3.18- 2.83 (br., 6H), 2.84- 2.68 (br., 3H), 2.19- 1.49 (br., 11H), 1.49- 1.27 (br., 2H), 1.27- 0.96 (br., 2H); [ES+ MS] m/z 595 $(MH)^+$.

Example 21: ***N'*-(5-bromo-2-cyano-4-pyrimidinyl)-*N'*-cyclohexyl-4-[(4-hydroxy-1-piperidinyl)methyl]benzohydrazido trifluoroacetate.**

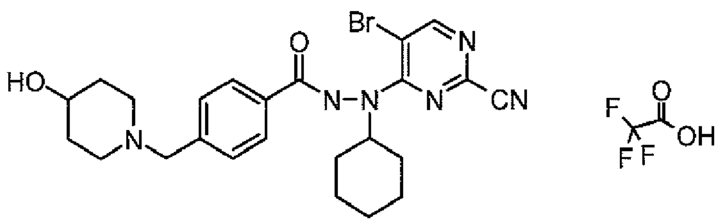

To a solution of Intermediate 24 (0.075 g, 0.17 mmol) in dry ACN (5 mL), DIPEA (FLUKA, 0.058 mL, 0.34 mmol), 4-hydroxypiperidine (ALDRICH, 0.02 g, 0.20 mmol) and a tip of a spatula of sodium iodide were added. The reaction mixture was stirred at room temperature overnight. Once it had reached completion, the mixture was filtered and solvent was evaporated under reduced pressure. The resulting crude product was purified by preparative HPLC (XTERRA 19x150 mm, ACN:$H_2O$, 0.1% TFA, gradient 20-100%) to give the title compound. $^1H$ NMR (300 MHz, DMSO-d6) δ ppm: 11.16 (s, 1H), 9.46 (br., 1H), 8.62 (s, 1H), 8.01 (d, 2H), 7.65 (m, 2H), 4.99 (br., 1H), 4.57 (m, 1H), 4.37 (m, 2H), 4.05-2.89 (m, 5H), 2.08-0.91 (m, 14H). [ES+ MS] m/z 513 $(MH)^+$.

Example 22: ***N'*-(5-bromo-2-cyano-4-pyrimidinyl)-*N'*-cyclohexyl-4-({4-[(4-methyl-1-piperazinyl)carbonyl]-1-piperidinyl}methyl)benzohydrazide trifluoroacetate.**

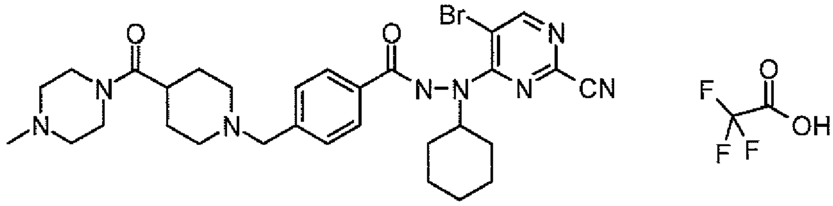

To a solution of Intermediate 24 (0.075 g, 0.17 mmol) in dry ACN (5 mL) DIPEA (FLUKA, 0.087 mL, 0.51 mmol), (4-methyl-piperazin-1-yl)-piperidin-4-yl-methanone (FLUOROCHEM, 0.056 g, 0.20 mmol) and a tip of a spatula of sodium iodide were added. The reaction mixture was stirred at room temperature overnight. Once it had reached completion, the mixture was filtered and solvent was evaporated under reduced pressure. The resulting crude product was purified by preparative HPLC (XTERRA 19x150 mm, ACN:$H_2O$, 0.1%TFA, gradient 20-100%) to give the title compound. $^1H$ NMR (300 MHz, DMSO-d6) δ ppm: 11.18 (s, 1H), 10.08-9.70 (br., 2H), 8.63 (s, 1H), 8.01 (d, 2H), 7.66 (d, 2H), 4.57 (m, 1H), 4.52-2.85 (m, 14H), 2.80 (s, 3H), 1.98-0.95 (m, 15H). [ES+ MS] m/z 623 $(MH)^+$.

Example 23: ***N'*-(5-bromo-2-cyano-4-pyrimidinyl)-*N'*-cyclohexyl-3-({4-[(4-methyl-1-piperazinyl)carbonyl]-1-piperidinyl}methyl)benzohydrazide trifluoroacetate.**

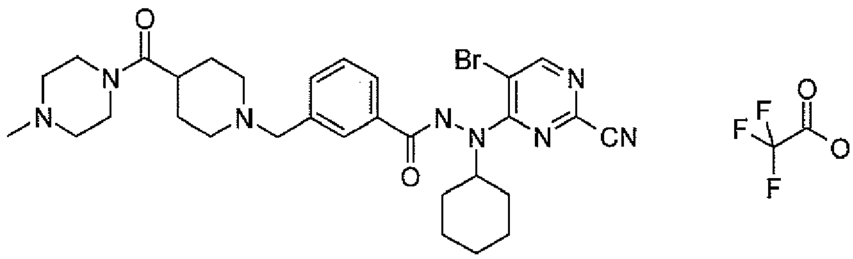

To a solution of Intermediate 25 (0.05 g, 0.1 mmol) in ACN (3 mL), catalytic sodium iodide was added and the mixture was stirred at room temperature for 10 min. Then, (4-methyl-piperazin-1-yl)-piperidin-4-yl-methanone dihydrochloride (FLUOROCHEM, 0.028 g, 0.1 mmol) and DIPEA (0.04 mL, 0.22 mmol) were added and the resulting reaction mixture was stirred at room temperature overnight. The mixture was then filtered and concentrated *in vacuo.* The crude reaction mixture was purified by preparative HPLC (X-TERRA 19x150 mm, ACN:$H_2O$ 0.1% TFA, gradient 20-80%, A= 230 nm) to give the title compound. $^1H$ NMR (300 MHz, $d_6$-DMSO) δ ppm: 11.20 (br.s, 1H), 10.03- 9.82 (br., 1H), 9.79-9.58 (br., 1H), 8.62 (s, 1H), 8.14- 8.06 (m, 1H), 8.05-7.99 (br., 1H), 7.79- 7.70 (m, 1H), 7.70- 7.59 (m, 1H), 4.64- 4.51 (m, 1H), 4.50- 4.02 (br., 4H), 3.53- 2.82 (br., 10H), 2.79 (br. s, 3H), 2.00- 1.50 (br., 10H), 1.48- 0.93 (br., 5H); [ES+ MS] m/z 623 $(MH)^+$.

Example 24: ***N'*-(5-bromo-2-cyano-4-pyrimidinyl)-*N'*-cyclohexyl-3-{[4-(4-morpholinyl)-1-piperidinyl]methyl}benzohydrazide trifluoroacetate.**

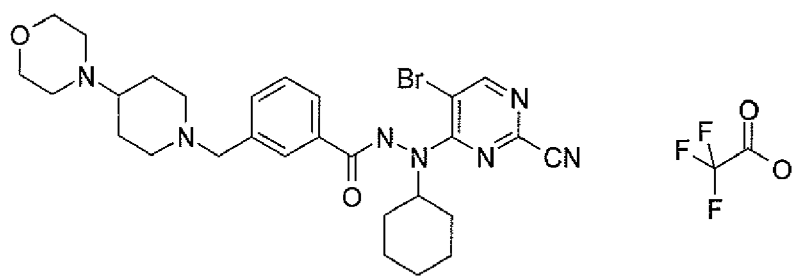

To a solution of Intermediate 25 (0.05 g, 0.1 mmol) in ACN (3 mL), catalytic sodium iodide was added and the mixture was stirred at room temperature for 10 min. Then, 4-morpholinopiperidine (ALDRICH, 0.023 g, 0.13 mmol) and DIPEA (0.04 mL, 0.22 mmol) were added and the resulting reaction mixture was stirred at room temperature overnight. The mixture was then filtered and concentrated *in vacuo.* The crude reaction mixture was purified by preparative HPLC (X-TERRA 19x150 mm, ACN:$H_2O$ 0.1% TFA, gradient 20-80%, A= 230 nm) to give the title compound. $^1H$ NMR (300 MHz, $d_6$-DMSO) δ ppm: 11.19 (br.s, 1H), 10.48- 9.72 (br., 1H), 8.62 (s, 1H), 8.15- 8.03 (m, 1H), 8.03- 7.94 (br., 1H), 7.76- 7.57 (m, 2H), 4.65- 4.51 (m, 1H), 4.49- 4.11 (br., 2H), 4.10-2.74 (br., 12H), 2.36-2.10 (br., 2H), 2.00- 1.52 (br., 8H), 1.50- 0.94 (br., 5H); [ES+ MS] m/z 582 $(MH)^+$.

**Example 25: *N'*-(5-bromo-2-cyano-4-pyrimidinyl)-*N'*-cyclohexyl-4-({4-[(1-methyl-3-piperidinyl)methyl]-1-piperazinyl}methyl)benzohydrazide trifluoroacetate.**

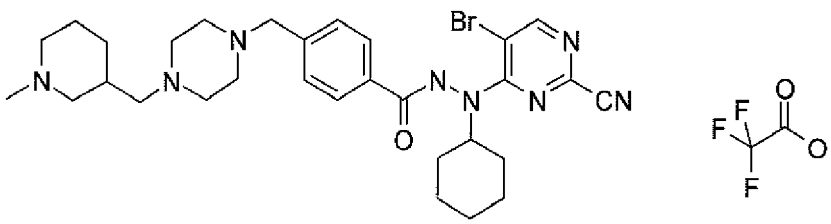

To a suspension of Intermediate 24 (0.075 g, 0.17 mmol) in ACN (5 mL), 1-(N-methyl-3-piperidylmethyl)-piperazine (FLUOROCHEM, 0.04 g, 0.2 mmol), DIPEA (0.06 mL, 0.33 mmol) and catalytic sodium iodide were added and the resulting reaction mixture was stirred at room temperature overnight. Then, the mixture was filtered and concentrated *in vacuo.* The crude reaction mixture was dissolved in methanol and purified by preparative HPLC (X-TERRA 19x150 mm, ACN:$H_2O$ 0.1% TFA, gradient 20-100% first, then re-purified using a 20-80% gradient, $\lambda$=230 nm) to give the title compound. $^1H$ NMR (300 MHz, $d_6$-DMSO) $\delta$ ppm: 11.2- 11.07 (br., 1H), 9.5- 9.3 (br., 1H), 8.62 (s, 1H), 7.97 (d, 2H), 7.74- 7.47 (br., 2H), 4.64- 4.49 (m, 1H), 4.43- 2.57 (br., 19H), 2.09- 1.49 (br., 10H), 1.48- 0.92 (br., 5H); [ES+ MS] m/z 609 $(MH)^+$.

Example 26: ***N'*-(5-bromo-2-cyano-4-pyrimidinyl)-*N'*-(3-methylcyclopentyl)-4-[(4-methyl-1-piperazinyl)methyl] benzohydrazide trifluoroacetate.**

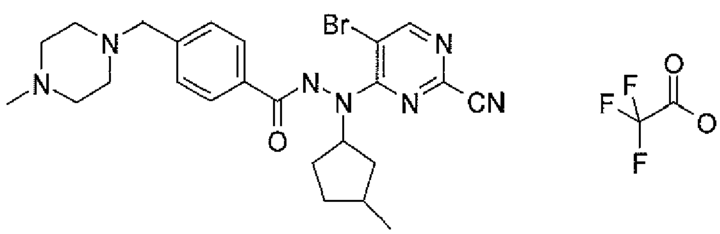

To a solution of Intermediate 17 (0.07 g, 0.15 mmol) in dry ACN (5 mL), N-methylpiperazine (ALDRICH, 0.02 mL, 0.18 mmol), DIPEA (0.05 mL, 0.29 mmol) and catalytic sodium iodide were added and the resulting reaction mixture was stirred at room temperature overnight. Then, the mixture was concentrated *in vacuo* and the crude reaction mixture was purified by preparative HPLC (X-TERRA 19x150 mm, ACN:$H_2O$ 0.1% TFA, gradient 20-100% first, then repurified using a 20-80% gradient) to give the title compound as a mixture of diastereomers, present in a non 1:1 ratio. $^1H$ NMR (300 MHz, $d_6$-DMSO+$CD_3OD$) $\delta$ ppm: 11.21- 11.13 (br., 1H), 9.50- 9.27 (br., 1H), 8.62 (s, 1H), 7.89 (d, 2H), 7.46 (d, 2H), 5.37- 5.28 (br., 1H), 3.67- 3.58 (br., 1H), 3.52-3.46 (br., 2H), 3.43- 2.86 (br., 6H), 2.85- 2.68 (br., 3H), 2.57- 2.39 (br., 1H), 2.36- 1.36 (br., 5H), 1.34- 1.07 (br., 2H), 1.06- 0.73 (br., 3H); [ES+ MS] m/z 512 $(MH)^+$.

Example 27: ***N'*-(5-bromo-2-cyano-4-pyrimidinyl)-*N'*-cyclohexyl-3-({4-[(1-methyl-3-piperidinyl)methyl]-1-piperazinyl}methyl)benzohydrazide trifluoroacetate.**

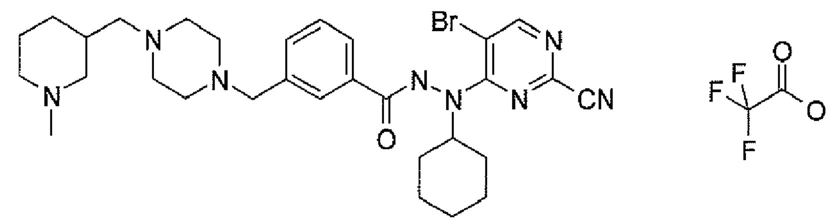

To a solution of Intermediate 25 (0.1 g, 0.22 mmol) in ACN (8 mL), catalytic sodium iodide was added and the mixture was stirred at room temperature for 10 min. Then, a solution of 1-(N-methyl-3-piperidylmethyl)-piperazine (FLUOROCHEM, 0.053 g, 0.27 mmol) in ACN (2 mL) and DIPEA (0.077 mL, 0.44 mmol) were added and the resulting reaction mixture was stirred at room temperature overnight. The mixture was then concentrated *in vacuo* and the crude reaction mixture was purified by preparative HPLC (LUNA 250x50 mm, ACN:$H_2O$ 0.1% TFA, gradient 20-60%) to give the title compound. $^1H$ NMR (300 MHz, $D_2O$) δ ppm: 8.36 (br.s, 1H), 7.84- 7.77 (m, 1H), 7.75-7.79 (br., 1H), 7.62- 7.65 (m, 1H), 7.55- 7.46 (m, 1H), 4.80- 4.45 (br., 1H), 4.17 (br., 2H), 3.41- 3.26 (br., 2H), 3.24- 3.07 (br., 4H), 3.02- 2.80 (br., 4H), 2.78- 2.69 (br., 1H), 2.67(s, 3H), 2.63- 2.48 (br., 3H), 2.10- 1.37 (br., 10H), 1.37- 0.84 (br., 5H); [ES+ MS] m/z 609 $(MH)^+$.

Example 28: ***N'*-(5-bromo-2-cyano-4-pyrimidinyl)-*N'*-cyclohexyl-3-({4-[(1-methyl-4-piperidinyl)methyl]-1-piperazinyl}methyl)benzohydrazide *trifluoroacetate.***

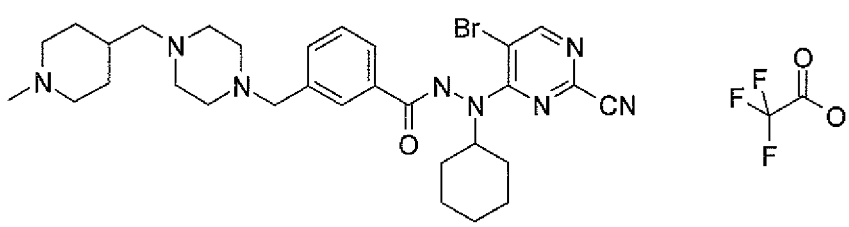

To a solution of Intermediate 25 (0.1 g, 0.22 mmol) in ACN (8 mL), catalytic sodium iodide was added and the mixture was stirred at room temperature for 10 min. Then, a solution of 1-(N-methyl-4-piperidinmethyl)piperazine (FLUOROCHEM, 0.053 g, 0.27 mmol) in ACN (2 mL) and DIPEA (0.077 mL, 0.44 mmol) were added and the resulting reaction mixture was stirred at room temperature overnight. The mixture was then concentrated *in vacuo* and the crude reaction mixture was purified by preparative HPLC (LUNA 250x50 mm, ACN:$H_2O$ 0.1% TFA, gradient 20-60%) to give the title compound. $^1H$ NMR (300 MHz, $D_2O$) δ ppm: 8.38- 8.35 (br., 1H), 7.88- 7.76 (m, 1H), 7.73- 7.69 (br., 1H), 7.60- 7.54 (m, 1H), 7.53- 7.44 (m, 1H), 4.71- 4.52 (br., 1H), 4.09 (br., 2H), 3.41- 3.32 (m, 2H), 3.24- 3.02 (br., 8H), 2.90- 2.74 (br., 4H), 2.66 (s, 3H), 2.06- 1.54 (br., 7H), 1.54- 0.84 (br., 8H); [ES+ MS] m/z 609 $(MH)^+$.

Example 29: ***N'*-(5-Bromo-2-cyano-4-pyrimidinyl)-4-{[4-(4-methyl-1-piperazinyl)-1-piperidinyl]methyl}-*N'*-(tetrahydro-2H-pyran-4-yl)benzohydrazide trifluoroacetate.**

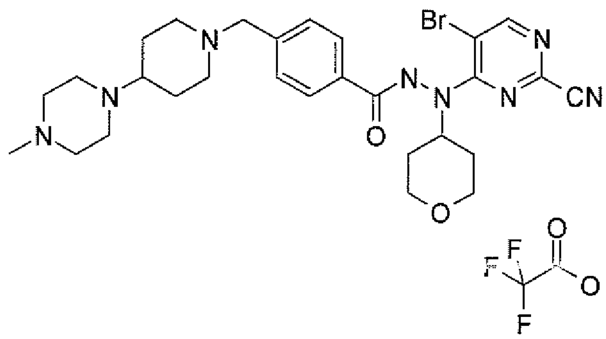

To a solution of Intermediate 30 (0.085 g, 0.17 mmol) in ACN (3 mL), DIPEA (FLUKA, 0.04 mL, 0.26 mmol) and 1-methyl-4-(piperidin-4-yl)-piperazine (FLUOROCHEM, 0.037 g, 0.2 mmol) were added and the resulting reaction mixture was stirred at room temperature overnight. Solvent was removed under reduced pressure and the resulting crude product was purified by preparative HPLC (SUNFIRE 30x150 mm, ACN:$H_2O$, 0.1%TFA, gradient 10-100%) to give the title compound. $^1H$ NMR (300 MHz, $d_6$-DMSO) δ ppm: 11.25 (s, 1H), 9.88- 9.60 (br., 1H), 8.67 (s, 1H), 8.02 (d, 2H), 7.64 (d, 2H), 4.91-4.77 (m, 1H), 4.47- 4.31 (br., 2H), 4.21- 3.57 (br., 4H), 3.55- 3.28 (br., 6H), 3.20- 2.84 (br., 6H), 2.76 (s, 3H), 2.63- 2.50 (br., 1H), 2.44- 2.24 (br., 2H), 2.07- 1.30 (br., 6H); [ES+ MS] m/z 597 $(MH)^+$.

Example 30: ***N'*-(5-Bromo-2-cyano-4-pyrimidinyl)-4-({4-[(1-methyl-4-piperidinyl)methyl]-1-piperazinyl}methyl)-*N'*-(tetrahydro-2H-pyran-4-yl)benzohydrazide trifluoroacetate.**

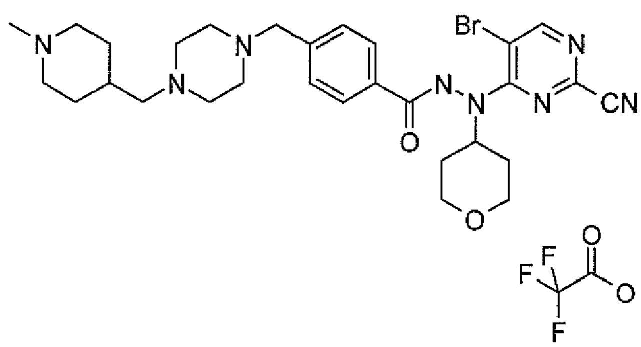

To a solution of Intermediate 30 (0.08 g, 0.16 mmol) in ACN (3 mL), DIPEA (FLUKA, 0.041 mL, 0.264mmol) and 1-(N-methyl-4-piperidinmethyl)piperazine (FLUOROCHEM, 0.038 g, 0.19 mmol) were added and the resulting reaction mixture was stirred at room temperature overnight. Solvent was removed under reduced pressure and the resulting crude product was purified by preparative HPLC (SUNFIRE 30x150 mm, ACN:$H_2O$, 0.1%TFA, gradient 10-100%) to give the title compound. $^1H$ NMR (300 MHz, $d_6$-DMSO) δ ppm: 11.18 (s, 1H), 9.45- 9.25 (br., 1H), 8.65 (s, 1H), 7.96 (d, 2H), 7.66- 7.42 (br., 2H), 4.91- 4.77 (m, 1H), 4.02- 3.84 (m, 4H), 3.54- 3.34 (br., 4H), 3.10- 2.61 (br., 15H), 2.01-1.16(br., 9H); [ES+ MS] m/z 611 $(MH)^+$.

**Example 31: *N'*-(5-Bromo-2-cyano-4-pyrimidinyl)-4-({4-[(1-methyl-3-piperidinyl)methyl]-1-piperazinyl}methyl)-*N'*-(tetrahydro-2H-pyran-4-yl)benzohydrazide trifluoroacetate.**

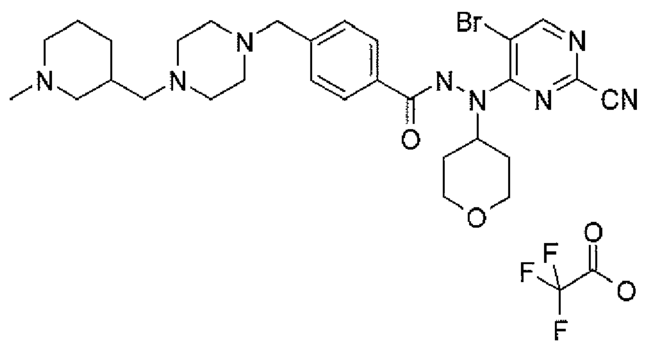

To a solution of Intermediate 30 (0.087 g, 0.18 mmol) in ACN (3 mL), DIPEA (FLUKA, 0.046 mL, 0.27 mmol) and 1-(N-methyl-3-piperidylmethyl)-piperazine (FLUOROCHEM, 0.043 g, 0.22 mmol) were added and the resulting reaction mixture was stirred at room temperature overnight. Solvent was removed under reduced pressure and the resulting crude product was purified by preparative HPLC (SUNFIRE 30x150 mm, ACN:$H_2O$, 0.1%TFA, gradient 10-100%) to give the title compound. $^1H$ NMR (300 MHz, $CD_3OD$) δ ppm: 8.56 (s, 1H), 8.01 (d, 2H), 7.67 (d, 2H), 5.06-4.91 (m, 1H), 4.33 (s, 2H), 4.11-3.92 (br., 2H), 3.69- 3.43 (br., 4H), 3.29- 3.14 (br., 4H), 2.95- 2.78 (br., 5H), 2.69- 2.44 (br., 4H), 2.18- 0.77 (br., 11H); [ES+ MS] m/z 611 $(MH)^+$.

Example 32: ***N'*-(5-Bromo-2-cyano-4-pyrimidinyl)-4-({4-[(4-methyl-1-piperazinyl)carbonyl]-1-piperidinyl}methyl)-*N'*-(tetrahydro-2H-pyran-4-yl)benzohydrazide trifluoroacetate.**

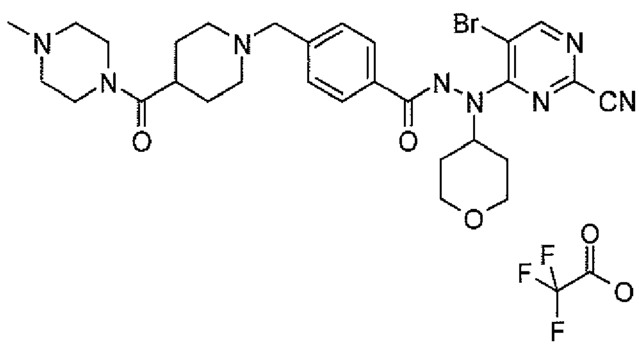

To a solution of Intermediate 30 (0.091 g, 0.18 mmol) in ACN (2 mL), DIPEA (FLUKA, 0.046 mL, 0.27 mmol) and (4-methyl-piperazin-1-yl)-piperidin-4-yl-methanone dihydrochloride (FLUOROCHEM, 0.047 g, 0.22 mmol) were added and the resulting reaction mixture was stirred at room temperature overnight. Then, more DIPEA (0.046 mL, 0.27 mmol) was added in order to drive the reaction to completion. The reaction mixture was stirred at room temperature over the weekend. Solvent was then removed under reduced pressure and the resulting crude product was purified by preparative HPLC (SUNFIRE 30x150 mm, ACN:$H_2O$, 0.1%TFA, gradient 10-100%) to give the title compound. $^1H$ NMR (300 MHz, DMSO) δ ppm: 11.28 (s, 1H), 10.11- 9.71 (br., 2H), 8.67 (s, 1H), 8.03 (d, 2H), 7.66 (d, 2H), 4.94- 4.76 (m, 1H), 4.55- 4.26 (br., 3H), 4.24-3.21 (br., 11H), 3.07- 2.68 (br., 8H), 2.01-1.68 (br., 7H), 1.52-1.32 (br., 1H); [ES+ MS] m/z 625 $(MH)^+$.

Example 33: ***N'*-(5-bromo-2-cyano-4-pyrimidinyl)-4-{[3-(1-pyrrolidinyl)-1-azetidinyl]methyl}-*N'*-(tetrahydro-2H-pyran-4-yl)benzohydrazide trifluoroacetate.**

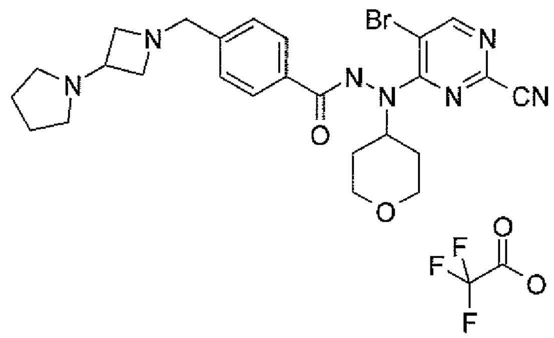

The title compound was synthesised following an analogous procedure to the one for example 32, with Intermediate 42 being the amine used. $^{1}H$ NMR (300 MHz, DMSO-$d_6$) δ ppm: 11.21 (br.s, 1H), 8.66 (s, 1H), 7.97 (d, 2H), 7.55 (d, 2H), 4.91- 4.76 (m, 1H), 4.49- 2.77 (br., 15H), 2.05- 1.71 (br., 7H), 1.51-1.31 (m, 1H); [ES+ MS] m/z 540 $(MH)^+$.

Example 34: ***N'*-(5-Chloro-2-cyano-4-pyrimidinyl)-4-{[4-(4-methyl-1-piperazinyl)-1-piperidinyl]methyl}-N'-(tetrahydro-2H-pyran-4-yl)benzohydrazide trifluoroacetate.**

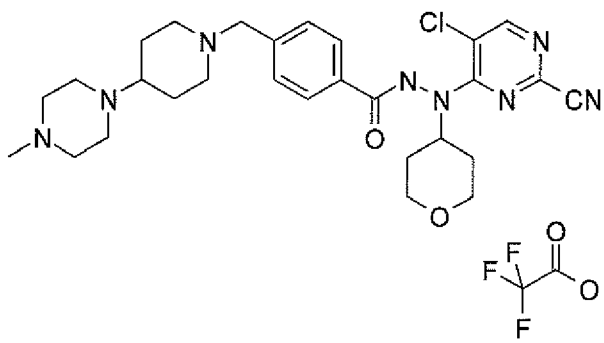

To a solution of Intermediate 34 (0.085 g, 0.2 mmol) in ACN (2 mL), DIPEA (FLUKA, 0.05 mL, 0.3 mmol) and 1-methyl-4-(piperidin-4-yl)-piperazine (FLUOROCHEM, 0.044 g, 0.24 mmol) were added and the resulting reaction mixture was stirred at room temperature overnight. Solvent was removed under reduced pressure and the resulting crude product was purified by preparative HPLC (SUNFIRE 30x150 mm, ACN:$H_2O$, 0.1 %TFA, gradient 10-70%) to give the title compound. $^{1}H$ NMR (300 MHz, $d_6$-DMSO) δ ppm: 11.27 (s, 1H), 9.96- 9.62 (br., 1H), 8.55 (s, 1H), 7.99 (d, 2H), 7.65 (d, 2H), 4.93-4.78 (m, 1H), 4.48- 4.30 (br., 2H), 4.29- 3.66 (br., 4H), 3.57- 3.26 (br., 6H), 3.22- 2.84 (br., 6H), 2.76 (s, 3H), 2.67- 2.51 (br., 1H), 2.47- 2.18 (br., 2H), 2.09- 1.34 (br., 6H); [ES+ MS] m/z 553 $(MH)^+$.

Example 35: ***N'*-(5-Chloro-2-cyano-4-pyrimidinyl)-4-({4-[(1-methyl-4-piperidinyl)methyl]-1-piperazinyl}methyl)-*N'*-(tetrahydro-2H-pyran-4-yl)benzohydrazide trifluoroacetate.**

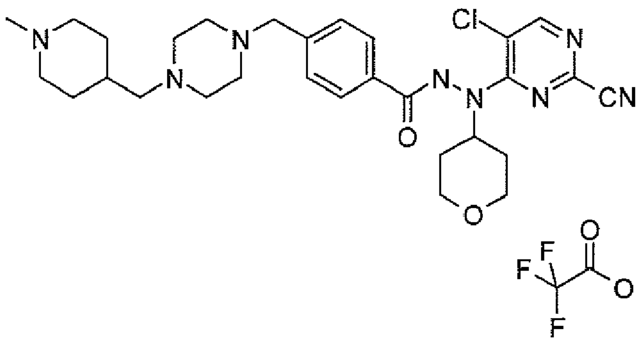

To a solution of Intermediate 34 (0.08 g, 0.19 mmol) in ACN (2 mL), DIPEA (FLUKA, 0.048 mL, 0.33 mmol) and 1-(N-methyl-4-piperidinmethyl)piperazine (FLUOROCHEM, 0.044 g, 0.22 mmol) were added and the resulting reaction mixture was stirred at room temperature overnight. Solvent was removed under reduced pressure and the resulting crude product was purified by preparative HPLC (SUNFIRE 30x150 mm, ACN:$H_2O$, 0.1%TFA, gradient 10-70%) to give the title compound. $^1H$ NMR (300 MHz, $d_6$-DMSO) δ ppm: 11.20 (s, 1H), 9.66- 9.22 (br., 1 H), 8.54 (s, 1 H), 7.93 (d, 2H), 7.60- 7.46 (br., 2H), 4.90- 4.79 (m, 1H), 4.19- 3.58 (m, 4H), 3.55- 3.33 (br., 6H), 3.27- 2.66 (br., 13H), 1.99- 1.18 (br., 9H); [ES+ MS] m/z 567 $(MH)^+$.

Example 36: ***N'*-(5-Chloro-2-cyano-4-pyrimidinyl)-4-({4-[(4-methyl-1-piperazinyl)carbonyl]-1-piperidinyl}methyl)-*N'*-(tetrahydro-2H-pyran-4-yl)benzohydrazide trifluoroacetate.**

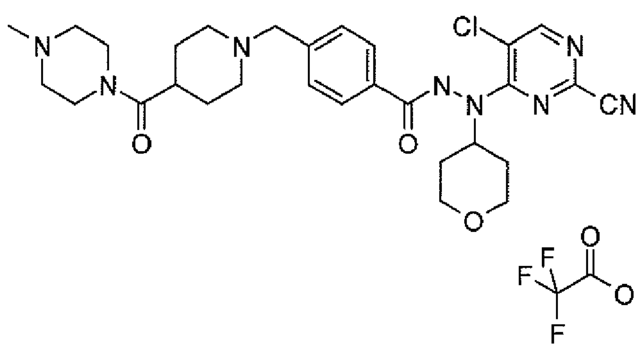

To a solution of Intermediate 34 (0.077 g, 0.17 mmol) in ACN (2 mL), DIPEA (FLUKA, 0.043 mL, 0.26 mmol) and (4-methyl-piperazin-1-yl)-piperidin-4-yl-methanone dihydrochloride (FLUOROCHEM, 0.043 g, 0.21 mmol) were added and the resulting reaction mixture was stirred at room temperature overnight. Then, more DIPEA (1 eq.) was added in order to drive the reaction to completion. The reaction mixture was stirred at room temperature for further 6 h and then kept at 4 °C over the weekend. Solvent was then removed under reduced pressure and the resulting crude product was purified by preparative HPLC (SUNFIRE 30x150 mm, ACN:$H_2O$, 0.1%TFA, gradient 10-100%) to give the title compound. $^1H$ NMR (300 MHz, DMSO) δ ppm: 11.29 (s, 1H). 10.15- 9.76 (br., 2H), 8.56 (s, 1H), 8.00 (d, 2H), 7.67 (d, 2H), 4.91- 4.80 (m, 1H), 4.55-4.27 (br., 3H), 4.25- 3.18 (br., 11H), 3.09- 2.70 (br., 8H), 2.01- 1.66 (br., 7H), 1.54- 1.34 (br., 1H); [ES+ MS] m/z 581 $(MH)^+$.

Example 37: ***N'*-(1-acetyl-4-piperidinyl)-*N'*-(5-bromo-2-cyano-4-pyrimidinyl)-4-[(4-methyl-1-piperazinyl)methyl] benzohydrazide trifluoroacetate.**

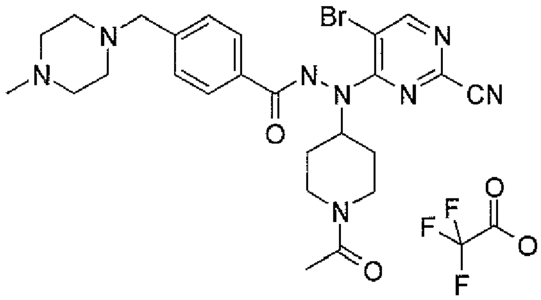

To a solution of Intermediate 39 (1 mmol) in THF (25 mL), DIPEA (FLUKA, 0.026 mL, 1.5 mmol) and N-methylpiperazine (ALDRICH, 0.134 g, 1.21 mmol) were added and the resulting reaction mixture was stirred at room temperature for 20.5 h. Solvent was removed under reduced pressure and the resulting crude product was purified by preparative HPLC (SUNFIRE 30x150 mm, ACN:$H_2O$, 0.1%TFA, gradient 20-60%) to give the title compound. $^1H$ NMR (300 MHz, $d_6$-DMSO) δ ppm: 11.11 (s, 1H), 9.89- 9.16 (br., 1H), 8.65 (s, 1H), 7.91 (d, 2H), 7.47 (d, 2H), 4.87- 4.79 (m, 1H), 4.52- 4.38 (m, 1H), 4.19- 2.85 (br., 8H), 2.78 (s, 3H), 2.73- 2.51 (br., 3H), 2.45- 2.24 (br., 2H), 2.04-1.07 (br., 7H); [ES+ MS] m/z 555 $(MH)^+$.

Example 38: ***N*'-(1-acetyl-4-piperidinyl)-*N*'-(5-bromo-2-cyano-4-pyrimidinyl)-4-{[4-(4-methyl-1-piperazinyl)-1-piperidinyl]methyl}benzohydrazide trifluoroacetate.**

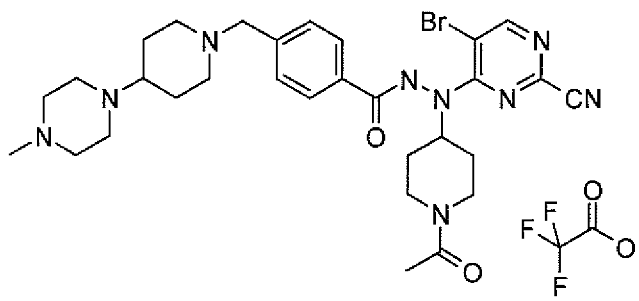

The title compound was synthesised using Intermediate 39 and 1-methyl-4-(piperidin-4-yl)-piperazine (FLUOROCHEM) and following an analogous procedure to the one for Example 37, to give the title compound in 80% purity. $^1H$ NMR (600 MHz, DMSO-$d_6$) δ ppm: 11.23 (s, 1H), 9.95- 9.70 (br., 1H), 9.68- 9.44 (br., 1H), 8.68 (s, 1H), 8.01 (d, 2H), 7.68- 7.63 (m, 2H), 4.88- 4.83 (m, 1H), 4.54- 4.40 (m, 1H), 4.37 (s, 2H), 3.93- 3.86 (m, 2H), 3.70- 2.87 (br., 9H), 2.81-2.77 (br., 3H), 2.71- 2.52 (br., 2H), 2.45- 2.28 (br., 2H), 2.09-1.04 (br., 12H).

Comparative Example 39: ***N*'-(5-bromo-2-cyano-4-pyrimidinyl)-*N*'-(2,2-dimethylpropyl)-4-[(4-methyl-1-piperazinyl)methyl]benzohydrazide trifluoroacetate.**

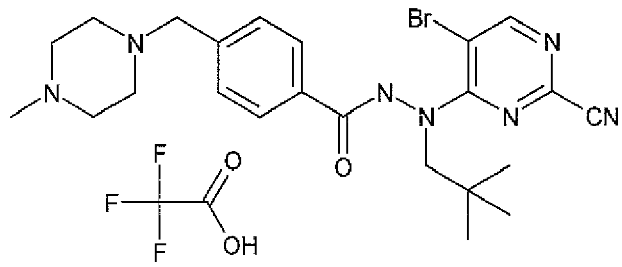

Intermediate 49 (1 g, 4.3 mmol) was dissolved in thionyl chloride (5 ml). The reaction mixture was stirred at room temperature for 17 hours. The solvent was evaporated in *vacuo* and the acid chloride was used without any further purification.

To a stirred solution of Intermediate 47 (200 mg, 0.70 mmol) in pyridine (1 mL) and DIPEA (5 mL), potassium carbonate (193 mg, 1.40 mmol) and previously obtained acid chloride (443 mg, 1.75 mmol) were added and the resulting reaction mixture was stirred at room temperature for 17 hours. The solvent was evaporated *in vacuo* and the crude reaction mixture was purified by flash chromatography (silica gel, dichloromethane:methanol). The solid was repurified by HPLC ($H_2O$:ACN) to give the title compound. $^1H$ NMR (300 MHz, DMSO) δ ppm: 11.33 (s, 1H), 8.64 (s, 1H), 7.91 (d, 2H), 7.49 (d, 2H), 3.72 (s, 2H), 3.37 (m, 2H), 3.25-2.81 (br, 6H), 2.78 (s, 3H) 0.99 (s, 9H). [ES+ MS] m/z 500 ($MH^+$).

**Biological Assays**

The compounds of this invention may be tested in one of several biological assays to determine the concentration of compound which is required to have a given pharmacological effect.

1) Determination of Falcipain-2, Falcipain-3, Vivapain-2, Cathepsin K Cathepsin S, Cathepsin L, and Cathepsin B proteolytic catalytic activity

Assays for Falcipain-2, Falcipain-3, and Vivapain-2 are carried out with parasitic recombinant enzymes. Cathepsins K, S, L, and B are carried out with human recombinant enzymes. Standard assay conditions for the determination of kinetic constants used a fluorogenic peptide substrate, typically H-D-VLR-AFC (Falcipain-2, Falcipain-3, Vivapain-2), Z-FR-AFC (Cathepsin K, L, B), or KQKLR-AMC (Cathepsin S) and are determined in 100 mM sodium acetate, pH 5.5, containing 10 mM DTT and 0.5 mM CHAPS (Falcipain-2, Falcipain-3, Vivapain-2), and 100 mM sodium acetate, pH 5.5, containing 5 mM L-cysteine, 1mM CHAPS and 5mM EDTA (Cathepsin K, L, B), or 50mM MES, pH 6.5, containing 0.5mM CHAPS, 10mM L-CYS, 5mM EDTA (Cathepsin S). Stock substrate solutions are prepared at 20 mM in DMSO. The activity assays contained 30 uM substrate (Falcipain-2, Falcipain-3, Vivapain-2), 20 uM substrate (Cathepsin K), 25uM substrate (Cathepsin B), 5uM substrate (Cathepsin L), and 30uM substrate (Cathepsin S). All assays contained 1% DMSO. Independent experiments found that this level of DMSO had no effect on enzyme activity or kinetic constants. All assays are conducted at ambient temperature as end point assays being quenched after 60 minutes with the exception of Cathepsin S at 90 minutes, with 16.6 uM E-64 in 1% DMSO. Product formation (AFC or AMC) is determined from fluorescence (excitation at 405nM; emission at 530nM, AFC, or excitation at 360 nM; emission at 460 nM, AMC) monitored with a LJL Aquest (Molecular Devices) fluorescent plate reader. In the case of kinetic reads (used in mechanism of action studies), the reaction is not quenched but is read in the plate reader every 3 minutes for approximately 90 minutes. In addition, the mechanism of action studies for Falcipain-2 utilize Z-LR-AMC as the substrate. Product formation is determined from the fluorescence of AMC, measured with a LJL Acquest (Molecular Devices) fluorescent plate reader (excitation at 360nM; emission at 460nM).

Inhibition studies

Potential inhibitors are evaluated using the quenched read (endpoint) method. Assays are carried out in the presence of variable concentrations of test compound. Reactions are initiated by addition of enzyme and substrate to wells containing inhibitor stamped in 100% DMSO. For endpoint assays, the reaction is quenched with the addition of E64. Dose response data is fit to an $IC_{50}$ curve with preset fitting tools according to equation 1:

$$y = a + (b-a)/(1+(10^x/10^c)^d) \qquad (1)$$

where y is the response at a particular inhibitor concentration x, a is the minimum response value, b is the maximum response value, c is the $IC_{50}$, and d is the slope of the $IC_{50}$ curve. Assuming the compound is a competitive inhibitor, the apparent $K_I$ can be calculated from $IC_{50}$, as shown in equation 2:

$$IC_{50} = appK_I\ (1+[S]/K_M) \qquad (2)$$

where $appK_I$ is the apparent $K_I$, S is the concentration of substrate, $K_M$ is the Michaelis binding constant for substrate, and $K_I$ is the binding constant of a competitive inhibitor for free enzyme. For a more direct measurement of the $K_I$ and the binding mechanism, we performed mechanism of action studies that included a titration of substrate and inhibitor with a kinetic read. If the progress curves for each of these kinetic assays are linear, the measured rates (v) were fit to equation 3:

$$v = V_m S / [(K_M\ (1 + [I]/K_I) + [S]\ (1 + [I]/\alpha K_I)] \qquad (3)$$

where $V_m$ is the maximum velocity, $S$ is the concentration of substrate with Michaelis constant of $K_M$, $[I]$ is the concentration of inhibitor, $K_I$ is the binding constant of inhibitor for free enzyme, and $\alpha K_I$ is the binding constant of inhibitor for a potential enzyme-substrate complex.

For those compounds whose progress curves were nonlinear, with a decrease in enzyme activity over time characteristic of time-dependent inhibition, the progress curves were fit to equation 4 to yield the $k_{obs}$:

$$[AMC] = v_s\, t + (v_0 - v_{ss})\ [1 - exp\ (-k_{obs} t)] / k_{obs} \qquad (4)$$

where [AMC] is the concentration of product formed over time $t$, $v_0$ is the initial reaction velocity and $v_S$ is the final steady state rate. The $k_{obs}$ values were fit to equations 5 and 6, describing a one-step and two-step time dependent binding mechansim respectively:

$$k_{obs} = k_{off}\,(1 + [I] / appK_I) \qquad (5)$$

$$k_{obs} = k_{off} + k_{on}\,([I] / (appK_I + [I])) \qquad (6)$$

$$appK_I = K_I\ (1+[S]/K_M) \qquad (7)$$

Equation 7 describes the apparent $K_I$ for competitive compounds and was substituted into equations 5 and 6 to generate the relevant binding constants from the fitting routine. In addition, the initial and final velocities were fit to equation 3 to further define the binding mechanism and potency. A complete discussion of this kinetic treatment has been fully described (Morrison et al., Adv. Enzymol. Relat. Areas Mol. Biol., 1988, 61, 201).

2) Determination of whole cell activity against the *Plasmodium falciparum* parasite

Compounds can be evaluated for whole cell actvity against the *Plasmodium falciparum* parasite according to the procedure described in Sijwali S. and Rosenthal P. J., (2004) Proceedings of the National Academy of Sciences of the United States of America (PNAS) 101 (13), 4384-4389 (see in particular "Measurement of Parasite Growth rates and Inhibitor Sensitivity" on page 4385); $IC_{50}$ values can be calculated as described in Singh A. and Rosenthal P. J., (2001) Antimicrobial Agents and Chemotherapy 45(3), 949-951 (see in particular page 950, first column); synchronised parasites can be prepared as described in Divo A. A. et al., (1985) Protozool. 32, 59-64.

3) *In vitro* models to evaluate activity against bone metastasis

Compounds can be evaluated for their actvity against bone metastasis using published *in vitro* models as follows: prostate cancer bone metastases model in rat (Liepe K. et al., (2005) Anticancer Research 25(2A), 1067-1073 and Neudert M. et al., (2003) International Journal of Cancer 107(3), 468-477); models of prostate and breast cancer metastases to bone in mice (Angelucci A. et al., (2004) International Journal of Oncology 25(6), 1713-1720 and Sasaki A. et al., (1995) Cancer Research 55(16), 3551-3557); and other models in various species for evaluating bone metastasis (Rosol T. J. et al, (2003) Cancer. 97, 748-757).

3) Comparator compound

One compound was employed as a comparator compound. Comparative Example 39, which is a trifluoroacetate salt, was prepared as described hereinabove. The free base of this compound is disclosed in WO 2005/103012 A1 (page 124, Example 15(2)).

Comparative Example 39: ***N'*-(5-bromo-2-cyano-4-pyrimidinyl)-*N'*-(2,2-dimethylpropyl)-4-[(4-methyl-1-piperazinyl)methyl]benzohydrazide trifluoroacetate.**

It will be understood by the skilled artisan that under the ezymatic assay conditions described hereinabove, the assay result obtained for the free base of a given compound is expected to be the same as that obtained when a salt of that compound is tested. This is because the buffer used in the assay determines the pH under which the compound is tested; the pH determines the relative amounts of free base to salt of the compound being tested. This has been confirmed by testing in the enzymatic assays the free base, the hydrochloride salt and the trifluoroacetate salt of certain compounds of the type exemplified herein.

Results of assays

Cathepsin K

Examples 1-12, 14-25, 27, and 29-36 were tested in the enzymatic assay for cathepsin K according to the procedure described hereinabove.

Examples 1, 3-12, 14-25, 27, and 29-30 were found to have an $IC_{50}$ value of less than 1.5 nM in the enzymatic assay for cathepsin K. All Examples tested were found to have $IC_{50}$ value of less than 13 nM in the enzymatic assay for cathepsin K.

Falcipain-2 and falcipain-3 enzymatic assays, and whole cell assay

All exemplified compounds (Examples 1-38 and comparative Example 39) were tested in the enzymatic assays for falcipain-2 and for falcipain-3 according to the procedure described hereinabove.

All exemplified compounds (Examples 1-38 and comparative Example 39) were tested in the whole cell assay according to the procedure described hereinabove.

The results of falcipain-2 and falcipain-3 enzymatic assays, and the whole cell assay for the exemplified compounds of the present invention (Examples 1-38) and for comparative Example 39 are shown in the table below.

**Table of falcipain-2, falcipain-3 and whole cell assay activities**

| Structure | Example No. | Falcipain-2 | Falcipain-3 | Whole cell |
|---|---|---|---|---|
| 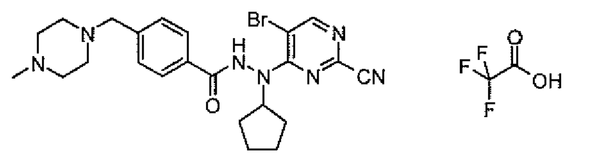 | 1 | A | D | D |
| 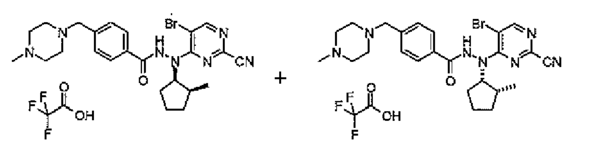 | 2 | B | D | D |
| 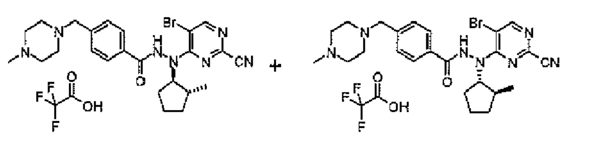 | 3 | A | C | C |
| 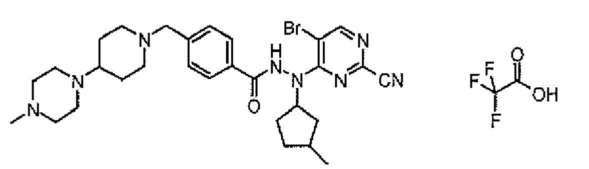 | 4 | A | B | A |
| 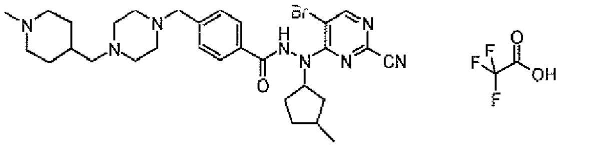 | 5 | A | D | C |
| 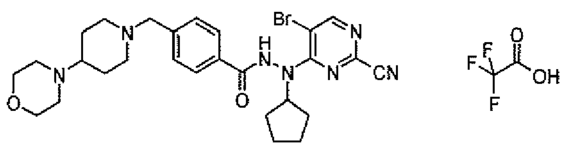 | 6 | A | C | D |
| 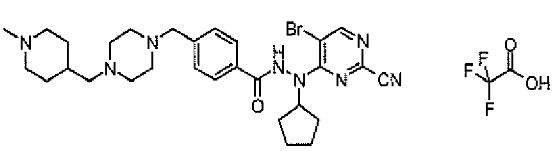 | 7 | A | C | B |
| 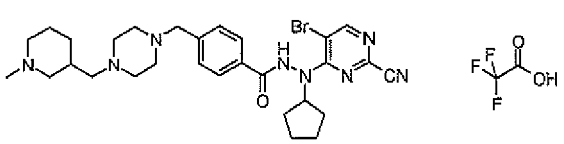 | 8 | A | C | C |
| 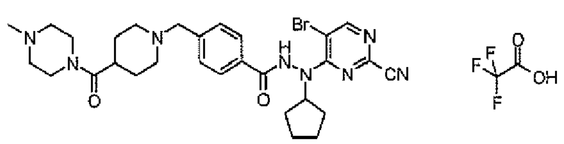 | 9 | A | C | C |
| 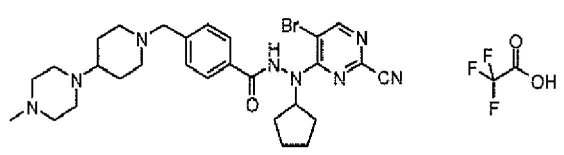 | 10 | A | C | C |

(continued)

| Structure | Example No. | Falcipain-2 | Falcipain-3 | Whole cell |
|---|---|---|---|---|
| 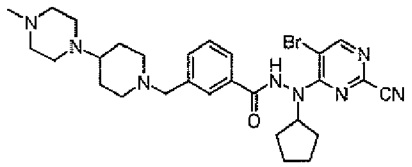 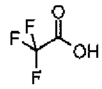 | 11 | A | C | C |
| 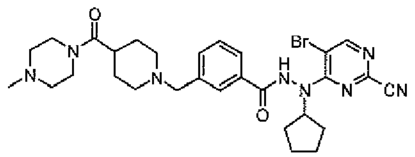 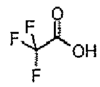 | 12 | A | C | C |
| 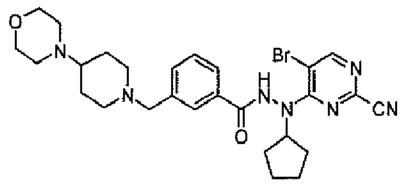 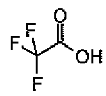 | 13 | A | C | D |
| 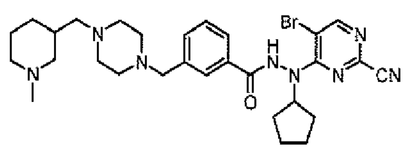 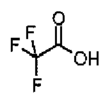 | 14 | A | C | C |
| 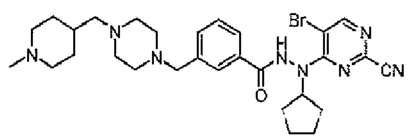 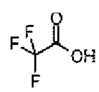 | 15 | A | D | C |
| 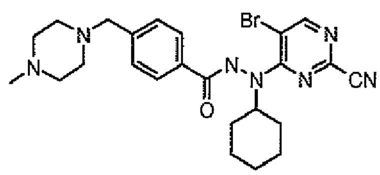 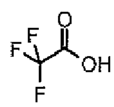 | 16 | A | C | C |
| 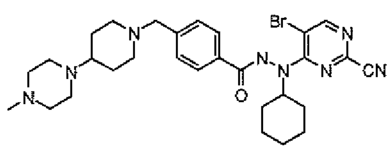 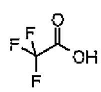 | 17 | A | C | C |
| 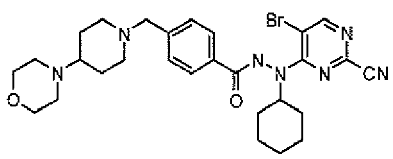 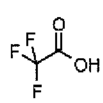 | 18 | A | C | D |
| 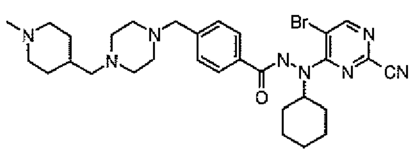 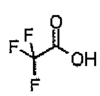 | 19 | A | C | C |

(continued)

| Structure | | Example No. | Falcipain-2 | Falcipain-3 | Whole cell |
|---|---|---|---|---|---|
| 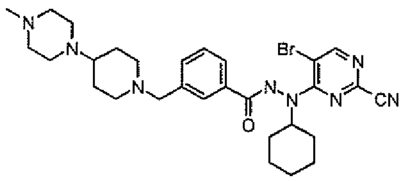 | 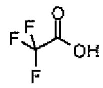 | 20 | A | D | C |
| 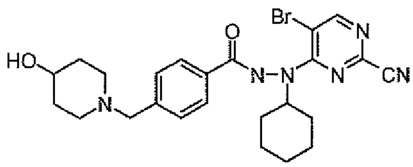 | 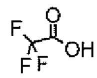 | 21 | A | C | D |
| 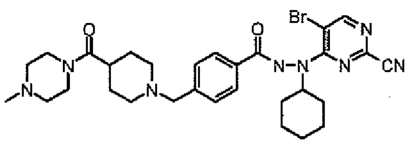 | 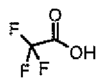 | 22 | A | C | C |
| 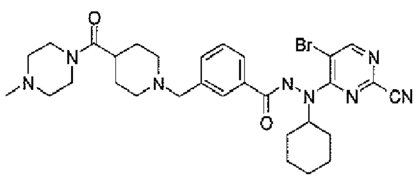 | 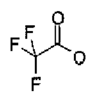 | 23 | A | C | C |
| 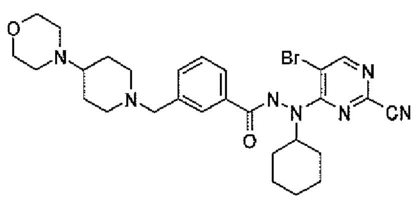 | 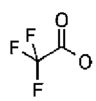 | 24 | A | C | C |
| 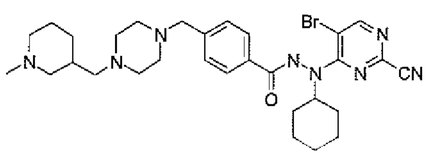 | 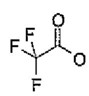 | 25 | A | C | C |
| 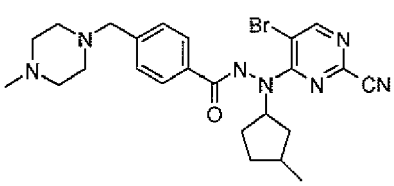 | 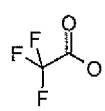 | 26 | A | D | D |
| 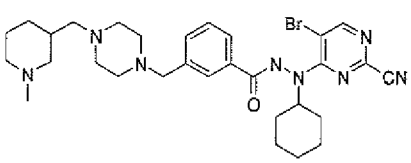 | 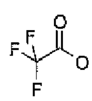 | 27 | A | C | C |

(continued)

| Structure | Example No. | Falcipain-2 | Falcipain-3 | Whole cell |
|---|---|---|---|---|
| 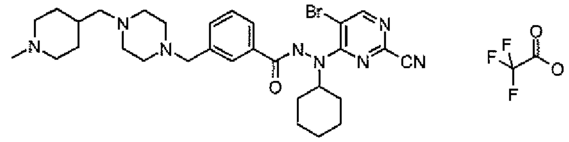 | 28 | A | C | C |
| 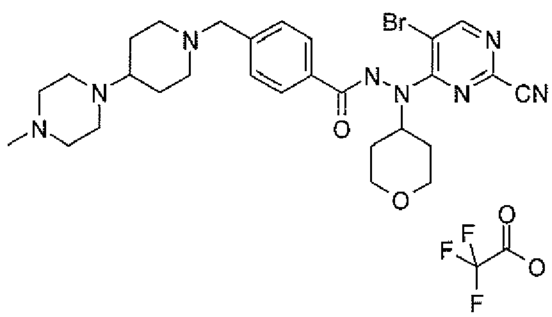 | 29 | A | D | D |
| 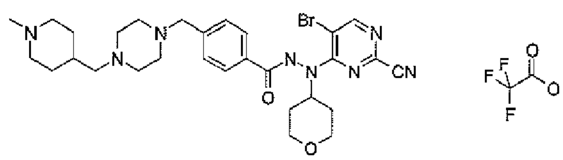 | 30 | B | C | D |
| 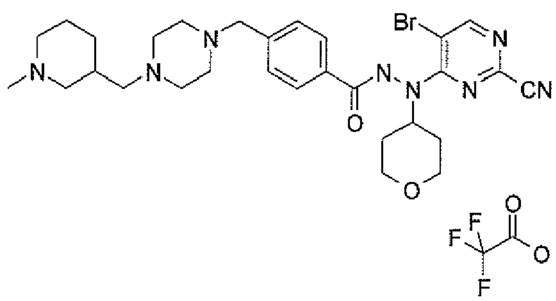 | 31 | A | D | D |
| 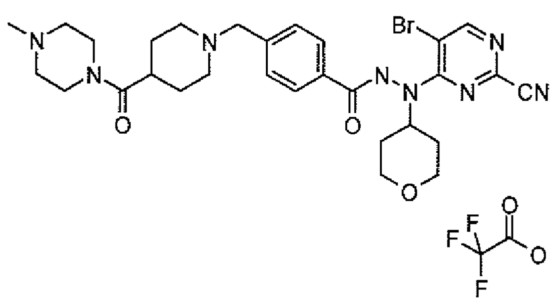 | 32 | A | C | D |
| 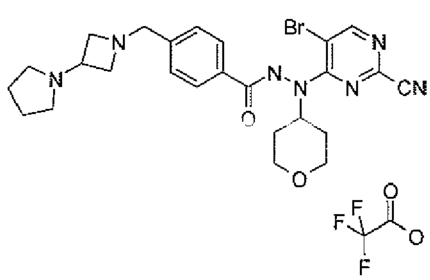 | 33 | A | D | D |

(continued)

| Structure | Example No. | Falcipain-2 | Falcipain-3 | Whole cell |
|---|---|---|---|---|
| 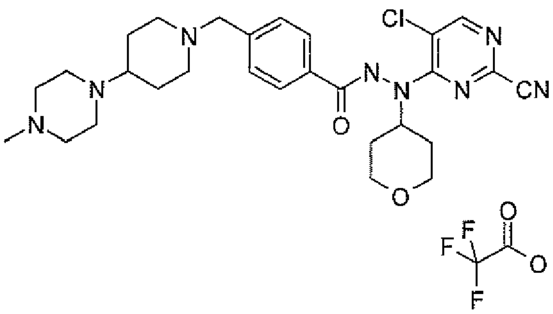 | 34 | B | C | C |
| 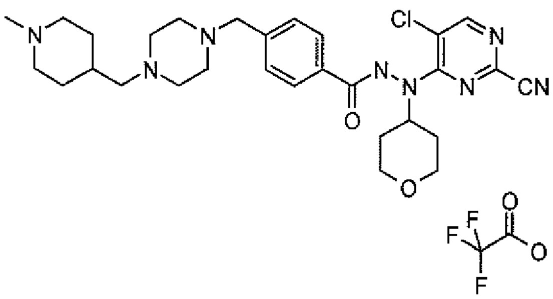 | 35 | B | D | D |
| 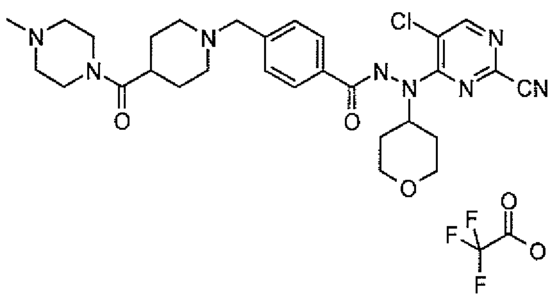 | 36 | B | D | D |
| 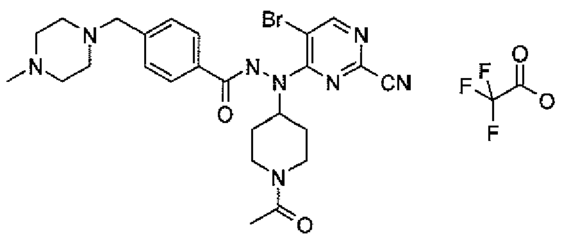 | 37 | A | D | D |
| 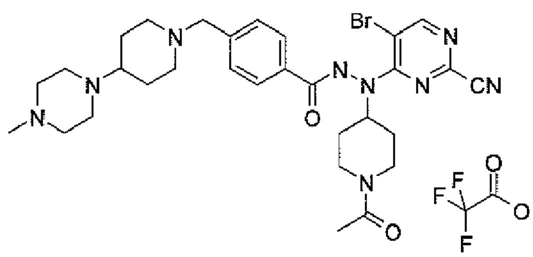 | 38 | A | D | E |

(continued)

| Structure | Example No. | Falcipain-2 | Falcipain-3 | Whole cell |
|---|---|---|---|---|
| 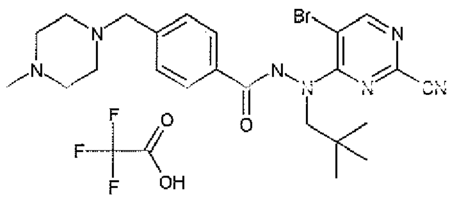 | comparative 39 | D | E | G |

**Key to Table**

X = $IC_{50}$ in nM
X<1 A
1 <X<2.5 B
2.5<X<15 C
15<X<150 D
150<X<250 E
250<X<400 F
X≥400 G

The exemplified compounds of the invention exhibit an improved activity in each of the falcipain-2, falcipain-3 and whole cell assays, as compared with comparative Example 39 of the prior art.

## Claims

**1.** At least one chemical entity selected from a compound of Formula I:

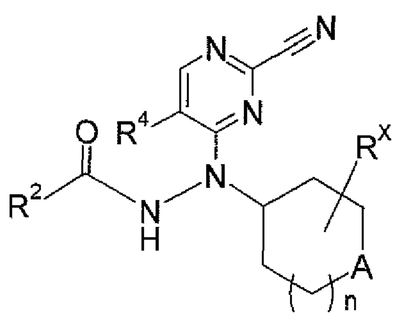

I

Wherein:

Either A represents $CH_2$ and n represents 0 or 1; or A represents -O- or N(C(O)$C_{1-3}$ alkyl) and n represents 1;
When A represents $CH_2$, $R^X$ represents an optional methyl substituent on any carbon atom of the ring to which it is attached, otherwise $R^X$ is absent;
$R^4$ represents halogen;
When A represents $CH_2$ or N(C(O)$C_{1-3}$alkyl), $R^2$ represents -phenyl-$C_{1-3}$alkylene-X or -phenyl-$C_{1-3}$alkylene-X-$R^J$ otherwise $R^2$ represents -phenyl-$C_{1-3}$alkylene-X-$R^J$; wherein any phenyl group in $R^2$ is optionally substituted with at least one group independently selected from halogen or $CF_3$;

$R^J$ represents Z, $C_{1-4}$alkylene-Z or C(O)Z;
X and Z independently represent a monocyclic 4-, 5- or 6-membered, saturated hydrocarbon group containing one or two nitrogen atoms and optionally an oxygen atom, which is optionally substituted with a group selected from: $C_{1-4}$alkyl, OH and $C_{1-4}$ alkylOH;

and pharmaceutically acceptable derivatives thereof.

**2.** At least one chemical entity according to claim 1 wherein A represents $CH_2$.

**3.** At least one chemical entity according to claim 1 or claim 2 wherein A represents $CH_2$, n represents 0 and $R^X$ represents methyl.

**4.** At least one chemical entity according to any one of claims 1 to 3 wherein $R^4$ represents chlorine, bromine or iodine.

**5.** At least one chemical entity according to any one of claims 1 to 4 wherein A represents $CH_2$ or N(C(O)$C_{1-3}$alkyl) and $R^2$ represents -phenyl-$C_{1-3}$alkylene-X-$R^J$,
wherein phenyl is optionally substituted with one group selected from halogen or $CF_3$.

**6.** At least one chemical entity according to any one of claims 1 to 5 wherein X represents piperidine, piperazine or morpholine, each of which is optionally substituted.

**7.** At least one chemical entity according to any one of claims 1 to 6 wherein Z represents piperidine, piperazine or morpholine, each of which is optionally substituted.

**8.** At least one chemical entity selected from:

N'-(5-bromo-2-cyano-4-pyrimidinyl)-N'-cyclopentyf-4-[(4-methyl-1-piperazinyl)methyl]benzohydrazide;
N'-(5-bromo-2-cyano-4-pyrimidinyl)-N'-[(1 R,2S+1 S,2R)-2-methylcyclo pentyl]-4-[(4-methyl-1-piperazinyl)methyl]benzohydrazide;
N'-(5-bromo-2-cyano-4-pyrimidinyl)-N' -[(1 R,2R+1S,2S)-2-methylcyclo pentyl]-4-[(4-methyl-1-piperazinyl)methyl]benzohydrazide;
N'-(5-bromo-2-cyano-4-pyrimidinyl)-N'-(3-methylcyclopentyl)-4-{[4-(4-methyl-1-piperazinyl)-1-piperidinyl]methyl}benzohydrazide;
N'-(5-bromo-2-cyano-4-pyrimidinyl)-N'-(3-methylcyclopentyl)-4-({4-[(1-methyl-4-piperidinyl)methyl]-1-piperazinyl}methyl)benzohydrazide;
N'-(5-bromo-2-cyano-4-pyrimidinyl)-N'-cyclopentyl-4-{[4-(4-morpholinyl)-1-piperidinyl]methyl}benzohydrazide;
N'-(5-bromo-2-cyano-4-pyrimidinyl)-N'-cyclopentyl-4-({4-[(1-methyl-4-piperidinyl)methyl]-1-piperazinyl}methyl)benzohydrazide;
N'-(5-bromo-2-cyano-4-pyrimidinyl)-N'-cyclopentyl-4-({4-[(1-methyl-3-piperidinyl)methyl]-1-piperazinyl}methyl)benzohydrazide;
N'-(5-bromo-2-cyano-4-pyrimidinyl)-N'-cyclopentyl-4-({4-[(4-methyl-1-piperazinyl)carbonyl]-1-piperidinyl}methyl)benzohydrazide;
N'-(5-bromo-2-cyano-4-pyrimidinyl)-N'-cyclopentyl-4-{[4-(4-methyl-1-piperazinyl)-1-piperidinyl]methyl}benzohydrazide;
N'-(5-bromo-2-cyano-4-pyrimidinyl)-N'-cyclopentyl-3-{[4-(4-methyl-1-piperazinyl)-1-piperidinyl]methyl}benzohydrazide;
N'-(5-bromo-2-cyano-4-pyrimidinyl)-N'-cyclopentyl-3-({4-[(4-methyl-1-piperazinyl)carbonyl]-1-piperidinyl}methyl)benzohydrazide;
N'-(5-bromo-2-cyano-4-pyrimidinyl)-N'-cyclopentyl-3-{[4-(4-morpholinyl)-1-piperidinyl]methyl}benzohydrazide;
N'-(5-bromo-2-cyano-4-pyrimidinyl)-N'-cyclopentyl-3-({4-[(1-methyl-3-piperidinyl)methyl]-1-piperazinyl}methyl)benzohydrazide;
N'-(5-bromo-2-cyano-4-pyrimidinyl)-N'-cyclopentyl-3-({4-[(1-methyl-4-piperidinyl)methyl]-1-piperazinyl}methyl)benzohydrazide;
N'-(5-bromo-2-cyano-4-pyrimidinyl)-N'-cyclohexyl-4-[(4-methyl-1-piperazinyl)methyl]benzohydrazide;
N'-(5-bromo-2-cyano-4-pyrimidinyl)-N'-cyclohexyl-4-{[4-(4-methyl-1-piperazinyl)-1-piperidinyl]methyl}benzohydrazide;
N'-(5-bromo-2-cyano-4-pyrimidinyl)-N'-cyclohexyl-4-{[4-(4-morpholinyl)-1-piperidinyl]methyl}benzohydrazide;
N'-(5-bromo-2-cyano-4-pyrimidinyl)-N'-cyclohexyl-4-({4-[(1-methyl-4-piperidinyl)methyl]-1-piperazinyl}methyl)

benzohydrazide;
N'-(5-bromo-2-cyano-4-pyrimidinyl)-N'-cyclohexyl-3-{[4-(4-methyl-1-piperazinyl)-1-piperidinyl]methyl}benzohydrazide;
N'-(5-bromo-2-cyano-4-pyrimidinyl)-N'-cyclohexyl-4-[(4-hydroxy-1-piperidinyl)methyl]benzohydrazide;
N'-(5-bromo-2-cyano-4-pyrimidinyl)-N'-cyclohexyl-4-({4-[(4-methyl-1-piperazinyl)carbonyl]-1-piperidinyl}methyl)benzohydrazide;
N'-(5-bromo-2-cyano-4-pyrimidinyl)-N'-cyclohexyl-3-({4-[(4-methyl-1-piperazinyl)carbonyl]-1-piperidinyl}methyl)benzohydrazide;
N'-(5-bromo-2-cyano-4-pyrimidinyl)-N'-cyclohexyl-3-{[4-(4-morpholinyl)-1-piperidinyl]methyl}benzohydrazide;
N'-(5-bromo-2-cyano-4-pyrimidinyl)-N'-cyclohexyl-4-({4-[(1-methyl-3-piperidinyl)methyl]-1-piperazinyl}methyl) benzohydrazide;
N'-(5- bromo- 2- cyano- 4- pyrimidinyl)-N'-(3- methylcyclopentyl)- 4-[(4- methyl- 1- piperazinyl) methyl] benzohydrazide;
N'-(5-bromo-2-cyano-4-pyrimidinyl)-N'-cyclohexyl-3-({4-[(1-methyl-3-piperidinyl)methyl]-1-piperazinyl}methyl) benzohydrazide;
N'-(5-bromo-2-cyano-4-pyrimidinyl)-N'-cydohexyl-3-({4-[(1-methyl-4-piperidinyl)methyl]-1-piperazinyl}methyl) benzohydrazide;
N'-(5-bromo-2-cyano-4-pyrimidinyl)-4-{[4-(4-methyl-1-piperazinyl)-1-piperidinyl]methyl}-N'-(tetrahydro-2H-pyran-4-yl)benzohydrazide;
N'-(5-bromo-2-cyano-4-pyrimidinyl)-4-({4-[(1-methyl-4-piperidinyl)methyl]-1-piperazinyl}methyl)-N'-(tetrahydro-2H-pyran-4-yl)benzohydrazide;
N'-(5-bromo-2-cyano-4-pyrimidinyl)-4-({4-[(1-methyl-3-piperidinyl)methyl]-1-piperazinyl}methyl)-N'-(tetrahydro-2H-pyran-4-yl)benzohydrazide;
N'-(5-bromo-2-cyano-4-pyrimidinyl)-4-({4-[(4-methyl-1-piperazinyl)carbonyl]-1-piperidinyl}methyl)-N'-(tetrahydro-2H-pyran-4-yl)benzohydrazide;
N'-(5-bromo-2-cyano-4-pyrimidinyl)-4-{[3-(1-pyrrolidinyl)-1-azetidinyl]methyl}-N'-(tetrahydro-2H-pyran-4-yl) benzohydrazide;
N'-(5-chloro-2-cyano-4-pyrimidinyl)-4-{[4-(4-methyl-1-piperazinyl)-1-piperidinyl]methyl}-N'-(tetrahydro-2H-pyran-4-yl)benzohydrazide,
N'-(5-chloro-2-cyano-4-pyrimidinyl)-4-({4-[(1-methyl-4-piperidinyl)methyl]-1-piperazinyl}methyl)-N'-(tetrahydro-2H-pyran-4-yl)benzohydrazide;
N'-(5-chloro-2-cyano-4-pyrimidinyl)-4-({4-[(4-methyl-1-piperazinyl)carbonyl]-1-piperidinyl}methyl)-N'-(tetrahydro-2H-pyran-4-yl)benzohydrazide;
N'-(1-acetyl-4-piperidinyl)-N'-(5-bromo-2-cyano-4-pyrimidinyl)-4-[(4-methyl-1-piperazinyl)methyl]benzohydrazide;
N'-(1-acetyl-4-piperidinyl)-N'-(5-bromo-2-cyano-4-pyrimidinyl)-4-{[4-(4-methyl-1-piperazinyl)-1-piperidinyl]methyl}benzohydrazide;

and pharmaceutically acceptable derivatives thereof.

**9.** At least one chemical entity selected from:

N'-(5-bromo-2-cyano-4-pyrimidinyi)-N'-cyclopentyl-4-[(4-methyl-1-piperazinyl)methyl]benzohydrazide trifluoroacetate;
N'-(5-bromo-2-cyano-4-pyrimidinyl)-N'-[(1R,2S+1S,2R)-2-methylcyclo pentyl]-4-[(4-methyl-1-piperazinyl)methyl]benzohydrazide trifluoroacetate
N'-(5-bromo-2-cyano-4-pyrimidinyl)-N'-[(1R,2R+1S,2S)-2-methylcyclo pentyl]-4-[(4-methyl-1-piperazinyl)methyl]benzohydrazide trifluoroacetate;
N'-(5-bromo-2-cyano-4-pyrimidinyl)-N'-(3-methylcyclopentyl)-4-{[4-(4-methyl-1-piperazinyl)-1-piperidinyl]methyl}benzohydrazide trifluoroacetate;
N'-(5-bromo-2-cyano-4-pyrimidinyl)-N'-(3-methylcyclopentyl)-4-({4-[(1-methyl-4-piperidinyl)methyl]-1-piperazinyl}methyl)benzohydrazide trifluoroacetate;
N'-(5-bromo-2-cyano-4-pyrimidinyl)-N'-cyclopentyl-4-{[4-(4-morpholinyl)-1-piperidinyl]methyl}benzohydrazide trifluoroacetate;
N'-(5-bromo-2-cyano-4-pyrimidinyl)-N'-cyclopentyl-4-({4-[(1-methyl-4-piperidinyl)methyl]-1-piperazinyl}methyl)benzohydrazide trifluoroacetate;
N'-(5-bromo-2-cyano-4-pyrimidinyl)-N'-cyclopentyl-4-({4-[(1-methyl-3-piperidinyl)methyl]-1-piperazinyl}methyl)benzohydrazide trifluoroacetate;

N'-(5-bromo-2-cyano-4-pyrimidinyl)-N'-cyclopentyl-4-({4-[(4-methyl-1-piperazinyl)carbonyl]-1-piperidinyl}methyl)benzohydrazide trifluoroacetate;
N'-(5-bromo-2-cyano-4-pyrimidinyl)-N'-cyclopentyl-4-{[4-(4-methyl-1-piperazinyl)-1-piperidinyl]methyl}benzohydrazide trifluoroacetate;
N'-(5-bromo-2-cyano-4-pyrimidinyl)-N'-cyclopentyl-3-{[4-(4-methyl-1-piperazinyl)-1-piperidinyl]methyl}benzohydrazide trifluoroacetate;
N'-(5-bromo-2-cyano-4-pyrimidinyl)-N'-cyclopentyl-3-({4-[(4-methyl-1-piperazinyl)carbonyl]-1-piperidinyl}methyl)benzohydrazide trifluoroacetate;
N'-(5-bromo-2-cyano-4-pyrimidinyl)-N'-cyclopentyl-3-{[4-(4-morpholinyl)-1-piperidinyl]methyl}benzohydrazide trifluoroacetate;
N'-(5-bromo-2-cyano-4-pyrimidinyl)-N'-cyclopentyl-3-({4-[(1-methy(-3-piperidinyl)methyl]-1-piperazinyl}methyl)benzohydrazide trifluoroacetate;
N'-(5-bromo-2-cyano-4-pyrimidinyl)-N'-cyclopentyl-3-({4-[(1-methyl-4-piperidinyl)methyl]-1-piperazinyl}methyl)benzohydrazide trifluoroacetate;
N'-(5-bromo-2-cyano-4-pyrimidinyl)-N'-cyclohexyl-4-[(4-methyl-1-piperazinyl)methyl]benzohydrazide trifluoroacetate;
N'-(5-bromo-2-cyano-4-pyrimidinyl)-N'-cyclohexyl-4-{[4-(4-methyl-1-piperazinyl)-1-piperidinyl]methyl}benzohydrazide trifluoroacetate;
N'-(5-bromo-2-cyano-4-pyrimidinyl)-N'-cyclohexyl-4-{[4-(4-morpholinyl)-1-piperidinyl]methyl}benzohydrazide trifluoroacetate;
N'-(5-bromo-2-cyano-4-pyrimidinyl)-N'-cyclohexyl-4-({4-[(1-methyl-4-piperidinyl)methyl]-1-piperazinyl}methyl) benzohydrazide trifluoroacetate;
N'-(5-bromo-2-cyano-4-pyrimidinyl)-N'-cyclohexyl-3-{[4-(4-methyl-1-piperazinyl)-1-piperidinyl]methyl}benzohydrazide trifluoroacetate;
N'-(5-bromo-2-cyano-4-pyrimidinyl)-N'-cyclohexyl-4-[(4-hydroxy-1-piperidinyl)methyl]benzohydrazide trifluoroacetate;
N'-(5-bromo-2-cyano-4-pyrimidinyl)-N'-cyclohexyl-4-({4-[(4-methyl-1-piperazinyl)carbonyl]-1-piperidinyl}methyl)benzohydrazide trifluoroacetate;
N'-(5-bromo-2-cyano-4-pyrimidinyl)-N'-cyclohexyl-3-({4-[(4-methyl-1-piperazinyl)carbonyl]-1-piperidinyl}methyl)benzohydrazide trifluoroacetate;
N'-(5-bromo-2-cyano-4-pyrimidinyl)-N'-cyclohexyl-3-{[4-(4-morpholinyl)-1-piperidinyl]methyl}benzohydrazide trifluoroacetate;
N'-(5-bromo-2-cyano-4-pyrimidinyl)-N'-cyclohexyl-4-({4-[(1-methyl-3-piperidinyl)methyl]-1-piperazinyl}methyl) benzohydrazide trifluoroacetate;
N'-(5-bromo-2-cyano-4-pyrimidinyl)-N'-(3-methylcyclopentyl)-4-[{4-methyl-1-piperazinyl}methyl]benzohydrazide trifluoroacetate;
N'-(5-bromo-2-cyano-4-pyrimidinyl)-N'-cyclohexyl-3-({4-[(1-methyl-3-piperidinyl)methyl]-1-piperazinyl}methyl) benzohydrazide trifluoroacetate;
N'-(5-bromo-2-cyano-4-pyrimidinyl)-N'-cyclohexyl-3-({4-[(1-methyl-4-piperidinyl)methyl]-1-piperazinyl}methyl) benzohydrazide trifluoroacetate;
N'-(5-bromo-2-cyano-4-pyrimidinyl)-4-{[4-(4-methyl-1-piperazinyl)-1-piperidinyl]methyl}-N'-(tetrahydro-2H-pyran-4-yl)benzohydrazide trifluoroacetate;
N'-(5-bromo-2-cyano-4-pyrimidinyl)-4-({4-[(1-methyl-4-piperidinyl)methyl]-1-piperazinyl}methyl)-N'-(tetrahydro-2H-pyran-4-yl)benzohydrazide trifluoroacetate;
N'-(5-bromo-2-cyano-4-pyrimidinyl)-4-({4-[(1-methyl-3-piperidinyl)methyl]-1-piperazinyl}methyl)-N'-(tetrahydro-2H-pyran-4-yl)benzohydrazide trifluoroacetate;
N'-(5-bromo-2-cyano-4-pyrimidinyl)-4-({4-[(4-methyl-1-piperazinyl)carbonyl]-1-piperidinyl}methyl)-N'-(tetrahydro-2H-pyran-4-yl)benzohydrazide trifluoroacetate;
N'-(5-bromo-2-cyano-4-pyrimidinyl)-4-{[3-(1-pyrrolidinyl)-1-azetidinyl]methyl}-N'-(tetra hyd ro-2H-pyran-4-yl) benzohyd razid e trifluoroacetate;
N'-(5-chloro-2-cyano-4-pyrimidinyl)-4-{[4-(4-methyl-1-piperazinyl)-1-piperidinyl]methyl}-N'-(tetrahydro-2H-pyran-4-yl)benzohydrazide trifluoroacetate;
N'-(5-chloro-2-cyano-4-pyrimidinyl)-4-({4-[(1-methyl-4-piperidinyl)methyl]-1-piperazinyl}methyl)-N'-(tetrahydro-2H-pyran-4-yl)benzohydrazide trifluoroacetate;
N'-(5-chloro-2-cyano-4-pyrimidinyl)-4-({4-[(4-methyl-1-piperazinyl)carbonyl]-1-piperidinyl}methyl)-N'-(tetrahydro-2H-pyran-4-yl)benzohydrazide trifluoroacetate;
N'-(1-acetyl-4-piperidinyl)-N'-(5-bromo-2-cyano-4-pyrimidinyl)-4-[(4-methyl-1-piperazinyl)methyl]benzohydrazide trifluoroacetate; and

N'-(1-acetyl-4-piperidinyl)-N'-(5-bromo-2-cyano-4-pyrimidinyl)-4-{[4-(4-methyl-1-piperazinyl)-1-piperidinyl]methyl}benzohydrazide trifluoroacetate.

**10.** At least one chemical entity according to according to any one of claims 1 to 9 for use in medical therapy.

**11.** Use of at least one chemical entity according to any one of claims 1 to 9 in the manufacture of a medicament for the treatment of a condition susceptible to mediation by a cysteine protease inhibitor.

**12.** Use of at least one chemical entity according to any one of claims 1 to 9 in the manufacture of a medicament for the treatment of malaria.

**13.** A method for the treatment of a human or animal subject suffering from a condition susceptible to mediation by a cysteine protease inhibitor, comprising administering to said human or animal subject an effective amount of at least one chemical entity according to any one of claims 1 to 9.

**14.** A method for the treatment of a human or animal subject suffering from malaria, comprising administering to said human or animal subject an effective amount of at least one chemical entity according to any one of claims 1 to 9.

**15.** A pharmaceutical composition comprising at least one chemical entity according to any one of claims 1 to 9 in admixture with one or more pharmaceutically acceptable carrier and/or excipient.

**16.** A process for the preparation of compounds of Formula I as defined in claim 1, from a reaction between compounds of Formula II, from a reaction between compounds of Formula II, wherein A, n, $R^4$ and $R^X$ are as defined for Formula I, compounds of Formula III, wherein Hal is chlorine or bromine, and compounds of Formula IV, wherein X and $R^J$ are as defined for Formula I, according to the Scheme below.

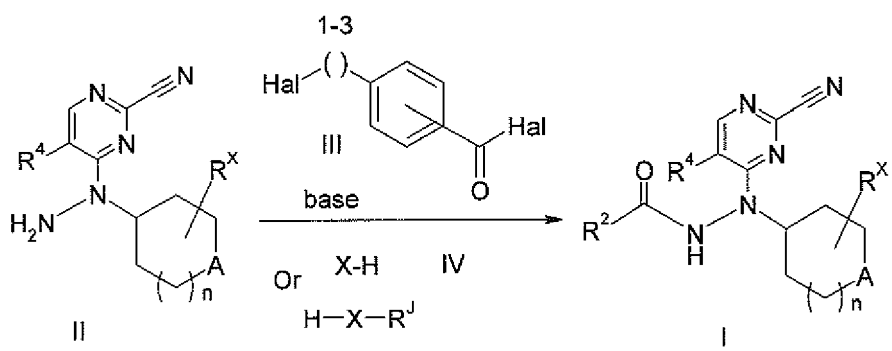


**17.** A process for the preparation of compounds of Formula I as defined in claim 1, from a reaction between compounds of Formula V, wherein A, n, $R^4$ and $R^X$ are as defined for Formula I and Hal is chlorine or bromine and compounds of Formula IV, wherein X and $R^J$ are as defined for Formula I, according to the Scheme below.

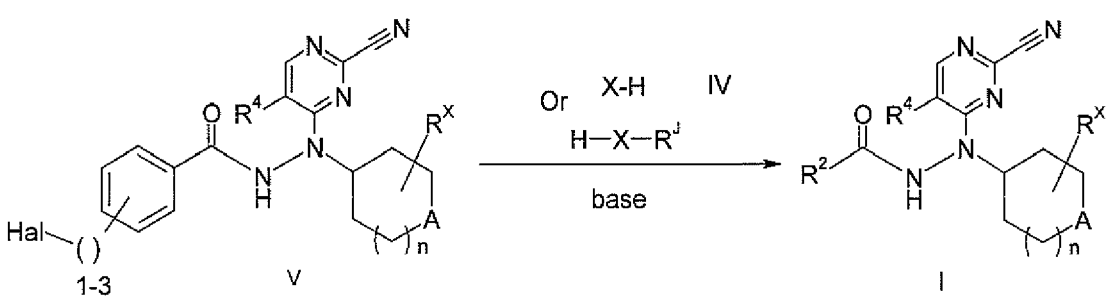

European Patent Office

# PARTIAL EUROPEAN SEARCH REPORT

which under Rule 45 of the European Patent Convention shall be considered, for the purposes of subsequent proceedings, as the European search report

Application Number
EP 06 38 1045

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2006/027211 A (GLAXO GROUP LTD [GB]; CASTRO PICHEL JULIA [ES]; CHAPARRO MARTIN MARIA) 16 March 2006 (2006-03-16)<br>* abstract *<br>* examples *<br>* claims *<br>----- | 1-17 | INV.<br>C07D239/42<br>C07D401/12<br>C07D403/12<br>C07D405/12<br>A61K31/505<br>A61K31/506<br>A61P33/06 |
| A | US 2004/097493 A1 (CHEN JIAN JEFFREY [US] ET AL) 20 May 2004 (2004-05-20)<br>* abstract *<br>* paragraph [0105] *<br>* examples *<br>* claims *<br>----- | 1-17 | |
| A | WO 03/101442 A (SMITHKLINE BEECHAM CORP [US]; AUBART KELLY M [US]; BENOWITZ ANDREW B [) 11 December 2003 (2003-12-11)<br>* abstract *<br>* examples *<br>* claims *<br>----- | 1-17 | |

TECHNICAL FIELDS SEARCHED (IPC)

C07D
A61K
A61P

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

3

| Place of search | Date of completion of the search | Examiner |
|---|---|---|
| Munich | 29 March 2007 | Stix-Malaun, Elke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 06 38 1045

Although claims 13 and 14 are directed to a method of treatment of the human/animal body (Article 52(4) EPC), the search has been carried out and based on the alleged effects of the compound/composition.

-----

## ANNEX TO THE EUROPEAN SEARCH REPORT ON EUROPEAN PATENT APPLICATION NO. EP 06 38 1045

This annex lists the patent family members relating to the patent documents cited in the above-mentioned European search report.
The members are as contained in the European Patent Office EDP file on
The European Patent Office is in no way liable for these particulars which are merely given for the purpose of information.

29-03-2007

| Patent document cited in search report | Publication date | Patent family member(s) | Publication date |
|---|---|---|---|
| WO 2006027211 A | 16-03-2006 | AR 050858 A1 | 29-11-2006 |
| | | EP 1632487 A1 | 08-03-2006 |
| US 2004097493 A1 | 20-05-2004 | US 2005261284 A1 | 24-11-2005 |
| WO 03101442 A | 11-12-2003 | AU 2003247445 A1 | 19-12-2003 |
| | | BR 0311318 A | 22-02-2005 |
| | | CA 2487805 A1 | 11-12-2003 |
| | | EP 1509218 A1 | 02-03-2005 |
| | | IS 7610 A | 22-12-2004 |
| | | JP 2006501155 T | 12-01-2006 |
| | | MX PA04011951 A | 31-03-2005 |
| | | OA 12823 A | 11-07-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5501969 A
- WO 9404172 A
- EP 0603873 A1
- WO 2005085210 A1
- WO 2005103012 A1


### Non-patent literature cited in the description


- **BREMAN, J. G. et al.** *Am. Trop. Med. Hyg.,* 2001, vol. 64, 1-11
- **FRANCIS S.E. et al.** *Annu. Rev. Microbiol.,* 1997, vol. 51, 97-123
- Protease inhibitors. **ROSENTHAL P.J.** Antimalarial Chemotherapy: Mechanisms of Action, Resistance, and New Directions in Drug Discovery. Humana Press, 2001, 325-345
- **ROSENTHAL P. J. et al.** *J. Clin. Invest.,* 1998, vol. 82, 1560-6
- **GAMBOA DE DOMINGUEZ N.D. ; ROSENTHAL P.J.** *Blood,* 1996, vol. 87, 4448-54
- **ROSENTHAL P.J. ; NELSON R.G.** *Mol Biochem Parasitol,* 1992, vol. 51, 143-52
- **SALAS F. et al.** *Infect. Immun.,* 1995, vol. 63, 2120-5
- **ROSENTHAL, P. J. et al.** *Curr. Pharm. Des.,* 2002, vol. 8, 1669-1672
- **SHENAI B.R.** *J Biol Chem,* 2000, vol. 275, 29000-10
- **SHENAI B.R. et al.** *J. Biol. Chem.,* 2000, vol. 275, 29000-10
- **SIJWALI P.S. et al.** *Biochem. J.,* 2001, vol. 360, 481-9
- **SHENAI B.R. ; ROSENTHAL P.J.** *Mol. Biochem. Parasitol.,* 2002, vol. 122, 99-104
- **SIJWALI, P. S. et al.** *Proceedings of the National Academy of Sciences of the United States of America,* vol. 101, 8721-8726
- **MENDIS, K ; SINA, B. J ; MARCHESINI, P ; CARTER, R.** The neglected burden of Plasmodium vivax malaria. *Am. J. Trop. Med. Hyg.,* 2001, vol. 64, 97-106
- **NA, B.K ; SHENAI, B. R ; SIJWALI, P. S ; CHOE, Y ; PANDEY, K. C ; SINGH, A ; CRAIK, C. S ; ROSENTHAL, P. J.** identification and biochemical characterization of vivapains, cysteine proteases of the malaria parasite Plasmodium vivax. *Biochem. J.,* 2004, vol. 378, 529-538
- **BOSSARD, M. J et al.** *J. Biol. Chem.,* 1996, vol. 271, 12517-12524
- **DRAKE, F.H et al.** *J. Biol. Chem.,* 1996, vol. 271, 12511-12516
- **BROMME, D et al.** *J. Biol. Chem.,* 1996, vol. 271, 2126-2132
- **JOYCE J. A et al.** *Cancer Cell,* 2004, vol. 5, 443-453
- **GOCHEVA V.** *Genes & Development,* 2006, vol. 20, 543-556
- **POTEMPA, J et al.** *Perspectives in Drug Discovery and Design,* 1994, vol. 2, 445-458
- **DELAISSE et al.** *Biochem. J.,* 1980, vol. 192, 365
- **DELAISSE.** *Biochem. Biophys. Res. Commun.,* 1984, vol. 125, 441
- **LERNER et al.** *J. Bone Min. Res.,* 1992, vol. 7, 433
- **TEZUKA et al.** *J. Biol. Chem.,* 1994, vol. 269, 1106
- **INAOKA et al.** *Biochem. Biophys. Res. Commun.,* 1995, vol. 206, 89
- **SHI et al.** *FEBS Lett.,* 1995, vol. 357, 129
- **DODDS et al.** *Arthritis & Rheumatism,* 1999, vol. 42, 1588
- **HOU et al.** *American Journal of Pathology,* 2002, vol. 159, 2167
- **KONTTINEN et al.** *Arthritis & Rheumatism,* 2002, vol. 46, 953
- **LITTLEWOOD-EVANS et al.** *Cancer Res.,* 1997, vol. 57, 5386
- **BRUBAKER et al.** *J. Bone Miner. Res.,* 2003, vol. 18, 222
- **ISHIKAWA et al.** *Mol. Carcinog.,* 2001, vol. 32, 84
- **LECAILLE et al.** *Chem. Rev.,* 2002, vol. 102, 4459
- **LIU et al.** *Arterioscler Throm Vasc Biol.,* 2004, vol. 24, 1359
- **POTTS et al.** *Int. J. Exp. Path.,* 2004, vol. 85, 85
- **URBICH et al.** *Nat. Med.,* 2005, vol. 11, 206
- **SCHEDEL et al.** *Gene Ther.,* 2004, vol. 11, 1040

- **NAKAGAWA et al.** *Immunity,* 1999, vol. 10, 207
- **LANG et al.** *J. Rheumatol,* 2000, vol. 27, 1970
- **ZHANG et al.** *Immunology,* 2000, vol. 100, 13
- **MORT et al.** *Biochem. J.,* 1998, vol. 335, 491
- **ELSAID, K.A. et al.** *Transactions of the Orthopedic Research Society, 51st Annual Meeting,* 2005
- Principles and Practice. Burger's Medicinal Chemistry and Drug Discovery. vol. 1
- **BERGE et al.** *J. Pharm. Sci.,* 1977, vol. 66, 1-19
- Remington's Pharmaceutical Sciences. Mack Publishing Company
- The Handbook of Pharmaceutical Additives. Gower Publishing Limited
- The Handbook of Pharmaceutical Excipients. the American Pharmaceutical Association and the Pharmaceutical Press
- **LUO G. et al.** *Tetrahedron Letters,* 2002, vol. 43 (33), 5739-5742
- **HILPERT, H.** *Tetrahedron,* 2001, vol. 57, 7675-7683
- **DYKER. H. et al.** *J. Org. Chem.,* 2001, vol. 66, 3760-3766
- **T.W. GREENE ; P.G.M. WUTS.** Protective groups in organic synthesis. John Wiley & sons, 1991
- **P.J. KOCIENSKI.** Protecting Groups. Georg Thieme Verlag, 1994
- **MORRISON et al.** *Adv. Enzymol. Relat. Areas Mol. Biol,* 1988, vol. 61, 201
- **SIJWALI S. ; ROSENTHAL P. J.** *Proceedings of the National Academy of Sciences of the United States of America (PNAS,* 2004, vol. 101 (13), 4384-4389
- *Measurement of Parasite Growth rates and Inhibitor Sensitivity,* 4385
- **SINGH A. ; ROSENTHAL P. J.** *Antimicrobial Agents and Chemotherapy,* 2001, vol. 45 (3), 949-951
- **DIVO A. A. et al.** *Protozool,* 1985, vol. 32, 59-64
- **LIEPE K. et al.** *Anticancer Research,* 2005, vol. 25 (2A), 1067-1073
- **NEUDERT M. et al.** *International Journal of Cancer,* 2003, vol. 107 (3), 468-477
- **ANGELUCCI A. et al.** *International Journal of Oncology,* 2004, vol. 25 (6), 1713-1720
- **SASAKI A. et al.** *Cancer Research,* 1995, vol. 55 (16), 3551-3557
- **ROSOL T. J. et al.** *Cancer,* 2003, vol. 97, 748-757